(19) Europäisches Patentamt
European Patent Office
Office européen des brevets

(11) **EP 4 211 142 B1**

(12) # EUROPEAN PATENT SPECIFICATION

(45) Date of publication and mention of the grant of the patent:
**05.11.2025 Bulletin 2025/45**

(21) Application number: **21773090.2**

(22) Date of filing: **07.09.2021**

(51) International Patent Classification (IPC):
***C07H 3/02*** *(2006.01)* ***C12P 19/02*** *(2006.01)*
***A23L 33/125*** *(2016.01)* ***A23L 29/30*** *(2016.01)*
***A23P 30/20*** *(2016.01)* ***C07H 1/00*** *(2006.01)*

(52) Cooperative Patent Classification (CPC):
**A23L 29/30; A23L 33/125; A23P 30/20; C07H 1/00; C07H 3/02;** C12P 19/02

(86) International application number:
**PCT/EP2021/074613**

(87) International publication number:
**WO 2022/049307 (10.03.2022 Gazette 2022/10)**

(54) **EXTRUSION PROCESS FOR THE PREPARATION OF A SOLID ALLULOSE COMPOSITION**

EXTRUSIONSVERFAHREN ZUR HERSTELLUNG EINER FESTEN ALLULOSE-ZUSAMMENSETZUNG

PROCÉDÉ D'EXTRUSION POUR LA PRÉPARATION D'UNE COMPOSITION D'ALLULOSE SOLIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.09.2020 EP 20194769**

(43) Date of publication of application:
**19.07.2023 Bulletin 2023/29**

(73) Proprietor: **SAVANNA Ingredients GmbH**
**50189 Elsdorf (DE)**

(72) Inventors:
- **KOCH, Timo Johannes**
  **50189 Elsdorf (DE)**
- **HANFT, Sebastian**
  **50670 Köln (DE)**

(74) Representative: **Kutzenberger Wolff & Partner**
**Waidmarkt 11**
**50676 Köln (DE)**

(56) References cited:
**EP-A1- 3 553 071**
**WO-A1-2017/029244**
**WO-A1-2018/087261**
**WO-A1-2020/005021**
**US-A1- 2017 064 988**
**US-A1- 2018 271 113**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

The invention relates to a process for the preparation of a product allulose composition according to claim 1. The present disclosure further relates to a product allulose composition, preferably solid allulose composition that can be prepared by the process according to the invention and its use. This product allulose composition is not literally claimed as such.

Various methods for preparing allulose syrups have been described in the prior art. These allulose syrups, however, have comparatively high transportation costs due to the water content. It has been attempted to maximize the concentration of allulose in these syrups in order to decrease the overall weight. However, as solubility of allulose in water is limited, such highly concentrated allulose syrups tend to spontaneous crystallization which hinders processing of the syrup and thus have limited storage stability and shelf-life. Further, liquid allulose compositions are more prone to contamination and chemical as well as biological degradation than product allulose composition, preferably solid allulose compositions.

Various methods for preparing crystalline allulose by crystallization from aqueous solutions have also been described in the prior art. Compared to other carbohydrates and monosaccharides, allulose is very difficult to crystallize. The known methods for preparing crystalline allulose by crystallization from aqueous solutions are laborious, very time consuming and require sophisticated equipment. Crystallization needs to be performed under strictly controlled conditions and for long periods of time. Various crystallization methods have been described requiring crystallization times of 40 hours and more. There is thus a demand for methods that provide particulate allulose from aqueous solutions in shorter time and that are therefore suitable for production on industrial scale.

Further, highly crystalline allulose has disadvantages with regard to the preparation of water-containing food or beverages.

On the one hand, D-allulose (D-psicose) crystallizes from aqueous solution solely as β-D-pyranose with 1C ($^1C_4$ (D)) conformation. Thus, in the crystalline state obtained from aqueous solution, the entire amount of D-allulose is present in form of one anomer, namely the β-D-pyranose form. The solubility of D-allulose at 25°C is 291 g per 100 g water. Mutarotation [α] for β-D-psicopyranose in water is about -85 deg $dm^{-1}$ $g^{-1}$ $cm^3$. The time course of absorbance at 280 nm after the dissolution of D-allulose reveals the development of open-chain carbonyl-form in solution at first-order kinetic behavior with a rate constant k = 4.44 $ms^{-1}$. The equilibrium content of carbonyl-form of D-allulose has been estimated to amount to about 0.2% (A. Kwiecien et al., Carboh Res, 343(13), 2008, 2336-2339; K. Fukada et al., Bull Chem Soc Japan, 2010, 83(10), 1193-1197).

When allulose is employed in processing foodstuffs or beverages, it is desirable to evenly distribute the allulose. When using crystalline allulose, however, this cannot always be easily achieved. For example, various foodstuffs are provided in deep-frozen form and are processed during defrosting. When adding crystalline allulose to deep-frozen foodstuff during defrosting, the crystallites merely slowly dissolve in the water film that emerges at the surface of the defrosting goods.

On the other hand, in aqueous solution, D-allulose is a mixture of various anomeric forms. It has been reported that the equilibrium composition of D-psicose in water, as determined from its $^{13}C$ NMR spectrum, is 7 (+37%) α-furanose : 2 (~11%) β-furanose : 5 (~26%) α-pyranose: 5 (~26%) β-pyranose (P.H.M. Herve du Penhoat et al., Carboh Res 36(1), 1974, 111-120). Other authors report that by nuclear magnetic resonance (NMR), the four ring forms of psicose, α- and β-pyranose and α- and β-furanose, have almost equal concentrations in aqueous solution (A.D. French et al., Computational Chemistry, 15(5), 1994, 561-570). Still further authors report that in $D_2O$, the anomeric forms of allulose at 303.15 K have the following percent compositions: 39% α-furanose, 15% β-furanose, 22% α-pyranose, 24% β-pyranose, and 0.2% keto form (S.J. Angyal et al., Advances in Carbohydrate Chemistry, Vol. 42, Academic Press, New York, 1984; R.N. Goldberg et al., J. Phys. Chem. Ref. Data, Vol. 18(2), 1989, 809-880, page 827).

Thus, when crystalline allulose is employed in the preparation of a water-containing foodstuff, the allulose crystals dissolve in water and it takes considerable time until the β-D-pyranose form has equilibrated with the other forms of allulose that coexist in aqueous solution. As the individual forms of allulose (a-pyranose, β-pyranose, α-furanose, β-furanose, open keto form) have different properties, the overall properties of the freshly prepared and not yet equilibrated allulose solution change over time until the equilibrium has been reached (see Figures 1 and 2).

Various chemical modifications of allulose that are relevant for its use in the preparation of beverages and foodstuff depend upon the anomeric form of allulose. The carbonyl group of the free keto form of allulose has a different chemical reactivity compared to the ketal group of the furanose form and the pyranose form, respectively. This is relevant for various possible reactions of allulose such as enolizations, isomerizations, Lowry de Bruyn-Alberda van Ekenstein transformations, dedydrations e.g. by β-elimination, formation of hydroxymethyl furfural (HMF), fragmentation, caramelization, oxidative browning, non-oxidative browning, sugar-ammonia reactions, sugar-amine reactions (Maillard reaction), and the like. Thus, it can be expected that a freshly prepared and not yet equilibrated allulose solution has a different behavior and is more difficult to control compared to an equilibrated allulose solution.

Likewise, physical properties of allulose in solution that are relevant for processing beverages and foodstuff

depend upon the anomeric form of allulose, such as refractive index. Automated high-throughput processes are frequently regulated by monitoring properties of the processed beverages and foodstuff and may include physical properties of allulose in solution. When these physical properties change over time because the anomeric forms are not yet in equilibrium with one another, monitoring and regulation are not possible in a reliable manner. Thus, it can be expected that a freshly prepared and not yet equilibrated allulose solution has a different behavior and is more difficult to process in such highly sophisticated and automated processes compared to an equilibrated allulose solution.

CN 104 447 888 discloses preparing a crystal allulose product by virtue of working procedures such as chemical differential phase isomerism, refined chromatography separation and purification, concentration and crystallization and the like by adopting glucose as the raw material.

CN 106 480 125 relates to a method using a solid D-psicose3-epimerase to convert fructose to obtain a conversion solution containing high-concentration D-psicose. A cooling crystallization process is used for crystallizing the conversion solution to obtain the high-concentration D-psicose crystal.

CN 107 699 557 discloses a method for preparation of high-purity D-psicose comprising the step of performing chromatographic separation, concentration, crystallization or drying on a D-psicose solution to obtain D-psicose.

US 2011 237 790 relates to a method of producing D-psicose crystals from a D-psicose solution by using supersaturation.

US 2014 370 171 aims at providing a homogeneous sugar composition containing sucrose and D-psicose. A sugar composition containing sucrose and D-psicose is provided, wherein crystals of the sucrose are coated with the D-psicose in a crystalline or amorphous state. The content of the D-psicose is 1 part by weight or more and 50 parts by weight or less relative to 100 parts by weight of the total weight of the sucrose and the D-psicose.

US 2016 050 954 concerns a composition for a non-cariogenic confectionery or pharmaceutical product obtained by a hard sugar-coating method, and having improved crispiness.

US 2016 302 463 relates to the use of high levels of allulose in food and beverage products.

KR 2016 062 349 relates to a method for producing D-psicose having high purity of 99% (w/w) or higher comprising the step of concentrating a D-psicose solution, performing heat exchange cooling on the D-psicose solution, crystallizing the D-psicose solution; performing heat exchange cooling on a crystallization separation base solution, and re-circulating the crystallization separation base solution within a procedure. A continuous chromatography separation fructose base solution and a crystallization separation base solution are collected and fed during an enzyme reaction procedure, so D-psicose may be stably separated through continuous chromatography despite long-term recirculation thereof.

WO 2017 029 244 relates to a powder comprising allulose, wherein the average particle size of the powder is within the range of at most 5.0 mm, preferably within the range of (i) at most 900 μm; (ii) from 900 μm to 2.0 mm; or (iii) from 2.0 mm to 5.0 mm.

US 2017 064 988 discloses substantially water soluble, substantially non-dusting delivery systems for natural high-potency sweeteners, methods for their formulation, and uses.

US 2017 313 734 discloses a method for producing high purity D-psicose crystals having a purity of 98% (w/w) or more and a grain size of MA200 or more. The method includes: removing impurities from a D-psicose solution to obtain a purified D-psicose solution; concentrating the purified D-psicose solution; cooling the concentrated D-psicose solution to 30°C to 40°C through a heat exchanger; seed crystallizing the D-psicose solution at 30°C to 40°C to obtain a seed crystallized massecuite; and full-scale crystallizing the seed crystallized massecuite. The method can produce pure D-psicose crystals in a suitable form for industrial application through an economical crystallization process from the D-psicose solution without using organic solvents.

US 2018 049 458 relates to allulose syrups, use of allulose syrups in the manufacture of food or beverage products, and food and beverage products made using the allulose syrups.

WO 2018 105 931 relates to a method for preparing psicose by introducing and recycling a psicose crystallization mother liquor obtained from a psicose crystallization process into at least one process selected from the group consisting of an activated carbon treatment process, an ion purification process, a simulated moving bed chromatographic separation process, and a process for concentrating a psicose fraction.

WO 2018 149 707 provides a process for production of a solid material containing isomaltulose crystals and trehalulose.

US 2018 255 814 relates to a mixed saccharide composition containing psicose, glucose and fructose with improved sweetness quality and crystallization, and a method for preventing crystallization of a mixed saccharide composition containing a psicose.

US 2018 271 112 relates to a low calorie, low laxation confection such as chewy candy, hard candy, tableted candy, or gelled candy having acceptable texture, stability, clarity, and flavor delivery that contains a bulk sweetener comprising allulose (psicose). Allulose is combined with sugars, carbohydrates, or polyols to make consumer acceptable confections.

US 2018 271 113 relates to a chewing gum composition comprising crystalline allulose particles and optionally an

aqueous allulose syrup, and to the use of allulose for increasing the hardening rate of chewing gum compositions. US 2018 271 113 does not relate to the extrusion of an allulose solution.

US 2018 279 643 discloses chewing gums containing allulose and methods of making such gums. In one embodiment, the gum comprises about 5% to about 95% gum base, about 0.1% to about 10% flavoring agent and allulose, the allulose being part or all of the bulk sweetener in the gum. The allulose provides the gum with unique properties; the gum is low in calories and may be non-cariogenic. In other embodiments, the allulose is co-dried with other sweeteners or co-evaporated with other sweeteners or with a plasticizing syrup to produce sweetening ingredients and syrups for gum.

US 2018 327 796 relates to a method of producing D-psicose including the steps of subjecting D-fructose to D-psicose epimerization to produce a D-psicose-containing solution, subjecting the D-psicose-containing solution to first cooling and ion purification, subjecting the purified D-psicose-containing solution to first concentration and second cooling, subjecting the D-psicose-containing solution, which has been subjected to first concentration and second cooling, to chromatography to obtain a D-fructose-containing mother liquor and a D-psicose-containing separated solution, and subjecting the D-psicose-containing separated solution to second concentration and third cooling to obtain D-psicose crystals, wherein the D-fructose-containing mother liquor produced by chromatography is reused in the D-psicose epimerization.

WO 2019 004 554 relates to a method for producing a functional crystalline sweetener, and, more specifically, to a method for producing a crystalline sweetener for improving crystal yield and increasing particle size by controlling the content of impurities included in a solution for producing crystals, or the generation of the impurities.

WO 2019 082 206 discloses a sweetener formulation for enhancing the sweetness, creating upfront taste profile and mouth fullness of low intensity sweeteners / less sweetening sugars (rare sugars) comprising at least two of the ingredients selected from (a) rare sugars; (b) disaccharides or (c) oligosaccharides and/or polysaccharides.

WO 2019 083 069 relates to an allulose syrup and a method for manufacturing same. The allulose syrup comprises a viscosity controlling agent and a dispersing agent, and has an appropriate range of viscosity.

WO 2019 088 654 relates to a syrup comprising a citrus extract and saccharides including allulose; a method for manufacturing the syrup, the method comprising a step for mixing the citrus extract, the saccharides including allulose and an acidity regulator; a food composition comprising the syrup comprising the citrus extract and the saccharides including allulose; a flavor-improving composition comprising the citrus extract and the saccharides including allulose; a method for improving the flavor retention of the citrus extract, the method comprising a step for adding the saccharides including allulose to the citrus extract; and a flavor-manifesting composition comprising the citrus extract and the saccharides including allulose.

US 2019 029 299 discloses a syrup composition and a food comprising the same. The syrup composition includes: gum, pectin, or a combination thereof; and allulose.

US 2019 297 931 relates to an aqueous liquid composition comprising allulose, wherein the weight content of allulose is at least 10 wt.-%, relative to the total weight of the liquid composition; and wherein the weight content of allulose is at least 10 wt.-%, relative to the total content of all carbohydrates that are contained in the liquid composition; and wherein the liquid composition has a viscosity of at most 200 mPa·s.

US 2019 328 014 relates to a D-allulose syrup including, besides D-allulose, a D-allulose dimer mass content, expressed in terms of dry mass, greater than 1.5%.

US 2019 330 253 discloses a method for producing D-allulose crystals that allows for a continuous production process and ensures a high yield. A nanofiltration unit is used for producing D-allulose crystals to improve the yield and/or quality of the resulting crystals.

CN 110 872 332 discloses a method for preparing allulose crystals by a two-step process, wherein in a first step an allulose-containing solution is concentrated and preliminary crystallized under reduced pressure and in a second step is deep crystallized via cooling crystallization.

WO 2020 005 021 relates to a sweetener powder composition and a preparation method therefor and, more specifically, to a sweetener powder composition for preparing an amorphous powder containing a functional sweetener and a preparation method therefor.

US 2020 040 023 relates to a method wherein allulose crystals are efficiently produced from an allulose syrup using seed crystals.

US 2020 062 792 discloses a method for preparing high-purity D-psicose, comprising the step of subjecting a crude D-psicose solution to decolorization, filtration, ion exchange, chromatographic separation, concentration, and then crystallization or drying, obtaining D-psicose.

US 2020 085 090 discloses a D-allulose syrup including, besides D-allulose, a D-allulose dimer mass content, expressed in terms of dry mass, lower than 1.5%. Also, a method for producing the syrup and to the use thereof for producing food or pharmaceutical products is disclosed.

There is a demand for allulose preparations that have advantages compared to the allulose preparations of the prior art. Further, there is a demand for processes that make available such allulose preparations in a time efficient and economic manner.

It is an object of the invention to provide processes for the preparation of allulose preparations that have advantages compared to the prior art. The allulose preparations should be ready to use for processing foodstuff and beverages and should contain allulose essentially as the only relevant ingredient, possibly along with residual amounts of fructose that cannot or can hardly be avoided for reasons of synthesis and purification. Minor amounts of glucose may also be present, especially if the starting material for enzymatic synthesis of allulose from fructose was obtained from sucrose. The allulose preparations should quickly dissolve in water without requiring lengthy subsequent equilibration processes e.g. with regard to mutarotation. The allulose preparations should be rapidly obtainable in economic processes on industrial scale, should have good storage stability and low transportation costs. The processes for the preparation of the allulose preparations should be compatible with existing processes for the synthesis of allulose and should be economic and fast.

This object has been achieved by the subject-matter of the patent claims.

It has been surprisingly found that concentrated liquid allulose compositions may be converted into solidified product allulose compositions by extrusion. The extrusion process can be advantageously integrated in processes for the synthesis of allulose, preferably after a concentration step where the concentration of allulose is increased in order to obtain allulose syrups.

Further, it has been surprisingly found that after extrusion a significant proportion of allulose is present in the product allulose compositions in a glassy amorphous state.

Still further, it has been surprisingly found that after extrusion the relative proportion of the various allulose anomers that exist in equilibrated aqueous solution may be maintained in product allulose compositions obtained from extrusion, or that the relative proportion of the various allulose anomers in the product allulose composition at least resembles the corresponding proportion in equilibrated aqueous solution.

Yet further, it has been surprisingly found that the thus prepared product allulose compositions are ready to use and provide a fast dissolution rate. They have a comparatively low weight, i.e. can be shipped at low transportation costs, can be used as powdery bulk material, and have good storage stability.

Furthermore, it has been surprisingly found that compared to conventional crystallization processes the overall yield of allulose in the product is increased.

A first aspect of the invention relates to a process for the preparation of a product allulose composition comprising the steps of

(a) providing a liquid allulose composition comprising allulose dissolved in water, wherein the liquid allulose composition provided in step (a) has an allulose content of at least 69 wt.-% and a water content of at most 30 wt.-%; in each case relative to the total weight of the liquid allulose composition;

(b) heating the liquid allulose composition to an elevated temperature, wherein step (b) is performed under evaporative conditions thereby reducing the content of water of the allulose composition and wherein the elevated temperature is at most 65°C;

(c) feeding the allulose composition into an extruder, wherein the allulose composition that is fed into the extruder in step (c) has a water content of at most 5.0 wt.-%, relative to the total weight of the allulose composition that is fed into the extruder;

(d) extruding the allulose composition in the extruder; preferably subjecting the allulose composition to shearing forces in the extruder and/or optionally, drying the allulose composition in the extruder under evaporative conditions;

(e) obtaining a product allulose composition from the extruder;

(f) allowing the product allulose composition to solidify; preferably to precipitate and/or to crystallize; and

(g) optionally, grinding and/or post-drying the product allulose composition.

Unless expressly stated otherwise, for the purpose of the specification, all percentages are weight percent.

Step (a), step (b), step (c), step (d), step (e), step (f), and optional step (g) of the process according to the invention are preferably performed in alphabetical order. However, it is also contemplated that some or more consecutive steps are performed simultaneously or partially simultaneously.

Preferably, the process according to the invention is operated continuously or semi-continuously.

In preferred embodiments of the process according to the invention, extrusion in step (d) is performed under evaporative conditions so that upon extrusion the allulose composition is further concentrated compared to the liquid allulose composition provided in step (a) and optionally also to the allulose composition obtained in step (b) by removing

water under evaporative conditions.

Preferably, extrusion is performed under evaporative conditions, whereas

- in step (a) the provided liquid allulose composition has a water content within the range of from 2.5 to 30 wt.- %, preferably from 2.5 to 20 wt.-%, more preferably from 2.5 to 10 wt.-%, and even more preferably from 2.5 to 5.0 wt.-%, relative to the total weight of the liquid allulose composition; preferably in step (a) the provided liquid allulose composition has a water content within the range of from 20 to 30 wt.-% (preferably centrifuge discharge) relative to the total weight of the liquid allulose composition;

- in step (d) the extrusion temperature is within the range of from 35 to 75 °C, preferably from 40 to 70 °C, more preferably from 45 to 65 °C, still more preferably from 50 to 60 °C, yet more preferably from 52 to 58 °C, and even more preferably from 54 to 56 °C; preferably, in step (d) the extrusion temperature is within the range of from 50 to 60°C, wherein a liquid allulose composition having a temperature within the range of from 50 to 60°C is conveyed in the extruder, preferably without supplying additional heat by means of heating elements, whereas due to dissipation of heat the temperature of the composition at the extruder outlet may be lower than the temperature at the extruder inlet, whereas the temperature of the composition at the extruder outlet is preferably also within the range of from 50 to 60°C; and

- step (d) involves conveying in the extruder the allulose composition containing allulose and a gas phase containing water, wherein the gas phase has a gas pressure that is maintained below atmospheric pressure; preferably the gas pressure is (i) within the range of from 20 to 300 mbar, more preferably from 40 to 250 mbar, still more preferably from 60 to 200 mbar, and yet more preferably from 80 to 150 mbar; or (ii) within the range of from 800 to 950 mbar.

In other preferred embodiments of the process according to the invention, extrusion in step (d) is performed under non-evaporative conditions, whereas in preceding step (b) the allulose composition is further concentrated compared to the liquid allulose composition provided in step (a) by removing water under evaporative conditions.

Preferably, extrusion is performed under non-evaporative conditions, whereas

- in step (a) a liquid allulose composition is provided that has an allulose content of at least 70 wt.-%, preferably at least 72.5 wt.-%, relative to the total weight of the liquid allulose composition; such liquid allulose compositions are also referred to as *"allulose syrups"* that are stable solutions at room temperature, i.e. that do not tend to spontaneously crystallize over time; preferably in step (a) the provided liquid allulose composition has a water content within the range of from 20 to 30 wt.-% (preferably centrifuge discharge) relative to the total weight of the liquid allulose composition;

- in step (b) the liquid allulose composition is further concentrated under evaporative conditions at elevated temperature and/or reduced pressure thereby increasing the allulose content

  (i) relatively by at least 2.5 wt.-%, preferably by at least 5.0 wt.-%, still more preferably by at least 7.5 wt.- %, yet more preferably by at least 10 wt.-%, even more preferably by at least 12.5 wt.-%, most preferably by at least 15 wt.-%, and in particular by at least 17.5 wt.-%; in preferred embodiments preferably by at least 20 wt.-%, more preferably by at least 22.5 wt.-%, still more preferably by at least 25 wt.-%, yet more preferably by at least 27.5 wt.-%, even more preferably by at least 30 wt.-%; relative to the allulose content of the liquid allulose composition provided in step (a); and/or

  (ii) absolutely to at least 75 wt.-%, more preferably to at least 77.5 wt.-%, still more preferably at least 80 wt.-%, yet more preferably at least 82.5 wt.-%, even more preferably at least 85 wt.-%, most preferably at least 87.5 wt.-%, and in particular at least 90 wt.-%; in preferred embodiments preferably at least 91 wt.-%, preferably at least 92 wt.-%, preferably at least 93 wt.-%, preferably at least 94 wt.-%, preferably at least 95 wt.-%; in preferred embodiments preferably at least 91 wt.-%, preferably at least 92 wt.-%, preferably at least 93 wt.-%, preferably at least 94 wt.-%, preferably at least 95 wt.-%; relative to the total weight of the allulose composition;

  whereas the allulose composition obtained in step (b) is preferably oversaturated and has a temperature above room temperature (23°C), preferably within the range of from 50 to 60°C;

- in step (c) the allulose composition provided in step (a) or the further concentrated allulose composition obtained in step (b) is fed into an extruder;

- in step (d) the allulose composition is subjected to shearing forces in the extruder; preferably at elevated temperature,

preferably within the range of from 40 to 90°C, more preferably within the range of from 50 to 60°C; preferably in the presence of seed crystals of allulose;

- in step (e) a product allulose composition is obtained from the extruder, which preferably is present in form of (i) a solid allulose composition, (ii) an allulose suspension, (iii) an oversaturated allulose solution, or (iv) an allulose melt;

- in step (f) the product allulose composition obtained from the extruder in step (e) is preferably allowed to cool to room temperature (23°C); and depending upon its form, is allowed to solidify; preferably to precipitate and/or to crystallize; and

- in optional step (g) the product allulose composition obtained in step (e) or (f) is ground and/or post-died.

According to this preferred embodiment, there is no active evaporation during the extrusion. While there may be minimal evaporation due to flash evaporation / flash evaporation after extrusion, the extruder is not equipped with evaporation elements.

Another aspect of the disclosure which is not literally claimed relates to a process for the preparation of a solid allulose composition comprising the steps of

(a) providing a liquid allulose composition comprising allulose dissolved in water;
(b) optionally, heating the liquid allulose composition to an elevated temperature;
(c) feeding the allulose composition into an extruder;
(d) optionally, drying the allulose composition in the extruder under evaporative conditions;
(e) obtaining a solid allulose composition from the extruder; and
(g) optionally, grinding and/or post-drying the solid allulose composition.

Figure 1 shows the change of angle of rotation [α] in the course of dissolving and subsequently equilibrating crystalline allulose in pure water at room temperature.

Figure 2 shows the change of specific angle of rotation [α] [(°*ml) /(dm*g)] in the course of dissolving and subsequently equilibrating crystalline allulose compared to extruded allulose according to the invention in pure water at room temperature.

Allulose, also referred to as *"psicose",* is a ketohexose. For the purpose of the specification, allulose is preferably provided in form of the D-enantiomer, i.e. D-allulose (CAS no. 551-68-8), which in open chain Fischer projection has the following structure:

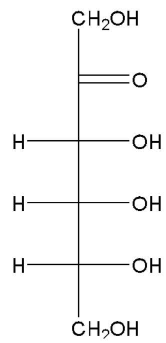

D-allulose

D-allulose can be present in form of the two enantiomers, D-allulose and L-allulose. According to the invention, allulose is present essentially only in form of the D-enantiomer. Thus, unless expressly stated otherwise, the term *"allulose"* as used herein refers to D-allulose (D-psicose).

Likewise, unless expressly stated otherwise, the term *"fructose"* as used herein refers to D-fructose.

Unless expressly stated otherwise, *"essentially"* refers to a quantitative degree of at least 99.0%, more preferably at least 99.5%, and in particular at least 99.9%.

Unless expressly stated otherwise, all conditions are ambient conditions, i.e. room temperature (23°C), 1013 hPa atmospheric pressure, and 50% relative humidity.

In order for a particulate product to emerge from a liquid, it is not sufficient to achieve appropriate thermodynamic states, because surface energy and kinetic factors also play an important role in the creation of the new particulate phase. Therefore, the point must be exceeded which is thermodynamically necessary for the creation of the new phase. If particles

are to be created from a solution, this excess is supersaturation, while for the creation of particles from a melt, undercooling is necessary.

When trying to obtain a particulate allulose product from a highly concentrated aqueous allulose solution (allulose syrup), various factors need to be taken into account, especially

- mass fraction of allulose in the starting material,
- temperature,
- pressure,
- presence or absence of nucleation sites,
- entropy reducing effects (e.g. shear forces),
- and the like.

Depending upon the given experimental conditions, the process can be regarded as a crystallization/precipitation of particles from solution; or the process can be regarded as a solidification from a melt. In reality, both processes may superimpose one another. Therefore, supersaturation as well as undercooling can both play an important role.

Under thermodynamic control (slow, low supersaturation), relatively few large crystals typically form from a solution (crystallization). Under kinetic control (fast, high supersaturation), a relatively large number of small particles are formed, which can be amorphous or crystalline (precipitation). When solidifying from a melt, a crystalline or amorphous solid forms, which can be monolithic or more or less particulate.

Experimental conditions cannot be freely chosen. For example, for economic and other reasons, the process needs to be performed in a timely manner at reasonable energy consumption. Thus, the residence time of the material in the equipment for performing the process is limited. Further, evaporation conditions should achieve a satisfactory evaporation rate of water typically requiring elevated temperature and/or reduced pressure. However, the properties of allulose also play an important role and highly depend upon the given temperature as well. Thus, process conditions need to be between the properties of water on the one hand and the properties of allulose on the other hand.

As the properties of allulose differ from the properties of other saccharides, experimental conditions that have been revealed to be suitable for one saccharide cannot be easily transferred to allulose. In other words, the binary system of allulose in water shows a specific behavior that differs from that of binary systems of other carbohydrates in water.

Expressed as a mass fraction, the solubility of allulose in water increases with increasing temperature. 291 g of allulose (74% solubility) dissolve in 100 g of water at 25 °C, and 489 g of allulose (83% solubility) at 50 °C. Allulose is less soluble in water than fructose and more soluble than glucose and lactose. Compared to invert sugar, allulose is more soluble in water at room temperature; the solubility is comparable at higher temperatures. Compared to sucrose, allulose is less soluble at temperatures below room temperature, at higher temperatures the solubility of allulose is better than that of sucrose.

In step (a) of the process according to the invention, a liquid allulose composition is provided comprising allulose dissolved in water.

Preferably, essentially the total amount of allulose comprised in the liquid allulose composition provided in step (a) is present in dissolved form.

Preferably, the liquid allulose composition is an allulose syrup.

Preferably, the liquid allulose composition is a highly concentrated aqueous solution essentially containing no undissolved material.

Preferably, the liquid allulose composition has a viscosity at room temperature (23°C) within the range of from 100 to 250 mPa·s.

In preferred embodiments, the liquid allulose composition provided in step (a) of the process according to the invention is a mother liquor that originates from a process for the crystallization of allulose from solution. As processes for the crystallization of allulose from solution have a comparatively low yield, they produce as byproducts significant volumes of mother liquor containing significant quantities of dissolved allulose which may then advantageously be solidified by means of the process according to the invention.

It has been surprisingly found that highly concentrated solutions of allulose, from which allulose cannot be crystallized (residual mother liquor), can advantageously be employed as starting material in the process according to the invention.

In preferred embodiments, the process for the crystallization of allulose from solution involves a separation of a crystalline phase comprising allulose crystals from the liquid mother liquor by centrifugation. Preferably, the liquid allulose

composition provided in step (a) of the process according to the invention originates from or is the centrifuge discharge.

Preferably, the total weight content of allulose, fructose and water of the liquid allulose composition provided in step (a) is at least 70 wt.-%, still more preferably at least 75 wt.-%, yet more preferably at least 80 wt.-%, even more preferably at least 85 wt.-%, and most preferably at least 90 wt.-%; in each case relative to the total weight of the liquid allulose composition.

Preferably, the liquid allulose composition essentially consists of allulose, fructose and water. Preferably, the total weight content of allulose, fructose and water of the liquid allulose composition provided in step (a) is at least 95 wt.-%; preferably at least 96 wt.-%, preferably at least 97 wt.-%, preferably at least 98 wt.-%, preferably at least 99 wt.-%, preferably at least 99.5 wt.-%; in each case relative to the total weight of the liquid allulose composition.

Preferably, the liquid allulose composition essentially contains essentially neither glucose nor sucrose. It is contemplated, however, that the liquid allulose composition may preferably contain up to 1.0 wt.-% glucose, more preferably up to 0.5 wt.-% glucose, relative to the total weight of the liquid allulose composition.

The liquid allulose composition provided in step (a) may be obtained by any one of the various methods for the preparation of allulose syrups that are known from the prior art. In this regard, reference is made to e.g. US 2018 049458, US 2018 255814, WO 2019 083069, WO 2019 088654, US 2019 029299, US 2019 297931, US 2019 328014, US 2020 085090.

Preferably, the liquid allulose composition, preferably allulose syrup, provided in step (a) has an allulose content (D-allulose) of at least 70 wt.-%, preferably at least 71 wt.-%, preferably at least 72 wt.-%, preferably at least 73 wt.-%, preferably at least 74 wt.-%, preferably at least 75 wt.-%, preferably at least 76 wt.-%, preferably at least 77 wt.-%, preferably at least 78 wt.-%, preferably at least 79 wt.-%, preferably at least 80 wt.-%, preferably at least 81 wt.-%, preferably at least 82 wt.-%, preferably at least 83 wt.-%, preferably at least 84 wt.-%, preferably at least 85 wt.-%, preferably at least 86 wt.-%, preferably at least 87 wt.-%, preferably at least 88 wt.-%, preferably at least 89 wt.-%, preferably at least 90 wt.-%; in each case relative to the total weight of the liquid allulose composition, preferably allulose syrup.

Preferably, the liquid allulose composition provided in step (a) has an allulose content of at least 73 wt.- %, more preferably at least 77 wt.-%, even more preferably at least 81 wt.-%, most preferably at least 85 wt.-%, and in particular at least 89 wt.-%, in each case relative to the total weight of the liquid allulose composition.

The solubility of allulose in water increases with increasing temperature. Therefore, when the allulose content of the liquid allulose composition is above the threshold concentration at which allulose would spontaneously crystallize from aqueous solution at room temperature (oversaturation), the liquid allulose composition is preferably provided at a temperature above room temperature so as to avoid spontaneous crystallization.

Preferably, the liquid allulose composition provided in step (a) has a water content of at least 2.5 wt.-%; preferably at least 5.0 wt.-%, more preferably at least 7.5 wt.-%, still more preferably at least 10 wt.-%, yet more preferably at least 12.5 wt.-%, even more preferably at least 15 wt.-%, most preferably at least 17.5 wt.-%, and in particular at least 20 wt.-%; in each case relative to the total weight of the liquid allulose composition.

Preferably, the liquid allulose composition provided in step (a) has a water content of at most 20 wt.-%, and in particular at most 10 wt.-%; in each case relative to the total weight of the liquid allulose composition.

Preferably, the liquid allulose composition provided in step (a) has a fructose content (D-fructose) of at most 10 wt.-%; preferably at most 9.0 wt.-%, preferably at most 8.0 wt.-%, preferably at most 7.0 wt.-%, preferably at most 6.0 wt.-%, preferably at most 5.0 wt.-%, preferably at most 4.0 wt.-%, preferably at most 3.0 wt.-%, preferably at most 2.0 wt.-%, preferably at most 1.0 wt.-%; in each case relative to the total weight of the liquid allulose composition.

Preferably, the liquid allulose composition provided in step (a) has a total content of carbohydrates other than allulose and fructose of at most 5.0 wt.-%; preferably at most 4.0 wt.-%, preferably at most 3.0 wt.-%, preferably at most 2.0 wt.-%, preferably at most 1.0 wt.-%; in each case relative to the total content of all carbohydrates that are contained in the liquid allulose composition.

Preferably, the process according to the invention is implemented as an integral part in an overall process for the production of a product allulose composition encompassing one or more preceding steps selected from the group consisting of

($a_1$) synthesizing allulose in a reactor; preferably from fructose; preferably under enzymatic catalysis;

($a_2$) withdrawing the product composition containing the synthesized allulose from the reactor;

($a_3$) desalting the product composition;

($a_4$) decoloring the product composition;

($a_5$) purifying the synthesized allulose that is contained in the product composition; preferably by chromatography;

($a_6$) filtrating the product compositions; preferably by nanofiltration or sterile filtration, more preferably sterile filtration;

($a_7$) concentrating the product composition; preferably (i) by evaporating water at elevated temperature and/or under reduced pressure and/or (ii) by reverse osmosis; and

($a_8$) decoloring the concentrated product composition.

As one or more of the above preceding steps are performed at elevated temperatures, i.e. above room temperature (23°C), the liquid allulose composition thus processed preferably is the liquid allulose composition that is provided in step (a) according to the invention. Thus, the heat supplied to the liquid allulose composition in the course of any one of the above preceding steps and the resultant elevated temperature of the liquid allulose composition is preferably used upon commencement of the process according to the invention. In other words, the liquid allulose composition that is obtained by any one of the above proceeding steps has preferably an elevated temperature anyway and is preferably not allowed to cool down to room temperature but is supplied to step (a) of the process according to the invention.

It has been surprisingly found that when implementing the process according to the invention as an integral part into an overall process for the production of a product allulose composition, especially the preceding step ($a_7$) of concentrating the product composition; preferably (i) by evaporating water at elevated temperature and/or under reduced pressure and/or (ii) by reverse osmosis; can be performed until an allulose concentration is reached in the liquid allulose composition that - when being allowed to cool down to room temperature - would result in spontaneous crystallization of allulose from the liquid allulose composition. However, as the elevated temperature of the liquid allulose composition is preferably maintained, the thus heated liquid allulose composition can be further processed in the process according to the invention without premature crystallization just due to the higher solubility of allulose in water at higher temperatures.

In step (b) of the process according to the invention, the liquid allulose composition is heated to an elevated temperature under evaporative conditions thereby reducing the content of water of the allulose composition.

In a preferred embodiment, step (b) is performed outside the extruder by means of suitable heating equipment known to the skilled person.

In another preferred embodiment, step (b) is partially or completely performed inside the extruder. Many commercially available extruders are equipped with heating sections that usually can be set at different temperatures in order to adjust predetermined temperature profiles. According to this preferred embodiment, the heating sections located upstream in the extruder are preferably used for performing step (b).

Preferably, in step (b) the elevated temperature is at least 30°C; preferably at least 31°C, preferably at least 32°C, preferably at least 33°C, preferably at least 34°C, preferably at least 35°C, preferably at least 36°C, preferably at least 37°C, preferably at least 38°C, preferably at least 39°C, preferably at least 40°C, preferably at least 41°C, preferably at least 42°C, preferably at least 43°C, preferably at least 44°C, preferably at least 45°C, preferably at least 46°C, preferably at least 47°C, preferably at least 48°C, preferably at least 49°C, preferably at least 50°C, preferably at least 51°C, preferably at least 52°C, preferably at least 53°C, preferably at least 54°C, preferably at least 55°C, preferably at least 56°C, preferably at least 57°C, preferably at least 58°C, preferably at least 59°C, preferably at least 60°C.

Preferably, in step (b) the elevated temperature is at least 35°C; preferably at least 40°C, more preferably at least 43°C, still more preferably at least 46°C, yet more preferably at least 49°C, even more preferably at least 52°C, most preferably at least 55°C, and in particular at least 58°C.

Preferably, in step (b) the elevated temperature is at most 64°C, preferably at most 63°C, preferably at most 62°C, preferably at most 61°C, preferably at most 60°C, preferably at most 59°C, preferably at most 58°C, preferably at most 57°C, preferably at most 56°C, preferably at most 55°C, preferably at most 54°C, preferably at most 53°C, preferably at most 52°C, preferably at most 51°C, preferably at most 50°C. Preferably, in step (b) the elevated temperature is below 60°C, more preferably at most 59°C.

Preferably, in step (b) the elevated temperature is at most 60°C, more preferably at most 57°C, yet more preferably at most 54°C, even more preferably at most 51°C, most preferably at most 48°C, and in particular at most 45°C.

In preferred embodiments, in step (b) the elevated temperature is within the range of 40±10°C, preferably 45 ±10°C, preferably 50±10°C, or preferably 55±10°C.

Preferably, in step (b) the elevated temperature is within the range of 53±12°C, still more preferably 53±10°C, yet more preferably 53±8.0°C, even more preferably 53±6.0°C, most 53±4.0°C, and in particular 53±3.0°C.

Step (b) is performed under evaporative conditions thereby reducing the content of water of the allulose composition, i.e. increasing the allulose content of the allulose composition.

In preferred embodiments, step (b) involves heating the allulose composition thereby producing a gas phase containing water, wherein the gas phase has a gas pressure that is maintained below atmospheric pressure.

Preferably, the gas pressure in step (b) is at most 800 mbar; preferably at most 750 mbar, preferably at most 700 mbar, preferably at most 650 mbar, preferably at most 600 mbar, preferably at most 550 mbar, preferably at most 500 mbar,

preferably at most 450 mbar, preferably at most 400 mbar, preferably at most 350 mbar, preferably at most 300 mbar, preferably at most 250 mbar, preferably at most 200 mbar, preferably at most 150 mbar, preferably at most 100 mbar, preferably at most 90 mbar. preferably at most 80 mbar, preferably at most 70 mbar, preferably at most 60 mbar, preferably at most 50 mbar, preferably at most 40 mbar, preferably at most 30 mbar, preferably at most 20 mbar, preferably at most 10 mbar.

Preferably, the gas pressure in step (b) is at least 40 mbar; preferably at least 60 mbar, more preferably at least 80 mbar, still more preferably at least 100 mbar, yet more preferably at least 120 mbar, even more preferably at least 140 mbar, most preferably at least 160 mbar, and in particular at least 180 mbar.

Preferably, the gas pressure in step (b) is at most 260 mbar; preferably at most 230 mbar, more preferably at most 200 mbar, still more preferably at most 170 mbar, yet more preferably at most 140 mbar, even more preferably at most 110 mbar, most preferably at most 80 mbar, and in particular at most 50 mbar.

Preferably, the gas pressure in step (b) is within the range of 450±400 mbar; preferably 400±350 mbar, more preferably 350±300 mbar, still more preferably 300±250 mbar, yet more preferably 250±200 mbar, even more preferably 200±150 mbar, most 150±100 mbar, and in particular 125±75 mbar.

Preferably, the allulose composition obtained in step (b) has a water content of at least 2.5 wt.-%; preferably at least 5.0 wt.-%, more preferably at least 7.5 wt.-%, still more preferably at least 10 wt.-%, yet more preferably at least 12.5 wt.-%, even more preferably at least 15 wt.-%, most preferably at least 17.5 wt.-%, and in particular at least 20 wt.-%; in each case relative to the total weight of the allulose composition. In other preferred embodiments, the allulose composition obtained in step (b) has a water content within the range of from 15 to 45 wt.-%, preferably 15 to 35 wt.-% (preferably allulose syrup), or 20 to 30 wt.-% (preferably centrifuge discharge), relative to the total weight of the liquid allulose composition.

Preferably, the allulose composition obtained in step (b) has a water content of at most 80 wt.-%; preferably at most 70 wt.-%, more preferably at most 60 wt.-%, still more preferably at most 50 wt.-%, yet more preferably at most 40 wt.-%, even more preferably at most 30 wt.-%, most preferably at most 20 wt.-%, and in particular at most 10 wt.-%; in each case relative to the total weight of the allulose composition.

Preferably, the allulose composition obtained in step (b) has a water content within the range of 70±30 wt.-%; preferably 70±25 wt.-%, more preferably 80±20 wt.-%, still more preferably 80±15 wt.-%, yet more preferably 85±15 wt.-%, even more preferably 85±10 wt.-%, most 90±10 wt.-%, and in particular 95±5.0 wt.-%; in each case relative to the total weight of the allulose composition.

Preferably, the allulose composition obtained in step (b) has a water content, relative to the total weight of the allulose composition, that is below the water content of the liquid allulose composition that is provided in step (a), relative to the total weight of the liquid allulose composition that is provided in step (a), whereas the relative difference is at least -2.5 wt.-%, preferably at least -5.0 wt.-%, more preferably at least -7.5 wt.-%, still more preferably at least -10 wt.-%, yet more preferably at least -12.5 wt.-%, even more preferably at least -15 wt.-%, most preferably at least -17.5 wt.-%, and in particular at least -20 wt.-%.

Preferably, the allulose composition obtained in step (b) has an allulose content has an allulose content of at least 65 wt.-%, preferably at least 66 wt.-%, preferably at least 67 wt.-%, preferably at least 68 wt.-%, preferably at least 69 wt.-%, preferably at least 70 wt.-%, preferably at least 71 wt.-%, preferably at least 72 wt.-%, preferably at least 73 wt.-%, preferably at least 74 wt.-%, preferably at least 75 wt.-%, preferably at least 76 wt.-%, preferably at least 77 wt.-%, preferably at least 78 wt.-%, preferably at least 79 wt.-%, preferably at least 80 wt.-%, preferably at least 81 wt.-%, preferably at least 82 wt.-%, preferably at least 83 wt.-%, preferably at least 84 wt.-%, preferably at least 85 wt.-%, preferably at least 86 wt.-%, preferably at least 87 wt.-%, preferably at least 88 wt.-%, preferably at least 89 wt.-%, preferably at least 90 wt.-%, preferably at least 91 wt.-%, preferably at least 92 wt.-%, preferably at least 93 wt.-%, preferably at least 94 wt.-%, preferably at least 95 wt.-%; in each case relative to the total weight of the allulose composition.

Preferably, the allulose composition obtained in step (b) has an allulose content of at least 61 wt.-%, preferably at least 65 wt.-%, more preferably at least 69 wt.-%, still more preferably at least 73 wt.-%, yet more preferably at least 77 wt.-%, even more preferably at least 81 wt.-%, most preferably at least 85 wt.-%, and in particular at least 89 wt.-%, in each case relative to the total weight of the allulose composition.

Preferably, the allulose composition obtained in step (b) has an allulose content, relative to the total weight of the allulose composition, that is greater than the allulose content of the liquid allulose composition that is provided in step (a), relative to the total weight of the liquid allulose composition that is provided in step (a), whereas the relative difference is at least 2.5 wt.-%, preferably at least 5.0 wt.-%, more preferably at least 7.5 wt.-%, still more preferably at least 10 wt.-%, yet more preferably at least 12.5 wt.-%, even more preferably at least 15 wt.- %, most preferably at least 17.5 wt.-%, and in particular at least 20 wt.-%.

In step (c) of the process according to the invention, the allulose composition is fed into an extruder. In general, extruders according to the invention are typically equipped with an inlet for feeding the starting material to be extruded into the extruder, and an outlet for withdrawing the extruded material from the extruder.

Commercial extruders are typically equipped with a feeding device (feeding module) for supply of material to be extruded, i.e. the feeding device is located at the extruder inlet. As according to the invention the allulose composition is fed into the extruder in a liquid state, the feeding device of the extruder is preferably adapted for receiving liquid or semi-liquid materials.

The extruder type is not particularly limited. Preferably, the extruder is a single screw extruder, twin screw extruder or planetary roller extruder (planetary gear extruder). Extruders of this type are known to the skilled person and commercially available. Various extruders are known to the skilled person that include both the combination with similar or other extruders as well as the sectional combination of different extruder systems in one extruder. The combination of extruder sections of the same system is likewise included. In other words, the term *"extruder"* as used herein refers to various types and designs of extruders, including modular, sectional, integral, in series, or otherwise.

In preferred embodiments, the extruder is a single screw extruder.

In preferred embodiments, the extruder is a twin screw extruder with contrarotating screw configuration or corotating screw configuration.

In other preferred embodiments, the extruder is a planetary roller extruder. Typically, planetary roller extruders are equipped with a number of planetary spindles rotating around a central spindle. The planetary spindles, like the central spindle, have a toothing, usually a helical toothing, in one possible exemplification in the form of an involute toothing.

The extruder housing of a planetary roller extruder may internally also be toothed, in one possible exemplification equipped with a toothed liner. The planetary spindles intermesh with both the central spindle and the extruder housing or the liner. Under the flank pressure of the toothing, the planetary spindles would be moved outwards in an axial direction if the axial pressure were not absorbed by a thrust bearing or a thrust ring. Conventional extruders preferably have the thrust ring at the end of the housing, such that the thrust ring is put in place when the associated housing is clamped/screwed with its above-described flange. The thrust ring preferably surrounds the central spindle with a greater or lesser interspace. The feedstock processed or treated in the planetary roller extruder, i.e. the allulose composition being extruded, is conveyed through the gap between thrust ring and central spindle.

Preferably, some extrusion units of planetary roller extruders have such planetary roller extruder sections. Each planetary roller extruder section has a central spindle, a housing, planetary spindles rotating on the central spindle and in the internally toothed housing. At the same time the thrust rings prevent, restrict, and/or minimize the planetary spindles from moving in the axial direction out of the housing. In this regard, the planetary spindles slide on or against the thrust rings. The thrust rings are held between the ends of the housings of the various planetary roller extruder sections. Between the thrust rings and the central spindle there typically is a gap, through which the feedstock, i.e. the allulose composition being extruded, from one extruder section passes into the next. A common central spindle is preferably provided for the planetary roller extruder sections. The formation of a gap between the thrust ring and the central spindle is beneficial if no toothing on the central spindle is provided in the gap. Some planetary roller extruders have various planetary roller extruder sections, whereas preferably a common central spindle extends through the sections (see e.g. US 2018 281 263).

Preferably, the extruder is equipped with a degassing section. Planetary roller extruders that are equipped with a degassing section are described e.g. in US 2020 001 502.

The extruder is preferably fitted with a barrel that can be heated to maintain the selected barrel temperature. The extruder outlet can also be fitted with a die face cutter to cut the extrudate into desired lengths.

Preferably, the extruder has an extrusion chamber in which extrusion is performed over a length of 3.0$\pm$2.5 m, more preferably 3.0$\pm$2.0 m, still more preferably 3.0$\pm$1.5 m, yet more preferably 3.0$\pm$1.0 m, even more preferably 3.0 $\pm$0.5 m.

Preferably, extrusion conditions are optimized with respect to shearing forces, use of specific transport elements, and/or use of specific back-mixing elements. These measures and elements are known to a skilled person. With respect to back-mixing elements reference is made to e.g. EP 0 919 127 A1. In an adjoining back-mixing zone, the extruder shafts have back-mixing elements or backward-acting screw sections with openings for the extruded mass to pass through during its forward transport movement. In the back-mixing zone, the extruded mass is particularly intimately mixed, which leads to an even distribution of air in the extruded mass.

Preferably, the extruder is equipped with kneading elements.

Preferably, the extruder is equipped with transport elements.

Preferably, the extruder is equipped with a screw or two screws each having a length to diameter ratio (L/D) within the range of from 5 to 50; preferably 10 to 40, or 15 to 35, or 20 to 30.

Preferably, the allulose composition that is fed into the extruder in step (c) has a temperature of at least 35°C; preferably at least 40°C, more preferably at least 43°C, still more preferably at least 46°C, yet more preferably at least 49°C, even more preferably at least 52°C, most preferably at least 55°C, and in particular at least 58°C.

Preferably, the allulose composition that is fed into the extruder in step (c) has a temperature of at most 70°C; preferably at most 65°C, more preferably at most 60°C, still more preferably at most 57°C, yet more preferably at most 54°C, even more preferably at most 51°C, most preferably at most 48°C, and in particular at most 45°C.

Preferably, the allulose composition that is fed into the extruder in step (c) has a temperature within the range of

53±20°C; preferably 53±15°C, more preferably 53±12°C, still more preferably 53±10°C, yet more preferably 53±8.0°C, even more preferably 53±6.0°C, most 53±4.0°C, and in particular 53±3.0°C.

Preferably, the extrusion temperature is within the range of from 50 to 60°C, wherein a liquid allulose composition having a temperature within the range of from 50 to 60°C is conveyed in the extruder, preferably without supplying additional heat by means of heating elements, whereas due to dissipation of heat the temperature of the composition at the extruder outlet may be lower than the temperature at the extruder inlet, whereas the temperature of the composition at the extruder outlet is preferably also within the range of from 50 to 60°C.

Preferably, the allulose composition that is fed into the extruder in step (c) has an allulose content of at least 65 wt.-%, preferably at least 66 wt.-%, preferably at least 67 wt.-%, preferably at least 68 wt.-%, preferably at least 69 wt.-%, preferably at least 70 wt.-%, preferably at least 71 wt.-%, preferably at least 72 wt.-%, preferably at least 73 wt.-%, preferably at least 74 wt.-%, preferably at least 75 wt.-%, preferably at least 76 wt.-%, preferably at least 77 wt.-%, preferably at least 78 wt.-%, preferably at least 79 wt.-%, preferably at least 80 wt.-%, preferably at least 81 wt.-%, preferably at least 82 wt.-%, preferably at least 83 wt.-%, preferably at least 84 wt.-%, preferably at least 85 wt.-%, preferably at least 86 wt.-%, preferably at least 87 wt.-%, preferably at least 88 wt.-%, preferably at least 89 wt.-%, preferably at least 90 wt.-%, preferably at least 91 wt.-%, preferably at least 92 wt.-%, preferably at least 93 wt.-%, preferably at least 94 wt.-%, preferably at least 95 wt.-%, preferably at least 96 wt.-%, preferably at least 97 wt.-%, preferably at least 98 wt.-%, preferably at least 99 wt.-%, preferably at least 91 wt.-%, preferably at least 92 wt.-%, preferably at least 93 wt.-%, preferably at least 94 wt.-%, preferably at least 95 wt.-%; in each case relative to the total weight of the allulose composition that is fed into the extruder.

Preferably, the allulose composition that is fed into the extruder in step (c) has an allulose content of at most 99.5 wt.-%; preferably at most 99.0 wt.-%, preferably at most 98.5 wt.-%, preferably at most 98.0 wt.-%, preferably at most 97.5 wt.-%, preferably at most 97.0 wt.-%, preferably at most 96.5 wt.-%, preferably at most 96.0 wt.-%, preferably at most 95.5 wt.-%, preferably at most 95.0 wt.-%; in each case relative to the total weight of the allulose composition that is fed into the extruder.

Preferably, the allulose composition that is fed into the extruder in step (c) has a water content of at least 2.5 wt.-%; relative to the total weight of the allulose composition that is fed into the extruder.

Preferably, the allulose composition that is fed into the extruder in step (c) has a water content of at most 4.0 wt.-%, preferably at most 3.0 wt.-%, and in particular at most 2.0 wt.-%; in each case relative to the total weight of the allulose composition that is fed into the extruder. Preferably, the allulose composition that is fed into the extruder in step (c) has a water content of from 3.0 to 5.0 wt.-%, relative to the total weight of the allulose composition that is fed into the extruder.

Preferably, the allulose composition that is fed into the extruder in step (c) is oversaturated compared to the saturation concentration of allulose in pure water at room temperature (23°C). When in step (c) the allulose composition is fed into the extruder at an elevated temperature, the allulose composition is preferably also oversaturated compared to the saturation concentration of allulose in pure water at said elevated temperature.

Preferably, the allulose composition that is fed into the extruder in step (c) has an allulose concentration that is at least 1.0 wt.-% above the saturation concentration of allulose in pure water at the given temperature, preferably at least 2.0 wt.-%, more preferably at least 3.0 wt.-%, still more preferably at least 4.0 wt.-%, yet more preferably at least 5.0 wt.-%, even more preferably at least 6.0 wt.-%, most preferably at least 7.0 wt.-%, and in particular at least 8.0 wt.-%. The saturation concentration of allulose in pure water at the given temperature can be determined by a skilled person by routine experimental test preferably employing seed crystals of crystalline allulose; when an allulose composition is oversaturated but crystallization is hindered, e.g. kinetically hindered, the addition of seed crystals typically induces crystallization. According to the specification, when to a given solution of allulose in pure water at a given temperature seed crystals are added and no crystallization occurs within 2 hours under constant conditions, said solution is not yet oversaturated.

Preferably, the allulose composition that is fed into the extruder in step (c) has an allulose content ("cont." in [wt.-%]), relative to the total weight of the allulose composition that is fed into the extruder, and a temperature ("T" in [°C]) according to any one of embodiments **$X^1$** to **$X^{56}$**:

| | **$X^1$** | **$X^2$** | **$X^3$** | **$X^4$** | **$X^5$** | **$X^6$** | **$X^7$** | **$X^8$** | **$X^9$** | **$X^{10}$** | **$X^{11}$** | **$X^{12}$** | **$X^{13}$** | **$X^{14}$** |
|---|---|---|---|---|---|---|---|---|---|---|---|---|---|---|
| cont. | ≥65 | ≥70 | ≥75 | ≥80 | ≥85 | ≥90 | ≥95 | ≥65 | ≥70 | ≥75 | ≥80 | ≥85 | ≥90 | ≥95 |
| T | ≥40 | ≥40 | ≥40 | ≥40 | ≥40 | ≥40 | ≥40 | ≥50 | ≥50 | ≥50 | ≥50 | ≥50 | ≥50 | ≥50 |
| | **$X^{15}$** | **$X^{16}$** | **$X^{17}$** | **$X^{18}$** | **$X^{19}$** | **$X^{20}$** | **$X^{21}$** | **$X^{22}$** | **$X^{23}$** | **$X^{24}$** | **$X^{25}$** | **$X^{26}$** | **$X^{27}$** | **$X^{28}$** |
| cont. | ≥65 | ≥70 | ≥75 | ≥80 | ≥85 | ≥90 | ≥95 | ≥65 | ≥70 | ≥75 | ≥80 | ≥85 | ≥90 | ≥95 |
| T | ≥60 | ≥60 | ≥60 | ≥60 | ≥60 | ≥60 | ≥60 | ≥70 | ≥70 | ≥70 | ≥70 | ≥70 | ≥70 | ≥70 |

(continued)

| | $X^{29}$ | $X^{30}$ | $X^{31}$ | $X^{32}$ | $X^{33}$ | $X^{34}$ | $X^{35}$ | $X^{36}$ | $X^{37}$ | $X^{38}$ | $X^{39}$ | $X^{40}$ | $X^{41}$ | $X^{42}$ |
|---|---|---|---|---|---|---|---|---|---|---|---|---|---|---|
| cont. | ≥65 | ≥70 | ≥75 | ≥80 | ≥85 | ≥90 | ≥95 | ≥65 | ≥70 | ≥75 | ≥80 | ≥85 | ≥90 | ≥95 |
| T | ≤90 | ≤90 | ≤90 | ≤90 | ≤90 | ≤90 | ≤90 | ≤80 | ≤80 | ≤80 | ≤80 | ≤80 | ≤80 | ≤80 |
| | $X^{43}$ | $X^{44}$ | $X^{45}$ | $X^{46}$ | $X^{47}$ | $X^{48}$ | $X^{49}$ | $X^{50}$ | $X^{51}$ | $X^{52}$ | $X^{53}$ | $X^{54}$ | $X^{55}$ | $X^{56}$ |
| cont. | ≥65 | ≥70 | ≥75 | ≥80 | ≥85 | ≥90 | ≥95 | ≥65 | ≥70 | ≥75 | ≥80 | ≥85 | ≥90 | ≥95 |
| T | ≤70 | ≤70 | ≤70 | ≤70 | ≤70 | ≤70 | ≤70 | ≤60 | ≤60 | ≤60 | ≤60 | ≤60 | ≤60 | ≤60 |

Preferably, the allulose composition that is fed into the extruder in step (c) has an allulose content ("cont." in [wt.-%]), relative to the total weight of the allulose composition that is fed into the extruder, and a temperature ("T" in [°C]) according to any one of embodiments $\mathbf{Y^1}$ to $\mathbf{Y^{91}}$:

| | | | | | | | | | | | | | | |
|---|---|---|---|---|---|---|---|---|---|---|---|---|---|---|
| | $Y^{1}$ | $Y^{2}$ | $Y^{3}$ | $Y^{4}$ | $Y^{5}$ | $Y^{6}$ | $Y^{7}$ | $Y^{8}$ | $Y^{9}$ | $Y^{10}$ | $Y^{11}$ | $Y^{12}$ | $Y^{13}$ | $Y^{14}$ |
| cont. | ≥65 | ≥70 | ≥75 | ≥80 | ≥85 | ≥90 | ≥95 | ≥65 | ≥70 | ≥75 | ≥80 | ≥85 | ≥90 | ≥95 |
| T | 40-90 | 40-90 | 40-90 | 40-90 | 40-90 | 40-90 | 40-90 | 50-90 | 50-90 | 50-90 | 50-90 | 50-90 | 50-90 | 50-90 |
| | $Y^{15}$ | $Y^{16}$ | $Y^{17}$ | $Y^{18}$ | $Y^{19}$ | $Y^{20}$ | $Y^{21}$ | $Y^{22}$ | $Y^{23}$ | $Y^{24}$ | $Y^{25}$ | $Y^{26}$ | $Y^{27}$ | $Y^{28}$ |
| cont. | ≥65 | ≥70 | ≥75 | ≥80 | ≥85 | ≥90 | ≥95 | ≥65 | ≥70 | ≥75 | ≥80 | ≥85 | ≥90 | ≥95 |
| T | 60-90 | 60-90 | 60-90 | 60-90 | 60-90 | 60-90 | 60-90 | 70-90 | 70-90 | 70-90 | 70-90 | 70-90 | 70-90 | 70-90 |
| | $Y^{29}$ | $Y^{30}$ | $Y^{31}$ | $Y^{32}$ | $Y^{33}$ | $Y^{34}$ | $Y^{35}$ | $Y^{36}$ | $Y^{37}$ | $Y^{38}$ | $Y^{39}$ | $Y^{40}$ | $Y^{41}$ | $Y^{42}$ |
| cont. | ≥65 | ≥70 | ≥75 | ≥80 | ≥85 | ≥90 | ≥95 | ≥65 | ≥70 | ≥75 | ≥80 | ≥85 | ≥90 | ≥95 |
| T | 40-80 | 40-80 | 40-80 | 40-80 | 40-80 | 40-80 | 40-80 | 50-80 | 50-80 | 50-80 | 50-80 | 50-80 | 50-80 | 50-80 |
| | $Y^{43}$ | $Y^{44}$ | $Y^{45}$ | $Y^{46}$ | $Y^{47}$ | $Y^{48}$ | $Y^{49}$ | $Y^{50}$ | $Y^{51}$ | $Y^{52}$ | $Y^{53}$ | $Y^{54}$ | $Y^{55}$ | $Y^{56}$ |
| cont. | ≥65 | ≥70 | ≥75 | ≥80 | ≥85 | ≥90 | ≥95 | ≥65 | ≥70 | ≥75 | ≥80 | ≥85 | ≥90 | ≥95 |
| T | 60-80 | 60-80 | 60-80 | 60-80 | 60-80 | 60-80 | 60-80 | 70-80 | 70-80 | 70-80 | 70-80 | 70-80 | 70-80 | 70-80 |
| | $Y^{57}$ | $Y^{58}$ | $Y^{59}$ | $Y^{60}$ | $Y^{61}$ | $Y^{62}$ | $Y^{63}$ | $Y^{64}$ | $Y^{65}$ | $Y^{66}$ | $Y^{67}$ | $Y^{68}$ | $Y^{69}$ | $Y^{70}$ |
| cont. | ≥65 | ≥70 | ≥75 | ≥80 | ≥85 | ≥90 | ≥95 | ≥65 | ≥70 | ≥75 | ≥80 | ≥85 | ≥90 | ≥95 |
| T | 40-70 | 40-70 | 40-70 | 40-70 | 40-70 | 40-70 | 40-70 | 50-70 | 50-70 | 50-70 | 50-70 | 50-70 | 50-70 | 50-70 |
| | $Y^{71}$ | $Y^{72}$ | $Y^{73}$ | $Y^{74}$ | $Y^{75}$ | $Y^{76}$ | $Y^{77}$ | $Y^{78}$ | Y79 | Y80 | Y81 | Y82 | Y83 | Y84 |
| cont. | ≥65 | ≥70 | ≥75 | ≥80 | ≥85 | ≥90 | ≥95 | ≥65 | ≥70 | ≥75 | ≥80 | ≥85 | ≥90 | ≥95 |
| T | 60-70 | 60-70 | 60-70 | 60-70 | 60-70 | 60-70 | 60-70 | 40-60 | 40-60 | 40-60 | 40-60 | 40-60 | 40-60 | 40-60 |
| | Y85 | Y86 | Y87 | Y88 | Y89 | Y90 | Y91 | | | | | | | |
| cont. | ≥65 | ≥70 | ≥75 | ≥80 | ≥85 | ≥90 | ≥95 | | | | | | | |
| T | 50-60 | 50-60 | 50-60 | 50-60 | 50-60 | 50-60 | 50-60 | | | | | | | |

In preferred embodiments, step (d) of the process according to the invention is performed under evaporative conditions and serves the purpose of drying the feed stock, i.e. the liquid allulose composition. According to these preferred embodiments, the extruder is capable of being used for extrusion drying, preferably heated extrusion drying.

For the purpose of the specification, extrusion under *"evaporative conditions"* are preferably conditions where evolved vapor, especially water vapor, is withdrawn from the extruder.

Heated extrusion drying is typically done by preferably continuously feeding the allulose composition to be dried into an inlet of an extruder fitted with a heated barrel and continuously collecting the dried material as it exits the extruder. The heated barrel of an extrusion drier heats the contents of the extruder to volatilize water contained in the material to be dried. The extruder barrel can be vented to allow the escape of water vapor.

Preferably, step (c) of the process according to the invention comprises feeding seed crystals into the extruder.

Preferably, the amount of seed crystals fed into the extruder in step (c) is at least 3.0 wt.-%; preferably at least 4.0 wt.-%, more preferably at least 5.0 wt.-%, still more preferably at least 6.0 wt.-%, yet more preferably at least 7.0 wt.-%, even more preferably at least 8.0 wt.-%, most preferably at least 9.0 wt.-%, and in particular at least 10 wt.-%; in each case relative to the total weight of the allulose composition.

Preferably, the amount of seed crystals fed into the extruder in step (c) is at most 24 wt.-%; preferably at most 22 wt.-%, more preferably at most 20 wt.-%, still more preferably at most 18 wt.-%, yet more preferably at most 16 wt.-%, even more preferably at most 14 wt.-%, most preferably at most 12 wt.-%, and in particular at most 10 wt.-%; in each case relative to the total weight of the allulose composition.

Preferably, the amount of seed crystals fed into the extruder in step (c) is within the range of 10±8.0 wt.- %; preferably 10±7.0 wt.-%, more preferably 10±6.0 wt.-%, still more preferably 10±5.0 wt.-%, yet more preferably 10±4.0 wt.-%, even more preferably 10±3.0 wt.-%, most 10±2.0 wt.-%, and in particular 10±1.0 wt.-%; in each case relative to the total weight of the allulose composition.

Preferably, the seed crystals have an average particle size of at least 12 μm, preferably at least 24 μm, more preferably at least 36 μm, still more preferably at least 48 μm, yet more preferably at least 75 μm, even more preferably at least 100 μm, most preferably at least 145 μm, and in particular at least 190 μm.

Preferably, the seed crystals have an average particle size of at most 190 μm, preferably at most 145 μm, more preferably at most 100 μm, still more preferably at most 75 μm, yet more preferably at most 48 μm, even more preferably at most 36 μm, most preferably at most 24 μm, and in particular at most 12 μm.

Preferably, the seed crystals have an average particle size within the range of 12±10 μm; preferably 12±8.0 μm, more preferably 12±7.0 μm, still more preferably 12±6.0 μm, yet more preferably 12±5.0 μm, even more preferably 12 ±4.0 μm, most 12±3.0 μm, and in particular 12±2.0 μm.

Preferably, the seed crystals have an average particle size within the range of 36±24 μm; preferably 36±18 μm, more preferably 36±14 μm, still more preferably 36±10 μm, yet more preferably 36±8.0 μm, even more preferably 36 ±6.0 μm, most 36±4.0 μm, and in particular 36±2.0 μm.

Preferably, the seed crystals have an average particle size within the range of 190±178 μm; preferably 190±154 μm, more preferably 190±96 μm, still more preferably 190±48 μm, yet more preferably 190±24 μm, even more preferably 190±12 μm, most 190±6.0 μm, and in particular 190±2.0 μm.

For the purpose of the specification, the average particle size is preferably expressed as the geometric mean diameter ($d_{gw}$) and is preferably determined by sieving analysis, preferably in accordance with American Society of Agricultural and Biological Engineers (ASABE), ANSI/ASAE S319.4 FEB2008 *"Method of Determining and Expressing Fineness of Feed Materials by Sieving".*

In step (d) of the process according to the invention, the allulose composition is extruded in the extruder. Preferably, the allulose composition is subjected to shearing forces in the extruder. In preferred embodiments, the allulose composition is dried in the extruder under evaporative conditions.

Preferably, in step (d) the extruder is operated in horizontal arrangement.

Preferably, in step (d) the extruder is operated at an elevated extrusion temperature.

Preferably, in step (d) the elevated extrusion temperature is at least 30°C; preferably at least 31°C, preferably at least 32°C, preferably at least 33°C, preferably at least 34°C, preferably at least 35°C, preferably at least 36°C, preferably at least 37°C, preferably at least 38°C, preferably at least 39°C, preferably at least 40°C, preferably at least 41°C, preferably at least 42°C, preferably at least 43°C, preferably at least 44°C, preferably at least 45°C, preferably at least 46°C, preferably at least 47°C, preferably at least 48°C, preferably at least 49°C, preferably at least 50°C, preferably at least 51°C, preferably at least 52°C, preferably at least 53°C, preferably at least 54°C, preferably at least 55°C, preferably at least 56°C, preferably at least 57°C, preferably at least 58°C, preferably at least 59°C, preferably at least 60°C.

Preferably, in step (d) the elevated extrusion temperature is at most 80°C; preferably at most 79°C, preferably at most 78°C, preferably at most 77°C, preferably at most 76°C, preferably at most 75°C, preferably at most 74°C, preferably at most 73°C, preferably at most 72°C. preferably at most 71°C, preferably at most 70°C, preferably at most 69°C, preferably at most 68°C, preferably at most 67°C, preferably at most 66°C, preferably at most 65°C, preferably at most 64°C, preferably at most 63°C, preferably at most 62°C, preferably at most 61°C, preferably at most 60°C, preferably at

most 59°C, preferably at most 58°C, preferably at most 57°C, preferably at most 56°C, preferably at most 55°C, preferably at most 54°C. preferably at most 53°C, preferably at most 52°C, preferably at most 51°C, preferably at most 50°C.

Preferably, in step (d) the elevated extrusion temperature is at least 35°C; preferably at least 40°C, more preferably at least 43°C, still more preferably at least 46°C, yet more preferably at least 49°C, even more preferably at least 52°C, most preferably at least 55°C, and in particular at least 58°C.

Preferably, in step (d) the elevated extrusion temperature is at most 70°C; preferably at most 65°C, more preferably at most 60°C, still more preferably at most 57°C, yet more preferably at most 54°C, even more preferably at most 51°C, most preferably at most 48°C, and in particular at most 45°C.

Preferably, in step (d) the elevated extrusion temperature is within the range of 53±20°C; preferably 53±15°C, more preferably 53±12°C, still more preferably 53±10°C, yet more preferably 53±8.0°C, even more preferably 53 ±6.0°C, most 53±4.0°C, and in particular 53±3.0°C.

Preferably, in step (d) the extrusion temperature is within the range of from 50 to 60°C, wherein a liquid allulose composition having a temperature within the range of from 50 to 60°C is conveyed in the extruder, preferably without supplying additional heat by means of heating elements, whereas due to dissipation of heat the temperature of the composition at the extruder outlet may be lower than the temperature at the extruder inlet, whereas the temperature of the composition at the extruder outlet is preferably also within the range of from 50 to 60°C.

Preferably, the extruder comprises a first temperature zone operated at temperature $T_1$, in direction of extrusion followed by a second temperature zone operated at temperature $T_2$, wherein $T_1 > T_2$, or $T_2 > T_1$.

Preferably, the extruder comprises a first temperature zone operated at temperature $T_1$, in direction of extrusion followed by a second temperature zone operated at temperature $T_2$, in direction of extrusion followed by a third temperature zone operated at temperature $T_3$, wherein $T_1 > T_2 > T_3$, or $T_1 > T_3 > T_2$, or $T_2 > T_1 > T_3$, or $T_2 > T_3 > T_1$, or $T_3 > T_1 > T_2$, or $T_3 > T_2 > T_1$.

It is principally also contemplated that in certain embodiments the feedstock, i.e. the allulose composition being extruded, may be cooled in the course of extrusion.

In preferred embodiment, the allulose composition being extruded is cooled in the course of extrusion such that the temperature of the allulose composition that is withdrawn from the extruder has a temperature below the temperature of the allulose composition that is fed into the extruder.

In preferred embodiments, the relative temperature difference between the temperature of the allulose composition that is withdrawn from the extruder compared to the temperature of the allulose composition that is fed into the extruder is at least -1.0°C, more preferably at least -2.0°C, still more preferably at least -3.0°C, yet more preferably at least -4.0°C, even more preferably at least -5.0°C, most preferably at least -6.0°C, and in particular at least -7.0°C.

In preferred embodiments, the relative temperature difference between the temperature of the allulose composition that is withdrawn from the extruder compared to the temperature of the allulose composition that is fed into the extruder is at least -8.0°C, more preferably at least -9.0°C, still more preferably at least -10°C, yet more preferably at least -12°C, even more preferably at least -14°C, most preferably at least -16°C, and in particular at least -18°C.

Extrusion preferably takes place in an extruder in which the feedstock, i.e. the mass to be extruded, is pressed through a die. The hole diameter of the die determines the particle diameter.

Preferably, the hole diameter of the die is at least 0.1 mm; preferably at least 0.2 mm, preferably at least 0.3 mm, preferably at least 0.4 mm. Preferably, the hole diameter of the die is at most 2.0 mm; preferably at most 1.8 mm, preferably at most 1.6 mm, preferably at most 1.4 mm, preferably at most 1.2 mm, preferably at most 1.0 mm. Preferably, the hole diameter of the die is in the range from 0.3 to 2 mm and in particular in the range from 0.4 to 1.0 mm.

Preferably, step (d) involves exerting the allulose composition in the extruder to an increased pressure.

Preferably, the increased pressure is at least 1.5 bar; preferably at least 2.0 bar, preferably at least 2.5 bar, preferably at least 3.0 bar, preferably at least 3.5 bar, preferably at least 4.0 bar, preferably at least 4.5 bar, preferably at least 5.0 bar, preferably at least 6.0 bar, preferably at least 7.0 bar, preferably at least 8.0 bar, preferably at least 9.0 bar, preferably at least 10 bar.

Preferably, in step (d) the mean residence time of the allulose composition in the extruder is at least 10 seconds; preferably at least 20 seconds, preferably at least 30 seconds, preferably at least 40 seconds, preferably at least 50 seconds, preferably at least 60 seconds, preferably at least 70 seconds, preferably at least 80 seconds, preferably at least 90 seconds, preferably at least 100 seconds, preferably at least 110 seconds, preferably at least 120 seconds.

Preferably, in step (d) the mean residence time of the allulose composition in the extruder is at most 1000 seconds; preferably at most 950 seconds, preferably at most 900 seconds, preferably at most 850 seconds, preferably at most 800 seconds, preferably at most 750 seconds, preferably at most 700 seconds, preferably at most 650 seconds, preferably at most 600 seconds, preferably at most 550 seconds, preferably at most 500 seconds, preferably at most 450 seconds, preferably at most 400 seconds, preferably at most 350 seconds, preferably at most 300 seconds.

Preferably, in step (d) the mean residence time of the allulose composition in the extruder is at least 2.0 seconds; preferably at least 4.0 seconds, more preferably at least 6.0 seconds, still more preferably at least 8.0 seconds, yet more preferably at least 10 seconds, even more preferably at least 15 seconds, most preferably at least 30 seconds, and in

particular at least 60 seconds.

Preferably, in step (d) the mean residence time of the allulose composition in the extruder is at most 1800 seconds; preferably at most 900 seconds, more preferably at most 600 seconds, still more preferably at most 540 seconds, yet more preferably at most 480 seconds, even more preferably at most 420 seconds, most preferably at most 360 seconds, and in particular at most 300 seconds.

Preferably, in step (d) the mean residence time of the allulose composition in the extruder is within the range of 320±240 seconds; preferably 160±120 seconds, more preferably 80±60 seconds, still more preferably 40±30 seconds, yet more preferably 20±15 seconds, even more preferably 10±7.5 seconds, most 7.5±5.0 seconds, and in particular 5.0 ±2.0 seconds.

In particularly preferred embodiments, in step (d) the mean residence time of the allulose composition in the extruder is within the range of from 5 to 10 minutes, i.e. 300 to 600 seconds.

Preferably, in step (d) the extruder is operated at a maximum torque of at least 10 Nm; preferably at least 20 Nm, preferably at least 30 Nm, preferably at least 40 Nm, preferably at least 50 Nm, preferably at least 60 Nm, preferably at least 70 Nm, preferably at least 80 Nm, preferably at least 90 Nm, preferably at least 100 Nm.

Preferably, in step (d) the extruder is operated at a maximum torque of at most 200 Nm; preferably at most 190 Nm, preferably at most 180 Nm, preferably at most 170 Nm, preferably at most 160 Nm, preferably at most 150 Nm, preferably at most 140 Nm, preferably at most 120 Nm.

Preferably, in step (d) the extruder is operated at a rotational speed of at least 5 rpm; preferably at least 10 rpm, preferably at least 15 rpm, preferably at least 20 rpm, preferably at least 25 rpm, preferably at least 30 rpm, preferably at least 35 rpm, preferably at least 40 rpm, preferably at least 50 rpm.

Preferably, in step (d) the extruder is operated at a rotational speed of at most 200 rpm; preferably at most 190 rpm, preferably at most 180 rpm, preferably at most 170 rpm, preferably at most 160 rpm, preferably at most 150 rpm, preferably at most 140 rpm, preferably at most 130 rpm, preferably at most 120 rpm, preferably at most 110 rpm, preferably at most 100 rpm.

In the course of the extrusion process, allulose preferably begins to precipitate and/or crystallize from solution. Preferably, the precipitate is essentially in a glassy amorphous, i.e. non-crystalline state. In a preferred embodiment, the solidified allulose composition may be regarded as a glass in which the various anomers of allulose that have been present in the liquid allulose composition have solidified. As allulose crystallizes from aqueous solution solely as β-pyranose and as under the extrusion conditions the liquid allulose has preferably not sufficient time to convert completely into the anomeric β-pyranose form, the extrusion step in the extruder preferably forces a significant amount of the allulose to solidify in a non-crystalline state.

In preferred embodiments, the allulose composition is extruded in the extruder under non-evaporative conditions, i.e. the extrusion as such preferably does not significantly alter the water content or the allulose content of the allulose composition being extruded. In this regard, it is contemplated that flash evaporation may occur after the extruded material has exited the extruder and that such flash evaporation may slightly reduce the water content or the allulose content of the allulose composition outside the extruder.

In other preferred embodiments, the allulose composition is dried in the extruder under evaporative conditions. Thus, in preferred embodiments, step (d) involves conveying in the extruder the allulose composition containing allulose and a gas phase containing water, wherein the gas phase has a gas pressure that is maintained below atmospheric pressure.

Preferably, the gas pressure in step (d) is at most 800 mbar; preferably at most 750 mbar, preferably at most 700 mbar, preferably at most 650 mbar, preferably at most 600 mbar, preferably at most 550 mbar, preferably at most 500 mbar, preferably at most 450 mbar, preferably at most 400 mbar, preferably at most 350 mbar, preferably at most 300 mbar, preferably at most 250 mbar, preferably at most 200 mbar, preferably at most 150 mbar, preferably at most 100 mbar, preferably at most 90 mbar. preferably at most 80 mbar, preferably at most 70 mbar, preferably at most 60 mbar, preferably at most 50 mbar, preferably at most 40 mbar, preferably at most 30 mbar, preferably at most 20 mbar, preferably at most 10 mbar.

Preferably, the gas pressure in step (d) is at least 40 mbar; preferably at least 60 mbar, more preferably at least 80 mbar, still more preferably at least 100 mbar, yet more preferably at least 120 mbar, even more preferably at least 140 mbar, most preferably at least 160 mbar, and in particular at least 180 mbar.

Preferably, the gas pressure in step (d) is at most 260 mbar; preferably at most 230 mbar, more preferably at most 200 mbar, still more preferably at most 170 mbar, yet more preferably at most 140 mbar, even more preferably at most 110 mbar, most preferably at most 80 mbar, and in particular at most 50 mbar.

Preferably, the gas pressure in step (d) is within the range of 450±400 mbar; preferably 400±350 mbar, more preferably 350±300 mbar, still more preferably 300±250 mbar, yet more preferably 250±200 mbar, even more preferably 200±150 mbar, most 150±100 mbar, and in particular 125±75 mbar.

Preferably, step (d) involves expanding the allulose composition in a compressed state into a vaporized state thereby evaporating water from the allulose composition.

Preferably, in step (d) at least a portion of the gas phase is separated from the allulose composition.

In step (e) of the process according to the invention, a product allulose composition, preferably solid allulose composition is obtained from the extruder.

For the purpose of the specification, the *"product allulose composition"* is the material exiting the outlet of the extruder. It may be a solid material as such, i.e. a solid allulose composition that exits the extruder in solid form, e.g. in form of a particulate material or powder. Alternatively, the product allulose composition may be a viscous mass, e.g. an allulose suspension, an oversaturated allulose solution, or an allulose melt, that subsequently spontaneously solidifies in the course of exiting the outlet of the extruder or thereafter, optionally after cooling to room temperature and/or optionally after a certain period of time but preferably within not more than 72 hours (e.g. after-crystallization, post-crystallization).

Therefore, the product allulose composition obtained from the extruder in step (e) is preferably selected from (i) a solid allulose composition, (ii) an allulose suspension, (iii) an oversaturated allulose solution, and (iv) an allulose melt.

When the product allulose composition obtained from the extruder in step (e) is a solid allulose composition, it may exit the extruder in form of a solidified extrusion strand of material or in form of a particulate material. While the product allulose composition may still have a significant water content, according to this embodiment the product allulose composition preferably forms a single solid phase but no liquid phase. Depending upon the temperature, the solid allulose composition may be a viscous mass that further solidifies upon cooling. In such state, the solid allulose composition may resemble an allulose melt. A skilled person recognizes that upon heating, a solid allulose composition transforms into an allulose melt, whereas melting may occur over a broad temperature range. Thus, there typically is no distinct temperature clearly discriminating a solid allulose composition from an allulose melt. For the purpose of the specification, an allulose melt is present when the temperature of the product allulose composition is above the melting point of pure crystalline allulose (96°C).

When the product allulose composition obtained from the extruder in step (e) is an allulose suspension, it forms a solid phase and a liquid phase, where solid particles are suspended in a liquid phase. For the purpose of the specification, an allulose suspension may be any product allulose composition having a solid phase and a liquid phase, irrespective of whether the particles are finely suspended in the liquid phase forming a stable suspension or whether the particles precipitate and clearly separate from the liquid phase. Preferably, the allulose suspension has a water content of at least 51 wt.-%, preferably at least 55 wt.-%.

When the product allulose composition obtained from the extruder in step (e) is an oversaturated allulose solution, it still does not contain a significant amount of solidified material, but preferably is present in a highly instable state that preferably after cooling to room temperature (23°C) has a pronounced tendency to spontaneously solidify.

When the product allulose composition obtained from the extruder in step (e) is an allulose melt, it forms a single phase but is present at a temperature above the melting point of pure crystalline allulose (96°C). When the allulose melt is allowed to cool to room temperature (23°C) it will typically convert into a solid allulose composition.

Preferably, the product allulose composition obtained from the extruder in step (e) is liquid, semi-solid, or pasty. Preferably, the allulose content of the liquid, semi-solid, or pasty composition obtained from the extruder in step (e) is higher than the allulose content of the allulose composition fed into the extruder in step (c). Preferably, the water content of the liquid, semi-solid, or pasty composition obtained from the extruder in step (e) is lower than the allulose content of the allulose composition fed into the extruder in step (c). In this regard, it is contemplated that flash evaporation may occur after the extruded material has exited the extruder and that such flash evaporation may slightly reduce the water content or the allulose content of the allulose composition outside the extruder.

Preferably, the product allulose composition solidifies completely, preferably precipitates and/or crystallizes after being obtained from the extruder. Preferably, the product allulose composition solidifies completely, preferably precipitates and/or crystallizes within a time of at most 76 hours, preferably at most 24 hours, more preferably at most 12 hours, still more preferably at most 60 minutes, yet more preferably at most 40 minutes, even more preferably at most 20 minutes, most preferably at most 15 minutes, and in particular at most 10 minutes.

When in preceding step (d) the allulose composition is dried in the extruder under evaporative conditions, vapor, especially water vapor, is withdrawn from the extruder thereby reducing the water content of the product allulose composition that is obtained in step (e) compared to the water content of the allulose composition that is fed into the extruder in step (c).

Preferably, the product allulose composition obtained in step (e) has a water content, relative to the total weight of the product allulose composition, that is lower than the water content of the allulose composition that is fed into the extruder in step (c), relative to the total weight of the allulose composition that is fed into the extruder in step (c), whereas the relative difference is at least -2.5 wt.-%, preferably at least -5.0 wt.-%, more preferably at least -7.5 wt.-%, still more preferably at least -10 wt.-%, yet more preferably at least -12.5 wt.-%, even more preferably at least -15 wt.-%, most preferably at least -17.5 wt.-%, and in particular at least -20 wt.-%.

Reducing the water content under evaporative conditions causes an increase of the allulose content relative to the total weight of the allulose composition.

Preferably, the product allulose composition obtained in step (e) has an allulose content, relative to the total

weight of the product allulose composition, that is greater than the allulose content of the allulose composition that is fed into the extruder in step (c), relative to the total weight of the allulose composition that is fed into the extruder in step (c), whereas the relative difference is at least 2.5 wt.-%, preferably at least 5.0 wt.-%, more preferably at least 7.5 wt.-%, still more preferably at least 10 wt.-%, yet more preferably at least 12.5 wt.-%, even more preferably at least 15 wt.-%, most preferably at least 17.5 wt.-%, and in particular at least 20 wt.-%.

Preferably, the evaporative conditions in step (d) are adjusted such that the above relative differences in water content and allulose content, respectively, are achieved. A skilled person can easily determine suitable evaporative conditions by routine experimental tests varying temperature, pressure (vacuum) and residence time. A skilled person immediately recognizes that higher evaporation rates can be achieved at higher temperatures, lower pressures and/or extended residence times.

Preferred operative conditions of the extrusion process according to the invention are compiled as embodiments **$A^1$** to **$A^{20}$** in the tables here below (in each case temperature of the allulose composition fed into the extruder: 61±12°C, preferably 61±11°C, more preferably 61±10°C, still more preferably 61±9.0°C, yet more preferably 61±8.0°C, even more preferably 61±7.0°C, most preferably 61±6.0°C, and in particular 61±5.0°C; temperature of the product allulose composition obtained from the extruder: 61±12°C, preferably 61±11°C, more preferably 61±10°C, still more preferably 61±9.0°C, yet more preferably 61±8.0°C, even more preferably 61±7.0°C, most preferably 61±6.0°C, and in particular 61±5.0°C; reduced pressure in the extruder: 50 to 200 mbar):

| | **$A^1$** | **$A^2$** | **$A^3$** | **$A^4$** | **$A^5$** |
|---|---|---|---|---|---|
| allulose content of allulose composition fed into the extruder [wt.-%] | 65-70 | 70-75 | 75-80 | 80-85 | 85-90 |
| allulose content of product allulose composition obtained from the extruder [wt.-] | 70-75 | 75-80 | 80-85 | 85-90 | 90-95 |
| | **$A^6$** | **$A^7$** | **$A^8$** | **$A^9$** | **$A^{10}$** |
| allulose content of allulose composition fed into the extruder [wt.-%] | 90-95 | 65-70 | 70-75 | 75-80 | 80-85 |
| allulose content of product allulose composition obtained from the extruder [wt.-%] | 95-100 | 75-80 | 80-85 | 85-90 | 90-95 |
| | **$A^{11}$** | **$A^{12}$** | **$A^{13}$** | **$A^{14}$** | **$A^{15}$** |
| allulose content of allulose composition fed into the extruder [wt.-%] | 85-90 | 65-70 | 70-75 | 75-80 | 80-85 |
| allulose content of product allulose composition obtained from the extruder [wt.-%] | 95-100 | 80-85 | 85-90 | 90-95 | 95-100 |
| | **$A^{16}$** | **$A^{17}$** | **$A^{18}$** | **$A^{19}$** | **$A^{20}$** |
| allulose content of allulose composition fed into the extruder [wt.-%] | 65-70 | 70-75 | 75-80 | 65-70 | 70-75 |
| allulose content of product allulose composition obtained from the extruder [wt.-%] | 85-90 | 90-95 | 95-100 | 90-95 | 95-100 |

Preferred operative conditions of the extrusion process according to the invention are compiled as embodiments **$B^1$** to **$B^{20}$** in the tables here below (in each case temperature of the allulose composition fed into the extruder: 61±12°C, preferably 61±11°C, more preferably 61±10°C, still more preferably 61±9.0°C, yet more preferably 61±8.0°C, even more preferably 61±7.0°C, most preferably 61±6.0°C, and in particular 61±5.0°C; temperature of the product allulose composition obtained from the extruder: 61±12°C, preferably 61±11°C, more preferably 61±10°C, still more preferably 61±9.0°C, yet more preferably 61±8.0°C, even more preferably 61±7.0°C, most preferably 61±6.0°C, and in particular 61±5.0°C; reduced pressure in the extruder: 50 to 100 mbar):

| | **$B^1$** | **$B^2$** | **$B^3$** | **$B^4$** | **$B^5$** |
|---|---|---|---|---|---|
| allulose content of allulose composition fed into the extruder [wt.-%] | 65-70 | 70-75 | 75-80 | 80-85 | 85-90 |

(continued)

| | $B^1$ | $B^2$ | $B^3$ | $B^4$ | $B^5$ |
|---|---|---|---|---|---|
| allulose content of product allulose composition obtained from the extruder [wt.-] | 70-75 | 75-80 | 80-85 | 85-90 | 90-95 |
| | $B^6$ | $B^7$ | $B^8$ | $B^9$ | $B^{10}$ |
| allulose content of allulose composition fed into the extruder [wt.-%] | 90-95 | 65-70 | 70-75 | 75-80 | 80-85 |
| allulose content of product allulose composition obtained from the extruder [wt.-%] | 95-100 | 75-80 | 80-85 | 85-90 | 90-95 |
| | $B^{11}$ | $B^{12}$ | $B^{13}$ | $B^{14}$ | $B^{15}$ |
| allulose content of allulose composition fed into the extruder [wt.-%] | 85-90 | 65-70 | 70-75 | 75-80 | 80-85 |
| allulose content of product allulose composition obtained from the extruder [wt.-%] | 95-100 | 80-85 | 85-90 | 90-95 | 95-100 |
| | $B^{16}$ | $B^{17}$ | $B^{18}$ | $B^{19}$ | $B^{20}$ |
| allulose content of allulose composition fed into the extruder [wt.-%] | 65-70 | 70-75 | 75-80 | 65-70 | 70-75 |
| allulose content of product allulose composition obtained from the extruder [wt.-%] | 85-90 | 90-95 | 95-100 | 90-95 | 95-100 |

Preferred operative conditions of the extrusion process according to the invention are compiled as embodiments **$C^1$** to **$C^{20}$** in the tables here below (in each case temperature of the allulose composition fed into the extruder: 61±12°C, preferably 61±11°C, more preferably 61±10°C, still more preferably 61±9.0°C, yet more preferably 61±8.0°C, even more preferably 61±7.0°C, most preferably 61±6.0°C, and in particular 61±5.0°C; temperature of the product allulose composition obtained from the extruder: 61±12°C, preferably 61±11°C, more preferably 61±10°C, still more preferably 61±9.0°C, yet more preferably 61±8.0°C, even more preferably 61±7.0°C, most preferably 61±6.0°C, and in particular 61±5.0°C; reduced pressure in the extruder: 100 to 150 mbar):

| | $C^1$ | $C^2$ | $C^3$ | $C^4$ | $C^5$ |
|---|---|---|---|---|---|
| allulose content of allulose composition fed into the extruder [wt.-%] | 65-70 | 70-75 | 75-80 | 80-85 | 85-90 |
| allulose content of product allulose composition obtained from the extruder [wt.-] | 70-75 | 75-80 | 80-85 | 85-90 | 90-95 |
| | $C^6$ | $C^7$ | $C^8$ | $C^9$ | $C^{10}$ |
| allulose content of allulose composition fed into the extruder [wt.-%] | 90-95 | 65-70 | 70-75 | 75-80 | 80-85 |
| allulose content of product allulose composition obtained from the extruder [wt.-%] | 95-100 | 75-80 | 80-85 | 85-90 | 90-95 |
| | $C^{11}$ | $C^{12}$ | $C^{13}$ | $C^{14}$ | $C^{15}$ |
| allulose content of allulose composition fed into the extruder [wt.-%] | 85-90 | 65-70 | 70-75 | 75-80 | 80-85 |
| allulose content of product allulose composition obtained from the extruder [wt.-%] | 95-100 | 80-85 | 85-90 | 90-95 | 95-100 |
| | $C^{16}$ | $C^{17}$ | $C^{18}$ | $C^{19}$ | $C^{20}$ |
| allulose content of allulose composition fed into the extruder [wt.-%] | 65-70 | 70-75 | 75-80 | 65-70 | 70-75 |

(continued)

| | $C^{16}$ | $C^{17}$ | $C^{18}$ | $C^{19}$ | $C^{20}$ |
|---|---|---|---|---|---|
| allulose content of product allulose composition obtained from the extruder [wt.-%] | 85-90 | 90-95 | 95-100 | 90-95 | 95-100 |

Preferred operative conditions of the extrusion process according to the invention are compiled as embodiments **$D^1$** to **$D^{20}$** in the tables here below (in each case temperature of the allulose composition fed into the extruder: 61±12°C, preferably 61±11°C, more preferably 61±10°C, still more preferably 61±9.0°C, yet more preferably 61±8.0°C, even more preferably 61±7.0°C, most preferably 61±6.0°C, and in particular 61±5.0°C; temperature of the product allulose composition obtained from the extruder: 61±12°C, preferably 61±11°C, more preferably 61±10°C, still more preferably 61±9.0°C, yet more preferably 61±8.0°C, even more preferably 61±7.0°C, most preferably 61±6.0°C, and in particular 61±5.0°C; reduced pressure in the extruder: 150 to 200 mbar):

| | $D^1$ | $D^2$ | $D^3$ | $D^4$ | $D^5$ |
|---|---|---|---|---|---|
| allulose content of allulose composition fed into the extruder [wt.-%] | 65-70 | 70-75 | 75-80 | 80-85 | 85-90 |
| allulose content of product allulose composition obtained from the extruder [wt.-] | 70-75 | 75-80 | 80-85 | 85-90 | 90-95 |
| | $D^6$ | $D^7$ | $D^8$ | $D^9$ | $D^{10}$ |
| allulose content of allulose composition fed into the extruder [wt.-%] | 90-95 | 65-70 | 70-75 | 75-80 | 80-85 |
| allulose content of product allulose composition obtained from the extruder [wt.-%] | 95-100 | 75-80 | 80-85 | 85-90 | 90-95 |
| | $D^{11}$ | $D^{12}$ | $D^{13}$ | $D^{14}$ | $D^{15}$ |
| allulose content of allulose composition fed into the extruder [wt.-%] | 85-90 | 65-70 | 70-75 | 75-80 | 80-85 |
| allulose content of product allulose composition obtained from the extruder [wt.-%] | 95-100 | 80-85 | 85-90 | 90-95 | 95-100 |
| | $D^{16}$ | $D^{17}$ | $D^{18}$ | $D^{19}$ | $D^{20}$ |
| allulose content of allulose composition fed into the extruder [wt.-%] | 65-70 | 70-75 | 75-80 | 65-70 | 70-75 |
| allulose content of product allulose composition obtained from the extruder [wt.-%] | 85-90 | 90-95 | 95-100 | 90-95 | 95-100 |

Preferred operative conditions of the extrusion process according to the invention are compiled as embodiments **$E^1$** to **$E^{20}$** in the tables here below (in each case temperature of the allulose composition at the extruder inlet is higher than temperature of the product allulose composition at the extruder outlet, preferably by not more than 10°C; reduced pressure in the extruder: 50 to 200 mbar):

| | $E^1$ | $E^2$ | $E^3$ | $E^4$ | $E^5$ |
|---|---|---|---|---|---|
| allulose content of allulose composition fed into the extruder [wt.-%] | 65-70 | 70-75 | 75-80 | 80-85 | 85-90 |
| allulose content of product allulose composition obtained from the extruder [wt.-] | 70-75 | 75-80 | 80-85 | 85-90 | 90-95 |
| | $E^6$ | $E^7$ | $E^8$ | $E^9$ | $E^{10}$ |
| allulose content of allulose composition fed into the extruder [wt.-%] | 90-95 | 65-70 | 70-75 | 75-80 | 80-85 |

(continued)

| | $E^6$ | $E^7$ | $E^8$ | $E^9$ | $E^{10}$ |
|---|---|---|---|---|---|
| allulose content of product allulose composition obtained from the extruder [wt.-%] | 95-100 | 75-80 | 80-85 | 85-90 | 90-95 |
| | $E^{11}$ | $E^{12}$ | $E^{13}$ | $E^{14}$ | $E^{15}$ |
| allulose content of allulose composition fed into the extruder [wt.-%] | 85-90 | 65-70 | 70-75 | 75-80 | 80-85 |
| allulose content of product allulose composition obtained from the extruder [wt.-%] | 95-100 | 80-85 | 85-90 | 90-95 | 95-100 |
| | $E^{16}$ | $E^{17}$ | $E^{18}$ | $E^{19}$ | $E^{20}$ |
| allulose content of allulose composition fed into the extruder [wt.-%] | 65-70 | 70-75 | 75-80 | 65-70 | 70-75 |
| allulose content of product allulose composition obtained from the extruder [wt.-%] | 85-90 | 90-95 | 95-100 | 90-95 | 95-100 |

Preferred operative conditions of the extrusion process according to the invention are compiled as embodiments $\mathbf{F^1}$ to $\mathbf{F^{20}}$ in the tables here below (in each case temperature of the allulose composition at the extruder inlet is higher than temperature of the product allulose composition at the extruder outlet, preferably by not more than 10°C; reduced pressure in the extruder: 50 to 100 mbar):

| | $F^1$ | $F^2$ | $F^3$ | $F^4$ | $F^5$ |
|---|---|---|---|---|---|
| allulose content of allulose composition fed into the extruder [wt.-%] | 65-70 | 70-75 | 75-80 | 80-85 | 85-90 |
| allulose content of product allulose composition obtained from the extruder [wt.-] | 70-75 | 75-80 | 80-85 | 85-90 | 90-95 |
| | $F^6$ | $F^7$ | $F^8$ | $F^9$ | $F^{10}$ |
| allulose content of allulose composition fed into the extruder [wt.-%] | 90-95 | 65-70 | 70-75 | 75-80 | 80-85 |
| allulose content of product allulose composition obtained from the extruder [wt.-%] | 95-100 | 75-80 | 80-85 | 85-90 | 90-95 |
| | $F^{11}$ | $F^{12}$ | $F^{13}$ | $F^{14}$ | $F^{15}$ |
| allulose content of allulose composition fed into the extruder [wt.-%] | 85-90 | 65-70 | 70-75 | 75-80 | 80-85 |
| allulose content of product allulose composition obtained from the extruder [wt.-%] | 95-100 | 80-85 | 85-90 | 90-95 | 95-100 |
| | $F^{16}$ | $F^{17}$ | $F^{18}$ | $F^{19}$ | $F^{20}$ |
| allulose content of allulose composition fed into the extruder [wt.-%] | 65-70 | 70-75 | 75-80 | 65-70 | 70-75 |
| allulose content of product allulose composition obtained from the extruder [wt.-%] | 85-90 | 90-95 | 95-100 | 90-95 | 95-100 |

Preferred operative conditions of the extrusion process according to the invention are compiled as embodiments $\mathbf{G^1}$ to $\mathbf{G^{20}}$ in the tables here below (in each case temperature of the allulose composition at the extruder inlet is higher than temperature of the product allulose composition at the extruder outlet, preferably by not more than 10°C; reduced pressure in the extruder: 100 to 150 mbar):

| | G[1] | G[2] | G[3] | G[4] | G[5] |
|---|---|---|---|---|---|
| allulose content of allulose composition fed into the extruder [wt.-%] | 65-70 | 70-75 | 75-80 | 80-85 | 85-90 |
| allulose content of product allulose composition obtained from the extruder [wt.-] | 70-75 | 75-80 | 80-85 | 85-90 | 90-95 |
| | **G[6]** | **G[7]** | **G[8]** | **G[9]** | **G[10]** |
| allulose content of allulose composition fed into the extruder [wt.-%] | 90-95 | 65-70 | 70-75 | 75-80 | 80-85 |
| allulose content of product allulose composition obtained from the extruder [wt.-%] | 95-100 | 75-80 | 80-85 | 85-90 | 90-95 |
| | **G[11]** | **G[12]** | **G[13]** | **G[14]** | **G[15]** |
| allulose content of allulose composition fed into the extruder [wt.-%] | 85-90 | 65-70 | 70-75 | 75-80 | 80-85 |
| allulose content of product allulose composition obtained from the extruder [wt.-%] | 95-100 | 80-85 | 85-90 | 90-95 | 95-100 |
| | **G[16]** | **G[17]** | **G[18]** | **G[19]** | **G[20]** |
| allulose content of allulose composition fed into the extruder [wt.-%] | 65-70 | 70-75 | 75-80 | 65-70 | 70-75 |
| allulose content of product allulose composition obtained from the extruder [wt.-%] | 85-90 | 90-95 | 95-100 | 90-95 | 95-100 |

Preferred operative conditions of the extrusion process according to the invention are compiled as embodiments **H[1]** to **H[20]** in the tables here below (in each case temperature of the allulose composition at the extruder inlet is higher than temperature of the product allulose composition at the extruder outlet, preferably by not more than 10°C; reduced pressure in the extruder: 150 to 200 mbar):

| | H[1] | H[2] | H[3] | H[4] | H[5] |
|---|---|---|---|---|---|
| allulose content of allulose composition fed into the extruder [wt.-%] | 65-70 | 70-75 | 75-80 | 80-85 | 85-90 |
| allulose content of product allulose composition obtained from the extruder [wt.-] | 70-75 | 75-80 | 80-85 | 85-90 | 90-95 |
| | **H[6]** | **H[7]** | **H[8]** | **H[9]** | **H[10]** |
| allulose content of allulose composition fed into the extruder [wt.-%] | 90-95 | 65-70 | 70-75 | 75-80 | 80-85 |
| allulose content of product allulose composition obtained from the extruder [wt.-%] | 95-100 | 75-80 | 80-85 | 85-90 | 90-95 |
| | **H[11]** | **H[12]** | **H[13]** | **H[14]** | **H[15]** |
| allulose content of allulose composition fed into the extruder [wt.-%] | 85-90 | 65-70 | 70-75 | 75-80 | 80-85 |
| allulose content of product allulose composition obtained from the extruder [wt.-%] | 95-100 | 80-85 | 85-90 | 90-95 | 95-100 |
| | **H[16]** | **H[17]** | **H[18]** | **H[19]** | **H[20]** |
| allulose content of allulose composition fed into the extruder [wt.-%] | 65-70 | 70-75 | 75-80 | 65-70 | 70-75 |
| allulose content of product allulose composition obtained from the extruder [wt.-%] | 85-90 | 90-95 | 95-100 | 90-95 | 95-100 |

Preferred operative conditions of the extrusion process according to the invention are compiled as embodiments **$I^1$** to **$I^{20}$** in the tables here below (in each case temperature of the allulose composition at the extruder inlet is lower than temperature of the product allulose composition at the extruder outlet, preferably by not more than 10°C; reduced pressure in the extruder: 50 to 200 mbar):

| | **$I^1$** | **$I^2$** | **$I^3$** | **$I^4$** | **$I^5$** |
|---|---|---|---|---|---|
| allulose content of allulose composition fed into the extruder [wt.-%] | 65-70 | 70-75 | 75-80 | 80-85 | 85-90 |
| allulose content of product allulose composition obtained from the extruder [wt.-] | 70-75 | 75-80 | 80-85 | 85-90 | 90-95 |
| | **$I^6$** | **$I^7$** | **$I^8$** | **$I^9$** | **$I^{10}$** |
| allulose content of allulose composition fed into the extruder [wt.-%] | 90-95 | 65-70 | 70-75 | 75-80 | 80-85 |
| allulose content of product allulose composition obtained from the extruder [wt.-%] | 95-100 | 75-80 | 80-85 | 85-90 | 90-95 |
| | **$I^{11}$** | **$I^{12}$** | **$I^{13}$** | **$I^{14}$** | **$I^{15}$** |
| allulose content of allulose composition fed into the extruder [wt.-%] | 85-90 | 65-70 | 70-75 | 75-80 | 80-85 |
| allulose content of product allulose composition obtained from the extruder [wt.-%] | 95-100 | 80-85 | 85-90 | 90-95 | 95-100 |
| | **$I^{16}$** | **$I^{17}$** | **$I^{18}$** | **$I^{19}$** | **$I^{20}$** |
| allulose content of allulose composition fed into the extruder [wt.-%] | 65-70 | 70-75 | 75-80 | 65-70 | 70-75 |
| allulose content of product allulose composition obtained from the extruder [wt.-%] | 85-90 | 90-95 | 95-100 | 90-95 | 95-100 |

Preferred operative conditions of the extrusion process according to the invention are compiled as embodiments **$J^1$** to **$J^{20}$** in the tables here below (in each case temperature of the allulose composition at the extruder inlet is lower than temperature of the product allulose composition at the extruder outlet, preferably by not more than 10°C; reduced pressure in the extruder: 50 to 100 mbar):

| | **$J^1$** | **$J^2$** | **$J^3$** | **$J^4$** | **$J^5$** |
|---|---|---|---|---|---|
| allulose content of allulose composition fed into the extruder [wt.-%] | 65-70 | 70-75 | 75-80 | 80-85 | 85-90 |
| allulose content of product allulose composition obtained from the extruder [wt.-] | 70-75 | 75-80 | 80-85 | 85-90 | 90-95 |
| | **$J^6$** | **$J^7$** | **$J^8$** | **$J^9$** | **$J^{10}$** |
| allulose content of allulose composition fed into the extruder [wt.-%] | 90-95 | 65-70 | 70-75 | 75-80 | 80-85 |
| allulose content of product allulose composition obtained from the extruder [wt.-%] | 95-100 | 75-80 | 80-85 | 85-90 | 90-95 |
| | **$J^{11}$** | **$J^{12}$** | **$J^{13}$** | **$J^{14}$** | **$J^{15}$** |
| allulose content of allulose composition fed into the extruder [wt.-%] | 85-90 | 65-70 | 70-75 | 75-80 | 80-85 |
| allulose content of product allulose composition obtained from the extruder [wt.-%] | 95-100 | 80-85 | 85-90 | 90-95 | 95-100 |

(continued)

| | $J^{16}$ | $J^{17}$ | $J^{18}$ | $J^{19}$ | $J^{20}$ |
|---|---|---|---|---|---|
| allulose content of allulose composition fed into the extruder [wt.-%] | 65-70 | 70-75 | 75-80 | 65-70 | 70-75 |
| allulose content of product allulose composition obtained from the extruder [wt.-%] | 85-90 | 90-95 | 95-100 | 90-95 | 95-100 |

Preferred operative conditions of the extrusion process according to the invention are compiled as embodiments $\mathbf{K^1}$ to $\mathbf{K^{20}}$ in the tables here below (in each case temperature of the allulose composition at the extruder inlet is lower than temperature of the product allulose composition at the extruder outlet, preferably by not more than 10°C; reduced pressure in the extruder: 100 to 150 mbar):

| | $K^1$ | $K^2$ | $K^3$ | $K^4$ | $K^5$ |
|---|---|---|---|---|---|
| allulose content of allulose composition fed into the extruder [wt.-%] | 65-70 | 70-75 | 75-80 | 80-85 | 85-90 |
| allulose content of product allulose composition obtained from the extruder [wt.-] | 70-75 | 75-80 | 80-85 | 85-90 | 90-95 |
| | $K^6$ | $K^7$ | $K^8$ | $K^9$ | $K^{10}$ |
| allulose content of allulose composition fed into the extruder [wt.-%] | 90-95 | 65-70 | 70-75 | 75-80 | 80-85 |
| allulose content of product allulose composition obtained from the extruder [wt.-%] | 95-100 | 75-80 | 80-85 | 85-90 | 90-95 |
| | $K^{11}$ | $K^{12}$ | $K^{13}$ | $K^{14}$ | $K^{15}$ |
| allulose content of allulose composition fed into the extruder [wt.-%] | 85-90 | 65-70 | 70-75 | 75-80 | 80-85 |
| allulose content of product allulose composition obtained from the extruder [wt.-%] | 95-100 | 80-85 | 85-90 | 90-95 | 95-100 |
| | $K^{16}$ | $K^{17}$ | $K^{18}$ | $K^{19}$ | $K^{20}$ |
| allulose content of allulose composition fed into the extruder [wt.-%] | 65-70 | 70-75 | 75-80 | 65-70 | 70-75 |
| allulose content of product allulose composition obtained from the extruder [wt.-%] | 85-90 | 90-95 | 95-100 | 90-95 | 95-100 |

Preferred operative conditions of the extrusion process according to the invention are compiled as embodiments $\mathbf{L^1}$ to $\mathbf{L^{20}}$ in the tables here below (in each case temperature of the allulose composition at the extruder inlet is lower than temperature of the product allulose composition at the extruder outlet, preferably by not more than 10°C; reduced pressure in the extruder: 150 to 200 mbar):

| | $L^1$ | $L^2$ | $L^3$ | $L^4$ | $L^5$ |
|---|---|---|---|---|---|
| allulose content of allulose composition fed into the extruder [wt.-%] | 65-70 | 70-75 | 75-80 | 80-85 | 85-90 |
| allulose content of product allulose composition obtained from the extruder [wt.-] | 70-75 | 75-80 | 80-85 | 85-90 | 90-95 |
| | $L^6$ | $L^7$ | $L^8$ | $L^9$ | $L^{10}$ |
| allulose content of allulose composition fed into the extruder [wt.-%] | 90-95 | 65-70 | 70-75 | 75-80 | 80-85 |
| allulose content of product allulose composition obtained from the extruder [wt.-%] | 95-100 | 75-80 | 80-85 | 85-90 | 90-95 |

(continued)

| | $L^{11}$ | $L^{12}$ | $L^{13}$ | $L^{14}$ | $L^{15}$ |
|---|---|---|---|---|---|
| allulose content of allulose composition fed into the extruder [wt.-%] | 85-90 | 65-70 | 70-75 | 75-80 | 80-85 |
| allulose content of product allulose composition obtained from the extruder [wt.-%] | 95-100 | 80-85 | 85-90 | 90-95 | 95-100 |
| | $L^{16}$ | $L^{17}$ | $L^{18}$ | $L^{19}$ | $L^{20}$ |
| allulose content of allulose composition fed into the extruder [wt.-%] | 65-70 | 70-75 | 75-80 | 65-70 | 70-75 |
| allulose content of product allulose composition obtained from the extruder [wt.-%] | 85-90 | 90-95 | 95-100 | 90-95 | 95-100 |

Preferably, the extruded feedstock strand, i.e. the product allulose composition leaving the extruder breaks up into short granule-like particles or can likewise be broken with the help of suitable cutting devices. The granule particles obtained in this way preferably have a homogeneous grain size, i.e. a narrow grain size distribution.

In step (f) of the process according to the invention, the product allulose composition is allowed to solidify, preferably precipitate and/or crystallize. This may take several hours and even days.

Preferably, step (f) involves allowing the product allulose composition to cool to room temperature (23°C).

If the product allulose composition is present in form of two phases, i.e. a solid phase and a liquid phase (allulose suspension), step (f) may involve separation of the two phases, e.g. by sieving and or filtering. The liquid phase is then preferably recycled into the process, preferably to step (a).

In optional step (g) of the process according to the invention, the product allulose composition is ground and/or post-dried.

In a preferred embodiment, the extruded granules of the product allulose composition are rounded, i.e. to spheronized. This reduces the formation of undesired dust particles in the end product, in particular.

Preferred devices used for the rounding of the granules are spheronizers, which essentially have a horizontally rotating disc onto which the extrudates are pressed as a result of the centrifugal force onto the wall. The extrudates break at the micronotches pregiven by the extrusion process, such that cylindrical particles are formed. As a result of the mechanical loading in the spheronizer, the initially cylindrical particles become somewhat rounded.

Depending upon the extrusion conditions, the extruded product allulose composition may still have a comparatively high water content, which may be at least 15 wt.-%, for example in the range of from 15 to 50 wt.-%, in particular in the range of from 20 to 45 wt.-%, based on the total weight of the product allulose composition.

In preferred embodiments, the extruded product allulose composition has a water content of at most 10 wt.-%, preferably at most 8.0 wt.-%, more preferably at most 6.0 wt.-%, still more preferably at most 5.0 wt.-%, in each based on the total weight of the product allulose composition,

In preferred embodiments, the product allulose composition is preferably subjected to a post-extrusion drying step. Preferably, the product allulose composition is post-dried in a manner such that the residual water content is not more than 30 wt.-% and is preferably in the range of from 0.1 to 15 wt.-%, in particular in the range from 0.5 to 10 wt.-%, and specifically in the range of from 1.0 to 5.0 wt.-%. Preferably, the product allulose composition is post-dried in a manner such that the residual water content is at most 0.5 wt.-%.

The post-extrusion drying step may be performed by means of conventional drying equipment such as a fluidized-bed drier in which a preferably heated gas, preferably air or a stream of nitrogen, is passed through the product layer from below. The amount of gas is preferably adjusted such that the particles of the product allulose composition are fluidized and swirl. As a result of the gas/particle heat transition, the water is evaporated. Preferably, it is ensured that the temperature of the granules does not increase too much, i.e. preferably not more than 80°C and more preferably not more than 70°C. Preferably, the temperature of the granules during post-extrusion drying is in the range of from 10 to 40°C. The post-extrusion drying step may be performed by means of a conveyor belt.

The post-extrusion drying can take place continuously or discontinuously.

After the optional post-extrusion drying, the granules of the product allulose composition can also be fractionated by means of a sieve. Coarse material and fines can be ground and returned to the mixer for the purposes of mashing up the granulating mass.

In preferred embodiments, the product allulose composition is subjected to a grinding step in order to adjust the desired average particle size and particle size distribution.

Grinding may be performed by conventional milling equipment such as ball mill, disintegrator, pulverizer, screen mill, hammer mill, malmal crusher, pin mill, disc mill, air jet mill, and the like

Preferably, the product allulose composition obtained in step (e), (f) or (g) has a residual water content of at least 0.05 wt.-%; preferably at least 0.10 wt.-%, preferably at least 0.15 wt.-%, preferably at least 0.20 wt.-%, preferably at least 0.25 wt.-%, preferably at least 0.30 wt.-%, preferably at least 0.35 wt.-%, preferably at least 0.40 wt.-%, preferably at least 0.45 wt.-%, preferably at least 0.50 wt.-%; in each case relative to the total weight of the product allulose composition.

Preferably, the product allulose composition obtained in step (e), (f) or (g) has a residual water content of at most 4.5 wt.-%; preferably at most 4.4 wt.-%, preferably at most 4.3 wt.-%, preferably at most 4.2 wt.-%, preferably at most 4.1 wt.-%, preferably at most 4.0 wt.-%, preferably at most 3.9 wt.-%, preferably at most 3.8 wt.-%, preferably at most 3.7 wt.-%, preferably at most 3.6 wt.-%, preferably at most 3.5 wt.-%, preferably at most 3.4 wt.-%, preferably at most 3.3 wt.-%, preferably at most 3.2 wt.-%, preferably at most 3.1 wt.-%, preferably at most 3.0 wt.-%, preferably at most 2.9 wt.-%, preferably at most 2.8 wt.-%, preferably at most 2.7 wt.-%, preferably at most 2.6 wt.-%, preferably at most 2.5 wt.-%.

Preferably, the product allulose composition obtained in step (e), (f) or (g) has a residual water content of at most 2.5 wt.-%; preferably at most 2.4 wt.-%, preferably at most 2.3 wt.-%, preferably at most 2.2 wt.-%, preferably at most 2.1 wt.-%, preferably at most 2.0 wt.-%, preferably at most 1.9 wt.-%, preferably at most 1.8 wt.-%, preferably at most 1.7 wt.-%, preferably at most 1.6 wt.-%, preferably at most 1.5 wt.-%, preferably at most 1.4 wt.-%, preferably at most 1.3 wt.-%, preferably at most 1.2 wt.-%, preferably at most 1.1 wt.-%, preferably at most 1.0 wt.-%, preferably at most 0.9 wt.-%, preferably at most 0.8 wt.-%, preferably at most 0.7 wt.-%, preferably at most 0.6 wt.-%, preferably at most 0.5 wt.-%.

Preferably, the product allulose composition obtained in step (e), (f) or (g) has a residual water content of at least 0.1 wt.-%; preferably at least 0.2 wt.-%, more preferably at least 0.3 wt.-%, still more preferably at least 0.4 wt.-%, yet more preferably at least 0.5 wt.-%, even more preferably at least 0.75 wt.-%, most preferably at least 1.0 wt.-%, and in particular at least 1.5 wt.-%; in each case relative to the total weight of the product allulose composition.

Preferably, the product allulose composition obtained in step (e), (f) or (g) has a residual water content of at most 3.5 wt.-%; preferably at most 3.0 wt.-%, more preferably at most 2.5 wt.-%, still more preferably at most 2.0 wt.-%, yet more preferably at most 1.5 wt.-%, even more preferably at most 1.0 wt.-%, most preferably at most 0.75 wt.-%, and in particular at most 0.5 wt.-%; in each case relative to the total weight of the product allulose composition.

Preferably, the product allulose composition obtained in step (e), (f) or (g) has a residual water content within the range of 2.0±1.5 wt.-%; preferably 1.5±1.0 wt.-%, more preferably 1.0±0.75 wt.-%, still more preferably 0.75±0.5 wt.-%, yet more preferably 0.5±0.4 wt.-%, even more preferably 0.5±0.3 wt.-%, most 0.5±0.2 wt.-%, and in particular 0.5±0.1 wt.-%; in each case relative to the product allulose composition.

Preferably, the product allulose composition obtained in step (e), (f) or (g) has an allulose content of at least 70 wt.-%; preferably at least 71 wt.-%, preferably at least 72 wt.-%, preferably at least 73 wt.-%, preferably at least 74 wt.-%, preferably at least 75 wt.-%, preferably at least 76 wt.-%, preferably at least 77 wt.-%, preferably at least 78 wt.-%, preferably at least 79 wt.-%, preferably at least 80 wt.-%, preferably at least 81 wt.-%, preferably at least 82 wt.-%, preferably at least 83 wt.-%, preferably at least 84 wt.-%, preferably at least 85 wt.-%, preferably at least 86 wt.-%, preferably at least 87 wt.-%, preferably at least 88 wt.-%, preferably at least 89 wt.-%, preferably at least 90 wt.-%, preferably at least 91 wt.-%, preferably at least 92 wt.-%, preferably at least 93 wt.-%, preferably at least 94 wt.-%, preferably at least 95 wt.-%, preferably at least 96 wt.-%, preferably at least 97 wt.-%, preferably at least 98 wt.-%, preferably at least 99 wt.-%; in each case relative to the total weight of the product allulose composition.

Preferably, the product allulose composition obtained in step (e), (f) or (g) has an allulose content of at most 99.5 wt.-%; preferably at most 99.0 wt.-%, preferably at most 98.5 wt.-%, preferably at most 98.0 wt.-%, preferably at most 97.5 wt.-%, preferably at most 97.0 wt.-%, preferably at most 96.5 wt.-%, preferably at most 96.0 wt.-%, preferably at most 95.5 wt.-%, preferably at most 95.0 wt.-%; in each case relative to the total weight of the product allulose composition.

Preferably, the product allulose composition obtained in step (e), (f) or (g) has a fructose content of at least 0.05 wt.-%; preferably at least 0.10 wt.-%, preferably at least 0.15 wt.-%, preferably at least 0.20 wt.-%, preferably at least 0.25 wt.-%, preferably at least 0.30 wt.-%, preferably at least 0.35 wt.-%, preferably at least 0.40 wt.-%, preferably at least 0.45 wt.-%, preferably at least 0.50 wt.-%; in each case relative to the total weight of the product allulose composition.

Preferably, the product allulose composition obtained in step (e), (f) or (g) has a fructose content of at most 10 wt.-%; preferably at most 9.0 wt.-%, preferably at most 8.0 wt.-%, preferably at most 7.0 wt.-%, preferably at most 6.0 wt.-%, preferably at most 5.0 wt.-%, preferably at most 4.0 wt.-%, preferably at most 3.0 wt.-%, preferably at most 2.0 wt.-%, preferably at most 1.0 wt.-%: in each case relative to the total weight of the product allulose composition.

Preferably, the product allulose composition obtained in step (e), (f) or (g) has a degree of crystallinity of at least 1%; preferably at least 2%, preferably at least 3%, preferably at least 4%, preferably at least 5%, preferably at least 6%, preferably at least 7%, preferably at least 8%, preferably at least 9%, preferably at least 10%.

Preferably, the product allulose composition obtained in step (e), (f) or (g) has a degree of crystallinity of at most 90%; preferably at most 80%, preferably at most 70%, preferably at most 60%, preferably at most 50%, preferably at most 40%, preferably at most 30%, preferably at most 20%, preferably at most 15%, preferably at most 10%, preferably at most 7.5%, preferably at most 5%, preferably at most 2.5%, preferably at most 1%.

Methods for determining the degree of crystallinity are known to the skilled person and include e.g. XRPD (x-ray powder diffraction) measurements in comparison to calibration curves that were determined for samples of known degree

of crystallinity with an amorphous sample as one extreme and a single crystal as the other extreme.

Preferably, the product allulose composition obtained in step (e), (f) or (g) is essentially amorphous.

Methods for determining the amorphous state of a solid material are known to the skilled person and include differential scanning calorimetry (DSC) and x-ray powder diffraction (XRPD).

In preferred embodiments, the product allulose composition obtained in step (e), (f) or (g), unlike purely crystalline allulose, comprises a mixture of at least two, preferably at least three, more preferably all anomeric forms of allulose, independently of one another selected from α-pyranose, β-pyranose, α-furanose, β-furanose, and the open keto form. Analytical methods for identifying these anomeric forms of allulose and for quantifying their relative content in a solid material are known to the skilled person and include but are not limited to solid-state nuclear magnetic resonance (NMR) spectroscopy. Other methods include NMR spectroscopy of solutions in aprotic solvents, e.g. DMSO-d6, preferably at low temperatures; or polarimetry or circular dichroism.

Preferably, the product allulose composition obtained in step (e), (f) or (g) has a content of allulose in its α-furanose form of at least 2.0 wt.-%; preferably at least 4.0 wt.-%, preferably at least 6.0 wt.-%, preferably at least 8.0 wt.-%, preferably at least 10 wt.-%, preferably at least 12 wt.-%, preferably at least 14 wt.-%, preferably at least 16 wt.-%, preferably at least 18 wt.-%, preferably at least 20 wt.-%, preferably at least 22 wt.-%, preferably at least 24 wt.-%, preferably at least 26 wt.-%, preferably at least 28 wt.-%, preferably at least 30 wt.-%, preferably at least 32 wt.-%, preferably at least 34 wt.-%, preferably at least 36 wt.-%, preferably at least 38 wt.-%; in each case relative to the total content of allulose that is contained in the product allulose composition.

Preferably, the product allulose composition obtained in step (e), (f) or (g) has a content of allulose in its α-furanose form of at most at most 95 wt.-%; preferably at most 90 wt.-%, preferably at most 85 wt.-%, preferably at most 80 wt.-%, preferably at most 75 wt.-%, preferably at most 70 wt.-%, preferably at most 65 wt.-%, preferably at most 60 wt.-%, preferably at most 55 wt.-%, preferably at most 50 wt.-%, preferably at most 45 wt.-%, preferably at most 40 wt.-%; in each case relative to the total content of allulose that is contained in the product allulose composition.

Preferably, the product allulose composition obtained in step (e), (f) or (g) has a content of allulose in its β-furanose form of at least 1.0 wt.-%; preferably at least 2.0 wt.-%, preferably at least 3.0 wt.-%, preferably at least 4.0 wt.-%, preferably at least 5.0 wt.-%, preferably at least 6.0 wt.-%, preferably at least 7.0 wt.-%, preferably at least 8.0 wt.-%, preferably at least 9.0 wt.-%, preferably at least 10 wt.-%, preferably at least 11 wt.-%, preferably at least 12 wt.-%, preferably at least 13 wt.-%, preferably at least 14 wt.-%; in each case relative to the total content of allulose that is contained in the product allulose composition.

Preferably, the product allulose composition obtained in step (e), (f) or (g) has a content of allulose in its β-furanose form of at most at most 95 wt.-%; preferably at most 90 wt.-%, preferably at most 85 wt.-%, preferably at most 80 wt.-%, preferably at most 75 wt.-%, preferably at most 70 wt.-%, preferably at most 65 wt.-%, preferably at most 60 wt.-%, preferably at most 55 wt.-%, preferably at most 50 wt.-%, preferably at most 45 wt.-%, preferably at most 40 wt.-%, preferably at most 35 wt.-%, preferably at most 30 wt.-%, preferably at most 25 wt.-%, preferably at most 20 wt.-%, preferably at most 15 wt.-%; in each case relative to the total content of allulose that is contained in the product allulose composition.

Preferably, the product allulose composition obtained in step (e), (f) or (g) has a content of allulose in its α-pyranose form of at least 2.0 wt.-%; preferably at least 4.0 wt.-%, preferably at least 6.0 wt.-%, preferably at least 8.0 wt.-%, preferably at least 10 wt.-%, preferably at least 12 wt.-%, preferably at least 14 wt.-%, preferably at least 16 wt.-%, preferably at least 18 wt.-%, preferably at least 20 wt.-%, preferably at least 22 wt.-%; in each case relative to the total content of allulose that is contained in the product allulose composition.

Preferably, the product allulose composition obtained in step (e), (f) or (g) has a content of allulose in its α-pyranose form of at most 95 wt.-%; preferably at most 90 wt.-%, preferably at most 85 wt.-%, preferably at most 80 wt.-%, preferably at most 75 wt.-%, preferably at most 70 wt.-%, preferably at most 65 wt.-%, preferably at most 60 wt.-%, preferably at most 55 wt.-%, preferably at most 50 wt.-%, preferably at most 45 wt.-%, preferably at most 40 wt.-%, preferably at most 35 wt.-%, preferably at most 30 wt.-%, preferably at most 25 wt.-%; in each case relative to the total content of allulose that is contained in the product allulose composition.

Preferably, the product allulose composition obtained in step (e), (f) or (g) has a content of allulose in its β-pyranose form of at least 2.0 wt.-%; preferably at least 4.0 wt.-%, preferably at least 6.0 wt.-%, preferably at least 8.0 wt.-%, preferably at least 10 wt.-%, preferably at least 12 wt.-%, preferably at least 14 wt.-%, preferably at least 16 wt.-%, preferably at least 18 wt.-%, preferably at least 20 wt.-%, preferably at least 22 wt.-%, preferably at least 24 wt.-%; in each case relative to the total content of allulose that is contained in the product allulose composition.

Preferably, the product allulose composition obtained in step (e), (f) or (g) has a content of allulose in its β-pyranose form of at most 99 wt.-%; preferably at most 95 wt.-%, preferably at most 90 wt.-%, preferably at most 85 wt.-%, preferably at most 80 wt.-%, preferably at most 75 wt.-%, preferably at most 70 wt.-%, preferably at most 65 wt.-%, preferably at most 60 wt.-%, preferably at most 55 wt.-%, preferably at most 50 wt.-%, preferably at most 45 wt.-%, preferably at most 40 wt.-%, preferably at most 35 wt.-%, preferably at most 30 wt.-%, preferably at most 25 wt.-%; in each case relative to the total content of allulose that is contained in the product allulose composition.

Preferably, the product allulose composition obtained in step (e), (f) or (g) has a content of allulose in its open keto form of at least 0.01 wt.-%; preferably at least 0.05 wt.-%, preferably at least 0.10 wt.-%, preferably at least 0.15 wt.-%; in each case relative to the total content of allulose that is contained in the product allulose composition.

Preferably, the product allulose composition obtained in step (e), (f) or (g) has a content of allulose in its open keto form of at most 5.0 wt.-%; preferably at most 4.5 wt.-%, preferably at most 4.0 wt.-%, preferably at most 3.5 wt.-%, preferably at most 3.0 wt.-%, preferably at most 2.5 wt.-%, preferably at most 2.0 wt.-%, preferably at most 1.5 wt.-%, preferably at most 1.0 wt.-%, preferably at most 0.5 wt.-%; in each case relative to the total content of allulose that is contained in the product allulose composition.

Preferably, the product allulose composition obtained in step (e), (f) or (g) of the process according to the invention is a particulate material, preferably a powder. The powder is preferably characterized by its average particle size and particle size distribution.

Suitable methods for determining the average particle size and the particle size distribution of a powder are known to a skilled person. Suitable methods include but are not limited to sieve analysis (gradation), laser diffraction, and dynamic picture analysis.

Preferably, the average particle size and all other parameters that are useful to describe the particle size, shape and distribution of the product allulose composition obtained in step (e), (f) or (g) are determined by optical methods, preferably by dynamic picture analysis according to ISO 13322-2. In this regard, the average particle size preferably corresponds to the value Mv (also referred to as "mean diameter" and "Mv3(x)", respectively). ISO 13322-2:2006 describes methods for controlling the position of moving particles in a liquid or gas and on a conveyor, as well as the image capture and image analysis of the particles. These methods are used to measure the particle sizes and their distributions, the particles being appropriately dispersed in the liquid or gas medium or on the conveyor. A suitable device is the particle size analyzer Camsizer® XT (Retsch Technology GmbH, Haan, Germany; X-Jet Module, 30 kPa for dispersion).

In a preferred embodiment of the product allulose composition obtained in step (e), (f) or (g), the average particle size of the powder is at most 900 μm, preferably at most 850 μm, preferably at most 800 μm, yet preferably at most 750 μm, preferably at most 700 μm, preferably at most 650 μm. In a particularly preferred embodiment, the average particle size of the powder is within the range of from 600 μm to 900 μm. In a particularly preferred embodiment of the product allulose composition obtained in step (e), (f) or (g), the average particle size of the powder (statistical value) is at most 900 μm and the powder essentially comprises no particles having a particle size (individual value) of more than 900 μm (grain size class ≤ 900 μm).

In a preferred embodiment of the product allulose composition obtained in step (e), (f) or (g), the average particle size of the powder is at most 600 μm, preferably at most 550 μm, preferably at most 500 μm, yet preferably at most 450 μm, preferably at most 400 μm, preferably at most 350 μm. In a particularly preferred embodiment, the average particle size of the powder is within the range of from 300 μm to 600 μm. In a particularly preferred embodiment of the product allulose composition obtained in step (e), (f) or (g), the average particle size of the powder (statistical value) is at most 600 μm and the powder essentially comprises no particles having a particle size (individual value) of more than 600 μm (grain size class ≤ 600 μm).

In a preferred embodiment of the product allulose composition obtained in step (e), (f) or (g), the average particle size of the powder is at most 300 μm, preferably at most 275 μm, preferably at most 250 μm, preferably at most 225 μm. In a particularly preferred embodiment, the average particle size of the powder is within the range of from 200 μm to 300 μm. In a particularly preferred embodiment of the product allulose composition obtained in step (e), (f) or (g), the average particle size of the powder (statistical value) is at most 300 μm and the powder essentially comprises no particles having a particle size (individual value) of more than 300 μm (grain size class ≤ 300 μm).

In a preferred embodiment of the product allulose composition obtained in step (e), (f) or (g), the average particle size of the powder is at most 200 μm, preferably at most 175 μm, preferably at most 150 μm, preferably at most 125 μm. In a particularly preferred embodiment, the average particle size of the powder is within the range of from 10 μm to 200 μm. In a particularly preferred embodiment of the product allulose composition obtained in step (e), (f) or (g), the average particle size of the powder (statistical value) is at most 200 μm and the powder essentially comprises no particles having a particle size (individual value) of more than 200 μm (grain size class ≤ 200 μm).

From a sensory perspective, powders with a D50 value of about 10 μm (very fine) are not recognized as powders by the consumer. Powders with a D50 value of about 20 μm (fine) have an attractive optical appearance. Powders with a D50 value of about 30 μm (coarse) are glossy. Powders with a D50 value of about 40 μm (extra coarse) have good flow properties, are not dusty and are glossy.

Preferably, the product allulose composition obtained in step (e), (f) or (g) is a powder having an average particle size

- of at most 900 μm, preferably at most 800 μm, preferably at most 700 μm, preferably at most 600 μm, preferably at most 500 μm, preferably at most 400 μm, preferably at most 300 μm, preferably at most 200 μm, preferably at most

100 µm, preferably at most 90 µm, preferably at most 80 µm, preferably at most 70 µm, preferably at most 60 µm, preferably at most 50 µm, preferably at most 40 µm, preferably at most 30 µm, preferably at most 20 µm, preferably at most 10 µm; and/or

- of at least 10 µm, preferably at least 20 µm, preferably at least 30 µm, preferably at least 40 µm, preferably at least 50 µm, preferably at least 60 µm, preferably at least 70 µm, preferably at least 80 µm, preferably at least 90 µm, preferably at least 100 µm, preferably at least 200 µm, preferably at least 300 µm, preferably at least 400 µm, preferably at least 500 µm, preferably at least 600 µm, preferably at least 700 µm, preferably at least 800 µm, preferably at least 900 µm; and/or

- within the range of 10 µm (±50%), 20 µm (±50%), 30 µm (±50%), 40 µm (±50%), 50 µm (±50%), 60 µm (±50%), 70 µm (±50%), 80 µm (±50%), 90 µm (±50%), 100 µm (±50%), 150 µm (±50%), 200 µm (±50%), 250 µm (±50%), 300 µm (±50%), 350 µm (±50%), 400 µm (±50%), 450 µm (±50%), 500 µm (±50%), 600 µm (±50%), 700 µm (±50%), 800 µm (±50%), or 900 µm (±50%).

Preferably, the product allulose composition obtained in step (e), (f) or (g) is a powder having a particle size distribution that is characterized by a D10 value

- of at most 1800 µm, preferably at most 1700 µm, preferably at most 1600 µm, preferably at most 1500 µm, preferably at most 1400 µm, preferably at most 1300 µm, preferably at most 1200 µm, preferably at most 1100 µm, preferably at most 1000 µm, preferably at most 900 µm, preferably at most 800 µm, preferably at most 700 µm, preferably at most 600 µm, preferably at most 500 µm, preferably at most 400 µm, preferably at most 300 µm, preferably at most 200 µm, preferably at most 100 µm, preferably at most 90 µm, preferably at most 80 µm, preferably at most 70 µm, preferably at most 60 µm, preferably at most 50 µm, preferably at most 40 µm, preferably at most 30 µm, preferably at most 20 µm, preferably at most 10 µm; and/or

- of at least 10 µm, preferably at least 20 µm, preferably at least 30 µm, preferably at least 40 µm, preferably at least 50 µm, preferably at least 60 µm, preferably at least 70 µm, preferably at least 80 µm, preferably at least 90 µm, preferably at least 100 µm, preferably at least 200 µm, preferably at least 300 µm, preferably at least 400 µm, preferably at least 500 µm, preferably at least 600 µm, preferably at least 700 µm, preferably at least 800 µm, preferably at least 900 µm, preferably at least 1000 µm, preferably at least 1100 µm, preferably at least 1200 µm, preferably at least 1300 µm, preferably at least 1400 µm, preferably at least 1500 µm, preferably at least 1600 µm, preferably at least 1700 µm, preferably at least 1800 µm; and/or

- within the range of 10 µm (±50%), 20 µm (±50%), 30 µm (±50%), 40 µm (±50%), 50 µm (±50%), 60 µm (±50%), 70 µm (±50%), 80 µm (±50%), 90 µm (±50%), 100 µm (±50%), 150 µm (±50%), 200 µm (±50%), 250 µm (±50%), 300 µm (±50%), 350 µm (±50%), 400 µm (±50%), 450 µm (±50%), 500 µm (±50%), 600 µm (±50%), 700 µm (±50%), 800 µm (±50%), 900 µm (±50%), 1000 µm (±50%), 1100 µm (±50%), 1200 µm (±50%), 1300 µm (±50%), 1400 µm (±50%), 1500 µm (±50%), 1600 µm (±50%), 1700 µm (±50%), or 1800 µm (±50%).

Preferably, the product allulose composition obtained in step (e), (f) or (g) is a powder having a particle size distribution that is characterized by a D50 value

- of at most 1300 µm, preferably at most 1200 µm, preferably at most 1100 µm, preferably at most 1000 µm, preferably at most 900 µm, preferably at most 800 µm, preferably at most 700 µm, preferably at most 600 µm, preferably at most 500 µm, preferably at most 400 µm, preferably at most 300 µm, preferably at most 200 µm, preferably at most 100 µm, preferably at most 90 µm, preferably at most 80 µm, preferably at most 70 µm, preferably at most 60 µm, preferably at most 50 µm, preferably at most 40 µm, preferably at most 30 µm, preferably at most 20 µm, preferably at most 10 µm; and/or

- of at least 10 µm, preferably at least 20 µm, preferably at least 30 µm, preferably at least 40 µm, preferably at least 50 µm, preferably at least 60 µm, preferably at least 70 µm, preferably at least 80 µm, preferably at least 90 µm, preferably at least 100 µm, preferably at least 200 µm, preferably at least 300 µm, preferably at least 400 µm, preferably at least 500 µm, preferably at least 600 µm, preferably at least 700 µm, preferably at least 800 µm, preferably at least 900 µm, preferably at least 1000 µm, preferably at least 1100 µm, preferably at least 1200 µm, preferably at least 1300 µm; and/or

- within the range of 10 µm (±50%), 20 µm (±50%), 30 µm (±50%), 40 µm (±50%), 50 µm (±50%), 60 µm (±50%), 70

µm (±50%), 80 µm (±50%), 90 µm (±50%), 100 µm (±50%), 150 µm (±50%), 200 µm (±50%), 250 µm (±50%), 300 µm (±50%), 350 µm (±50%), 400 µm (±50%), 450 µm (±50%), 500 µm (±50%), 600 µm (±50%), 700 µm (±50%), 800 µm (±50%), 900 µm (±50%), 1000 µm (±50%), 1100 µm (±50%), 1200 µm (±50%), or 1300 µm (±50%).

Preferably, the product allulose composition obtained in step (e), (f) or (g) is a powder having a particle size distribution that is characterized by a D90 value

- of at most 900 µm, preferably at most 800 µm, preferably at most 700 µm, preferably at most 600 µm, preferably at most 500 µm, preferably at most 400 µm, preferably at most 300 µm, preferably at most 200 µm, preferably at most 100 µm, preferably at most 90 µm, preferably at most 80 µm, preferably at most 70 µm, preferably at most 60 µm, preferably at most 50 µm, preferably at most 40 µm, preferably at most 30 µm, preferably at most 20 µm, preferably at most 10 µm; and/or

- of at least 10 µm, preferably at least 20 µm, preferably at least 30 µm, preferably at least 40 µm, preferably at least 50 µm, preferably at least 60 µm, preferably at least 70 µm, preferably at least 80 µm, preferably at least 90 µm, preferably at least 100 µm, preferably at least 200 µm, preferably at least 300 µm, preferably at least 400 µm, preferably at least 500 µm, preferably at least 600 µm, preferably at least 700 µm, preferably at least 800 µm, preferably at least 900 µm; and/or

- within the range of 10 µm (±50%), 20 µm (±50%), 30 µm (±50%), 40 µm (±50%), 50 µm (±50%), 60 µm (±50%), 70 µm (±50%), 80 µm (±50%), 90 µm (±50%), 100 µm (±50%), 150 µm (±50%), 200 µm (±50%), 250 µm (±50%), 300 µm (±50%), 350 µm (±50%), 400 µm (±50%), 450 µm (±50%), 500 µm (±50%), 600 µm (±50%), 700 µm (±50%), 800 µm (±50%), or 900 µm (±50%).

In another preferred embodiment of the product allulose composition obtained in step (e), (f) or (g), the average particle size of the powder is within the range of from 300 µm to 1.5 mm. In a particularly preferred embodiment of the product allulose composition obtained in step (e), (f) or (g), the average particle size of the powder (statistical value) is within the range of from 300 µm to 1.5 mm and the powder essentially comprises no particles having a particle size (individual value) of less than 300 µm and more than 1.5 mm (grain size class 300 µm ≤ x ≤ 1.5 mm).

Particularly preferred embodiments of this preferred product allulose composition obtained in step (e), (f) or (g) having an average particle size within the range of from 300 µm to 1.5 mm are described hereinafter.

Preferably, the product allulose composition obtained in step (e), (f) or (g) is a powder having an average particle size

- of at most 1500 µm, preferably at most 1400 µm, preferably at most 1300 µm, preferably at most 1200 µm, preferably at most 1100 µm, preferably at most 1000 µm, preferably at most 900 µm, preferably at most 800 µm, preferably at most 700 µm, preferably at most 600 µm, preferably at most 500 µm, preferably at most 400 µm, preferably at most 300 µm; and/or

- of at least 300 µm, preferably at least 400 µm, preferably at least 500 µm, preferably at least 600 µm, preferably at least 700 µm, preferably at least 800 µm, preferably at least 900 µm, preferably at least 1000 µm, preferably at least 1100 µm, preferably at least 1200 µm, preferably at least 1300 µm, preferably at least 1400 µm, preferably at least 1500 µm; and/or

- within the range of 300 µm (±50%), 350 µm (±50%), 400 µm (±50%), 450 µm (±50%), 500 µm (±50%), 600 µm (±50%), 700 µm (±50%), 800 µm (±50%), 900 µm (±50%), 1000 µm (±50%), 1100 µm (±50%), 1200 µm (±50%), 1300 µm (±50%), 1400 µm (±50%), or 1500 µm (±50%).

Preferably, the product allulose composition obtained in step (e), (f) or (g) is a powder having a particle size distribution that is characterized by a D10 value

- of at most 3000 µm, preferably at most 2500 µm, preferably at most 2400 µm, preferably at most 2300 µm, preferably at most 2200 µm, preferably at most 2100 µm, preferably at most 2000 µm, preferably at most 1900 µm, preferably at most 1800 µm, preferably at most 1700 µm, preferably at most 1600 µm, preferably at most 1500 µm, preferably at most 1400 µm, preferably at most 1300 µm, preferably at most 1200 µm, preferably at most 1100 µm, preferably at most 1000 µm, preferably at most 900 µm, preferably at most 800 µm, preferably at most 700 µm, preferably at most 600 µm, preferably at most 500 µm, preferably at most 400 µm, preferably at most 300 µm, preferably at most 200 µm,

preferably at most 100 μm; and/or

- of at least 100 μm, preferably at least 200 μm, preferably at least 300 μm, preferably at least 400 μm, preferably at least 500 μm, preferably at least 600 μm, preferably at least 700 μm, preferably at least 800 μm, preferably at least 900 μm, preferably at least 1000 μm, preferably at least 1100 μm, preferably at least 1200 μm, preferably at least 1300 μm, preferably at least 1400 μm, preferably at least 1500 μm, preferably at least 1600 μm, preferably at least 1700 μm, preferably at least 1800 μm, preferably at least 1900 μm, preferably at least 2000 μm, preferably at least 2100 μm, preferably at least 2200 μm, preferably at least 2300 μm, preferably at least 2400 μm, preferably at least 2500 μm, preferably at least 3000 μm; and/or

- within the range of 100 μm (±50%), 150 μm (±50%), 200 μm (±50%), 250 μm (±50%), 300 μm (±50%), 350 μm (±50%), 400 μm (±50%), 450 μm (±50%), 500 μm (±50%), 600 μm (±50%), 700 μm (±50%), 800 μm (±50%), 900 μm (±50%), 1000 μm (±50%), 1100 μm (±50%), 1200 μm (±50%), 1300 μm (±50%), 1400 μm (±50%), 1500 μm (±50%), 1600 μm (±50%), 1700 μm (±50%), 1800 μm (±50%), 1900 μm (±50%), 2000 μm (±50%), 2100 μm (±50%), 2200 μm (±50%), 2300 μm (±50%), 2400 μm (±50%), 2500 μm (±50%), or 3000 μm (±50%).

Preferably, the product allulose composition obtained in step (e), (f) or (g) is a powder having a particle size distribution that is characterized by a D50 value

- of at most 1800 μm, preferably at most 1700 μm, preferably at most 1600 μm, preferably at most 1500 μm, preferably at most 1400 μm, preferably at most 1300 μm, preferably at most 1200 μm, preferably at most 1100 μm, preferably at most 1000 μm, preferably at most 900 μm, preferably at most 800 μm, preferably at most 700 μm, preferably at most 600 μm, preferably at most 500 μm, preferably at most 400 μm, preferably at most 300 μm, preferably at most 200 μm; and/or

- of at least 200 μm, preferably at least 300 μm, preferably at least 400 μm, preferably at least 500 μm, preferably at least 600 μm, preferably at least 700 μm, preferably at least 800 μm, preferably at least 900 μm, preferably at least 1000 μm, preferably at least 1100 μm, preferably at least 1200 μm, preferably at least 1300 μm, preferably at least 1400 μm, preferably at least 1500 μm, preferably at least 1600 μm, preferably at least 1700 μm, preferably at least 1800 μm; and/or

- within the range of 200 μm (±50%), 250 μm (±50%), 300 μm (±50%), 350 μm (±50%), 400 μm (±50%), 450 μm (±50%), 500 μm (±50%), 600 μm (±50%), 700 μm (±50%), 800 μm (±50%), 900 μm (±50%), 1000 μm (±50%), 1100 μm (±50%), 1200 μm (±50%), 1300 μm (±50%), 1400 μm (±50%), 1500 μm (±50%), 1600 μm (±50%), 1700 μm (±50%), or 1800 μm (±50%).

Preferably, the product allulose composition obtained in step (e), (f) or (g) is a powder having a particle size distribution that is characterized by a D90 value

- of at most 1500 μm, preferably at most 1400 μm, preferably at most 1300 μm, preferably at most 1200 μm, preferably at most 1100 μm, preferably at most 1000 μm, preferably at most 900 μm, preferably at most 800 μm, preferably at most 700 μm, preferably at most 600 μm, preferably at most 500 μm, preferably at most 400 μm, preferably at most 300 μm; and/or

- of at least 300 μm, preferably at least 400 μm, preferably at least 500 μm, preferably at least 600 μm, preferably at least 700 μm, preferably at least 800 μm, preferably at least 900 μm, preferably at least 1000 μm, preferably at least 1100 μm, preferably at least 1200 μm, preferably at least 1300 μm, preferably at least 1400 μm, preferably at least 1500 μm; and/or

- within the range of 300 μm (±50%), 350 μm (±50%), 400 μm (±50%), 450 μm (±50%), 500 μm (±50%), 600 μm (±50%), 700 μm (±50%), 800 μm (±50%), 900 μm (±50%), 1000 μm (±50%), 1100 μm (±50%), 1200 μm (±50%), 1300 μm (±50%), 1400 μm (±50%), or 1500 μm (±50%).

Preferably, the product allulose composition obtained in step (e), (f) or (g) has a particle size distribution that is characterized by a span value (D90-D10)/D50 of at most 10, preferably at most 9, preferably at most 8, preferably at most 7, preferably at most 6, preferably at most 5, preferably at most 4, preferably at most 3.5, preferably at most 3, preferably at most 2.5, preferably at most 2, preferably at most 1.5.

Preferably, the product allulose composition obtained in step (e), (f) or (g) has a particle size distribution that is

characterized by a span value (D90-D10)/D50 of at least 1.5, preferably at least 2, preferably at least 2.5, preferably at least 3, preferably at least 3.5, preferably at least 4, preferably at least 5, preferably at least 6, preferably at least 7, preferably at least 8, preferably at least 9, preferably at least 10.

Preferably, the product allulose composition obtained in step (e), (f) or (g) has a particle size distribution that is characterized by a relative breath D90/D10 of at most 10, preferably at most 9, preferably at most 8, preferably at most 7, preferably at most 6, preferably at most 5, preferably at most 4, preferably at most 3.5, preferably at most 3, preferably at most 2.5, preferably at most 2, preferably at most 1.5.

Preferably, the product allulose composition obtained in step (e), (f) or (g) has a particle size distribution that is characterized by a relative breath D90/D10 of at least 1.5, preferably at least 2, preferably at least 2.5, preferably at least 3, preferably at least 3.5, preferably at least 4, preferably at least 5, preferably at least 6, preferably at least 7, preferably at least 8, preferably at least 9, preferably at least 10.

The particle size distribution of a powder can be analyzed by sieve analysis.

Preferably, at least 82.5 % of the powder passes a mesh size of 400 μm. More preferably at least 83 %, preferably at least 84 %, preferably at least 85 %, preferably at least 86 %, preferably at least 87 %, preferably at least 88 % of the powder passes a mesh size of 400 μm.

Preferably, at least 46 % of the powder passes a mesh size of 315 μm. More preferably at least 47 %, preferably at least 50 %, preferably at least 52 %, preferably at least 54 %, preferably at least 56 %, preferably at least 58 % of the powder passes a mesh size of 315 μm.

Preferably, at least 15.5 % of the powder passes a mesh size of 250 μm. More preferably at least 16 %, preferably at least 19 %, preferably at least 22 %, preferably at least 25 %, preferably at least 28 %, preferably at least 31 % of the powder passes a mesh size of 250 μm.

Preferably, at least 3.5 % of the powder passes a mesh size of 200 μm. More preferably at least 4 %, preferably at least 6 %, preferably at least 8 %, preferably at least 10 %, preferably at least 12 %, preferably at least 13 % of the powder passes a mesh size of 200 μm.

Preferably, the product allulose composition obtained in step (e), (f) or (g) has a bulk density, preferably measured by means of a Powtec 500 bulk density analysis device, of at most 900 $kg/m^3$, preferably at most 850 $kg/m^3$, preferably at most 800 $kg/m^3$, yet preferably at most 750 $kg/m^3$, preferably at most 700 $kg/m^3$, preferably at most 675 $kg/m^3$, preferably at most 650 $kg/m^3$.

In preferred embodiments, the product allulose composition obtained in step (e), (f) or (g) has a bulk density, preferably measured by means of a Powtec 500 bulk density analysis device, within the range of $300\pm100$ $kg/m^3$, or $350\pm100$ $kg/m^3$, or $400\pm100$ $kg/m^3$, or $450\pm100$ $kg/m^3$, or $500\pm100$ $kg/m^3$, or $550\pm100$ $kg/m^3$, or $600\pm100$ $kg/m^3$, or $650\pm100$ $kg/m^3$, or $700\pm100$ $kg/m^3$, or $750\pm100$ $kg/m^3$.

Preferably, the product allulose composition obtained in step (e), (f) or (g) is a free flowing powder.

Preferably, the product allulose composition obtained in step (e), (f) or (g) is an essentially dry powder.

Another aspect of the present disclosure which is not literally claimed relates to a product allulose composition, preferably solid allulose composition, having

(i) a residual water content of at least 0.05 wt.-% and/or preferably at most 5.0 wt.-%, more preferably at most 2.5 wt.-%, relative to the total weight of the product allulose composition, preferably solid allulose composition; and/or

(ii) an allulose content of at least 70 wt.-% and/or preferably at most 99.5 wt.-%, more preferably from 95 to 96 wt.-%, relative to the total weight of the product allulose composition, preferably solid allulose composition; and/or

(iii) a fructose content of at least 0.05 wt.-% and/or preferably at most 10 wt.-%, relative to the total weight of the product allulose composition, preferably solid allulose composition; and/or

(iv) a degree of crystallinity of at least 1% and/or preferably at most 90%; and/or

(v) a content of allulose in its α-furanose form of at least 2.0 wt.-% and/or preferably at most at most 95 wt.-%, relative to the total content of allulose that is contained in the product allulose composition, preferably solid allulose composition; and/or

(vi) a content of allulose in its β-furanose form of at least 1.0 wt.-% and/or preferably at most at most 95 wt.-%, relative to the total content of allulose that is contained in the product allulose composition, preferably solid allulose composition; and/or

(vii) a content of allulose in its α-pyranose form of at least 2.0 wt.-% and/or preferably at most 95 wt.-%, relative to the total content of allulose that is contained in the product allulose composition, preferably solid allulose composition;

and/or

(viii) a content of allulose in its β-pyranose form of at least 2.0 wt.-% and/or preferably at most 99 wt.-%, relative to the total content of allulose that is contained in the product allulose composition, preferably solid allulose composition; and/or

(ix) a content of allulose in its open keto form of at least 0.01 wt.-% and/or preferably at most 5.0 wt.-%, relative to the total content of allulose that is contained in the product allulose composition, preferably solid allulose composition; and/or

(x) an average particle size of at most 900 μm and/or preferably at least 10 μm; and/or

(xi) a particle size distribution that is characterized by a D10 value of at most 1800 μm and/or preferably at least 10 μm; and/or

(xii) a particle size distribution that is characterized by a D50 value of at most 1300 μm and/or preferably at least 10 μm; and/or

(xiii) a particle size distribution that is characterized by a D90 value of at most 900 μm and/or preferably at least 10 μm.

The preferred properties of the product allulose composition that is obtained by step (e), step (f) or step (g) of the process according to the invention as described above are analogously also preferred properties of the product allulose composition according to the invention, even when it is not obtained by said process, i.e. when it is not obtained by step (e), step (f) or step (g) of the process according to the invention, and are not repeated hereinafter.

Preferably, when dissolving 26.05 g of the product allulose composition according to the invention in 74.30 g of pure water at room temperature it takes at most 50 minutes; preferably at most 45 minutes, preferably at most 40 minutes, preferably at most 35 minutes, preferably at most 30 minutes, preferably at most 25 minutes, preferably at most 20 minutes, preferably at most 15 minutes, preferably at most 10 minutes, preferably at most 5 minutes; in each case until angle of rotation [α] does not essentially change anymore, i.e. until the anomeric forms of allulose have been equilibrated.

Preferably, the allulose composition according to the invention is obtainable by the process according to the invention as described above.

The following examples further illustrate the invention but are not to be construed as limiting its scope.

Example 1 - mutarotation:

Crystalline allulose was dissolved in water (DS 26 wt.-%). Quickly afterwards, the sample was placed in a polarimeter and the angle of rotation was read off at regular intervals (1 minute apart, 23°C). From this, the angle of rotation was then plotted over time (see Figure 1). 74.30 g of water and 26.05 g of crystalline allulose were mixed. The mixture was briefly stirred with a stir bar until the allulose was completely dissolved. Then a sample was placed in the polarimeter and the angle of rotation of the same sample was measured at regular intervals. There were approximately 5 minutes between the addition of the allulose and the first measured value (0 min).

It becomes clear from Figure 1 that the freshly prepared allulose needs more than 1 hour until the angle of rotation remains constant, i.e. until the chiral species in solution (a-pyranose, β-pyranose, α-furanose, β-furanose, open keto form) are equilibrated with one another.

Mutarotation of solid allulose obtained in accordance with the present invention was compared to mutarotation of highly crystalline allulose. For this purpose, solutions at a dry substance content of about DS 26 wt.-% were prepared from powdery compositions each having a particle size within the range of from 100 to 200 μm (sieve fraction).

15.61 g of powdery crystalline allulose (comparative) of grain size 100-200 μm were dissolved at 23°C in 47.6 g desalted water in a 500 mL beaker. The angle of rotation was measured over time with a polarimeter.

13.00 g of extruded allulose (inventive) of grain size 100-200 μm were dissolved at 23°C in 37.07 g desalted water in a 500 mL beaker. The angle of rotation was measured over time with a polarimeter.

The results are compiled in the following table:

| time [min] | angle of rotation [°] | | | time [min] | angle of rotation [°] | |
|---|---|---|---|---|---|---|
| | comparative | inventive | | | Comparative | |
| 0 | -33.4 | -27.10 | | 17 | -1.47 | -1.14 |
| 0.5 | -31.1 | -24.80 | | 17.5 | -1.23 | -0.94 |
| 1 | -29 | -23.08 | | 18 | -1 | -0.74 |
| 1.5 | -27 | -21.50 | | 18.5 | -0.8 | -0.56 |
| 2 | -25.1 | -20.07 | | 19 | -0.6 | -0.39 |
| 2.5 | -23.35 | -18.70 | | 19.5 | -0.41 | -0.23 |
| 3 | -21.7 | -17.39 | | 20 | -0.24 | -0.08 |
| 3.5 | -20.13 | -16.20 | | 20.5 | -0.08 | 0.06 |
| 4 | -18.71 | -15.05 | | 21 | 0.07 | 0.20 |
| 4.5 | -17.36 | -13.95 | | 21.5 | 0.22 | 0.32 |
| 5 | -16.1 | -13.00 | | 22 | 0.35 | 0.44 |
| 5.5 | -14.91 | - | | 22.5 | 0.47 | 0.55 |
| 6 | -13.8 | -11.80 | | 23 | 0.59 | 0.65 |
| 6.5 | -12.78 | -10.38 | | 23.5 | 0.7 | 0.75 |
| 7 | -11.81 | -9.60 | | 24 | 0.8 | 0.85 |
| 7.5 | -10.9 | -8.88 | | 24.5 | 0.9 | 0.94 |
| 8 | -10.05 | -8.20 | | 25 | 0.99 | 1.02 |
| 8.5 | -9.25 | -7.57 | | 25.5 | 1.07 | 1.09 |
| 9 | -8.51 | -6.97 | | 26 | 1.15 | 1.17 |
| 9.5 | -7.81 | -6.42 | | 26.5 | 1.22 | 1.23 |
| 10 | -7.18 | -5.89 | | 27 | 1.29 | 1.30 |
| 10.5 | -6.57 | -5.39 | | 27.5 | 1.36 | 1.36 |
| 11 | -6 | -4.93 | | 28 | 1.42 | 1.41 |
| 11.5 | -5.47 | -4.50 | | 28.5 | 1.48 | 1.47 |
| 12 | -4.98 | -4.09 | | 29 | 1.53 | 1.52 |
| 12.5 | -4.51 | -3.70 | | 29.5 | 1.58 | 1.56 |
| 13 | -4.08 | -3.34 | | 30 | 1.63 | 1.61 |
| 13.5 | -3.67 | -3.00 | | 35 | 1.96 | 1.94 |
| 14 | -3.29 | - | | 40 | 2.13 | 2.11 |
| 14.5 | -2.93 | -2.39 | | 45 | 2.22 | 2.21 |
| 15 | -2.6 | -2.10 | | 50 | 2.26 | 2.26 |
| 15.5 | -2.29 | - | | 55 | 2.29 | 2.29 |
| 16 | -2 | -1.60 | | 60 | 2.30 | 2.31 |
| 16.5 | -1.72 | -1.36 | | | | |

For the sake of better comparability, the specific angle of rotation [(°*ml) /(dm*g)] was calculated for each measured value. The results are visualized in Figure 2.

As shown in Figure 2, the specific angle of rotation immediately after the dissolution of extruded allulose is higher than that of crystalline allulose. This demonstrates that the tautomer equilibrium in the extruded allulose is different from that in the crystalline allulose.

Example 2 - preliminary tests:

Exceeding the thermodynamic equilibrium can be reduced by various measures, e.g. by

- native seed crystals,
- foreign nucleation sites,
- creation of an entropy-reducing order, e.g. by strong shear flow.

An extruder operated under evaporative conditions seems to be an optimal device for combining evaporation and shear. However, experimental conditions need to be found under which a particulate allulose phase can form from an allulose syrup at elevated temperature.

Therefore, preliminary tests were made in order to estimate supersaturation and overcooling of various carbohydrates in water under different conditions. The results of the preliminary tests are summarized in the table here below:

| | isomaltulose | fructose / glucose mixtures | sucrose | allulose | allulose |
|---|---|---|---|---|---|
| molecular weight | 342 g/mol | 180 g/mol | 342 g/mol | 180 g/mol | 180 g/mol |
| melting point | 120°C | 102°C (fructose)<br>146°C (glucose) | 186°C | 96°C | 96°C |
| mass fraction | 90% | 90% | 80% | 70%* | 80%* |
| temperature | 58°C | 56°C | 149°C | 150°C | 190°C |
| supersaturation | 35% | 1% (fructose)<br>16% (glucose) | -14% | -27% | -19% |
| undercooling | 62 K | 46 K (fructose)<br>90 K (glucose) | 37 K | -54 K | -94 K |
| * prior to evaporation | | | | | |

The data in the above table demonstrate significant differences in supersaturation and undercooling under the given conditions. Isomaltulose is highly supersaturated and undercooled, whereas the mixtures of fructose with glucose are less supersaturated but similarly undercooled. The solution of sucrose is undersaturated but at the same time undercooled because of its high melting point. Compared to sucrose, allulose has a much lower melting point. Thus, allulose is significantly above its melting point and the mixture with water is significantly undersaturated. It can be concluded from the data in the above table that for thermodynamic reasons, allulose cannot form a particulate phase under the given experimental conditions, because the solution is neither supersaturated nor undercooled.

It has surprisingly been found that the combination of evaporation and shear is particularly suitable for the production of particulate allulose. A stable temperature control and the action of shear forces play an important role. However, experimental findings indicate that, in addition to the supersaturation of the solution, the undercooling of the melt also plays an important role.

Example 3:

The following devices were used:

- RFM 81 - multiscale automatic refractometer (Bellingham Stanley)
- IR thermometer Testo 830-T1
- enclosed container W-3197 with spiral stirrer (Waldner Holding GmbH & Co. KG)
- vacuum pump LF 80/2C-11 (Sterling SIHI GmbH)
- circulation heater PT 60 (DIL e.V.)
- laboratory kneader LK5 with all-purpose system AR 403 (ERWEKA)

The following material was used:

- allulose syrup having a purity of 95.69%;
- seed crystals having an average particle size of 12 μm and a purity of 100%;
- seed crystals having an average particle size of 36 μm and a purity of 99.2%;
- seed crystals having an average particle size of 190 μm and a purity of about 99%.

The average Brix value was determined using a RFM 81 - multiscale automatic refractometer (Bellingham Stanley). 10 to 20 individual measurements were carried out in each case at 20 °C. The dry solid content was calculated using Eq. 1, which is suitable for an allulose syrup having a purity of 95%:

$$\text{ds [\%]} = \frac{Brix\ [\%]\text{-}1.15}{0.9251} \qquad \text{Eq. 1}$$

For the allulose syrup of batch 2 having a dry substance content (ds) of 95.6% the water content was determined by Karl-Fischer analysis to 92.8% (methanol/dichloromethane (1:1)).

Three batches of allulose syrup (each 25 to 35 L) having a dry substance content (ds) of 72.4% were concentrated stepwise by evaporation of water. Once the desired dry substance content of the concentrated allulose syrup was reached, parts of the concentrated allulose syrup (2 to 2.5 kg) were transferred to a laboratory kneader, wherein the allulose syrup was kneaded for a certain time. After kneading, a part of the allulose syrup was spread onto baking paper at room temperature and crystallization of the allulose syrup was examined. Parameters such as ds of the allulose syrup, kneading time, temperature and the optional addition of seed crystals during kneading of the allulose syrup were variated and the influence on the crystallization of the allulose syrup was examined.

After completion of each kneading test, the process chamber of the kneader was completely cleaned to avoid contamination with possible additional crystallization nuclei from the previous tests.

Concentrating of the allulose syrup was performed in an enclosed container. The allulose syrup was first heated to a maximum of 65 °C and then the vacuum pump was started and a vacuum of 0.85 to 0.95 bar was set. Evaporation of water was stopped every 15 or 30 min to determine the Brix value. For the duration of the Brix measurement heating was stopped. During operation of the vacuum pump, a small amount of fresh air was drawn in via the fresh air supply to entrain sufficient vapor flow.

Kneading of the allulose syrup was performed in a laboratory kneader at a speed of 40 rpm. Optional addition of seed crystals (10 wt.-%, relative to the amount of allulose syrup) was performed over a period of 60 seconds. It was ensured that the seed crystals were evenly distributed in the allulose syrup and that an accumulation around the kneading axis was avoided.

A first batch of allulose syrup (batch 1) having a dry substance content (ds) of 72.4 wt.-% was concentrated stepwise to a dry substance content (ds) of about 75 wt.-%, 80 wt.-%, 85%, 90 wt.-%, and 95 wt.-%. At a dry substance content (ds) of about 80 wt.-%, 85 wt.-%, 90 wt.-% and 95 wt.-%, respectively, a part of the allulose syrup was transferred to the kneader and crystallization was examined. To the allulose syrups with a dry substance content (ds) of about 85 wt.-% and 95 wt.-%, respectively, seed crystals were added.

A second batch of allulose syrup (batch 2) and a third batch of allulose syrup (batch 3) were concentrated stepwise to a dry substance content (ds) of about 95 wt.-%. Parts of the concentrated allulose syrups with a dry substance content (ds) of about 95 wt.-% were transferred to the kneader and crystallization of the allulose syrup was examined in each case.

The conditions for concentrating the allulose syrup batches 1 to 3 and the resulting Brix values and calculated ds values are compiled in the table here below:

| | Brix [%] | | | ds [wt.-%] | | temperature [°C] | | | stirrer speed | under pressure | time |
|---|---|---|---|---|---|---|---|---|---|---|---|
| | average | SD | no. | average | SD | enclosed container | allulose syrup start | allulose syrup end | [rpm] | [bar] | [min] |
| | 68.2 | 0.2 | 10 | 72.4 | 0.2 | - | - | | - | - | - |
| batch 1 | 71.1 | 0.7 | 15 | 75.6 | 0.7 | 65; 75[1] | 56 | 50 | 40 | 0.85-0.95 | 30 |
| batch 1 | 75.2 | 0.6 | 15 | 80.0 | 0.6 | 90 | 65 | 56 | 40 | 0.85-0.95 | 30 |
| batch 1 | 81.5 | 0.4 | 15 | 86.8 | 0.5 | 90 | 65 | 56 | 40 | 0.85-0.95 | 30 |
| batch 1 | 86.4 | 0.8 | 15 | 92.5 | 0.8 | 90 | 65 | 60 | 40 | 0.85-0.95 | 15 |
| batch 1 | 90.8 | 0.04 | 10 | 96.9 | 0.04 | 90 | 65 | 58 | 40 | 0.85-0.95 | 15 |
| batch 2 | 75.0 | 1.3 | 20 | 79.8 | 1.4 | 90 | 65 | 56 | 40 | 0.85-0.95 | 2x30 |
| batch 2 | 78.8 | 0.8 | 20 | 83.9 | 0.9 | 90 | 65 | 57 | 40 | 0.85-0.95 | 30+15 |
| batch 2 | 81.5 | 1.1 | 20 | 86.9 | 1.2 | 90 | 65 | 59 | 40 | 0.85-0.95 | 2x30 |
| batch 2 | 89.5 | 3.5 | 15 | 94.4 | 3.8 | 90 | 65 | 66 | 40 | 0.85-0.95 | 10 |
| batch 2 | 90.3 | 0.3 | 15 | 96.4 | 0.3 | - | - | 56 | 40 | - | - |
| batch 2 | 89.6 | 0.2 | 15 | 95.6 | 0.2 | 56 | 55±2 | 55 | 40 | 0.1-0.5[2] | - |
| batch 3 | 78.6 | 1.2 | 15 | 83.7 | 1.3 | 90 | 65 | 57 | 40 | -0.8-0.9 | 30 |
| batch 3 | 82.7 | 0.5 | 15 | 88.2 | 0.6 | 90 | 65 | 58 | 40 | -0.8-0.9 | 30 |
| batch 3 | 83.7 | 0.6 | 15 | 89.3 | 0.7 | 90 | 65 | 63 | 40 | -0.8-0.9 | 30 |
| batch 3 | 83.2 | 0.90 | 10 | 88.7 | 1.0 | 90 | 65 | 63 | 40 | -0.8-0.9 | 27 |
| batch 3 | 91.2 | 0.05 | 10 | 97.4 | 0.1 | 90 | 65 | 66 | 40 | -0.8-0.9 | 6 |
| batch 3 | 90.4 | 0.25 | 10 | 96.5 | 0.3 | 56 | - | - | 40 | - | - |

[1]after 10 min; [2]no vapor removed

The conditions for kneading of the allulose syrups and results of crystallization are compiled in the table here below:

| | | ds | allulose syrup | | | kneader | | seed crystals | | crystallization | | |
|---|---|---|---|---|---|---|---|---|---|---|---|---|
| | | average | m | T [°C] | | T | time | size | m | imme-diately | 24 h | 76 h |
| | | [%] | [g] | start | End | [°C] | [min] | [µm] | [g] | | | |
| batch 1 | 1-1 | 80.0 | 2460 | 53 | >50 | 60 | 10 | - | - | no | - | - |
| batch 1 | 1-2a | 86.8 | 1945 | 54 | >50 | 60 | 10 | - | - | no | - | - |
| batch 1 | 1-2b | 86.8 | 1945 | 54 | 54 | 60 | 20 | 190 | 195 | no | par-tially | - |
| batch 1 | 1-3 | 92.5 | 1961 | 57-58 | >50 | 60 | 10 | - | - | no | no | - |
| batch 1 | 1-4a | 96.9 | 1999 | 58 | >50 | 60 | 10 | - | - | no | par-tially | - |
| batch 1 | 1-4b | 96.9 | 1999 | 58 | >50 | 60 | 20 | 190 | 197 | yes | yes | |
| batch 1 | 1-4c | 96.9 | 1999 | 58 | 43-43.5 | - | 50 | 190 | 197 | yes | yes | |
| batch 2 | 2-1 | 96.4 | 2685 | 56 | 55±1 | 60 | 10 | - | - | no | - | yes |
| batch 2 | 2-2 | 95.6 | 2554 | 54 | 55±1 | 60 | 10 | 190 | 256 | yes | | |
| batch 2 | 2-3 | 95.6 | 2581 | 53 | 55±1 | 60 | 10 | 36 | 258 | yes | | |
| batch 2 | 2-4a | 95.6 | 2490 | 53 | 41-42 | 40 | 10 | 190 | 249 | yes | | |
| batch 2 | 2-4b | 95.6 | 2490 | 53 | 41-42 | 40 | 40 | 190 | 249 | yes | | |
| batch 2 | 2-5 | 95.6 | 2580 | 55 | 40 | - | 10 | 190 | 258 | yes | | |
| batch 2 | 2-6 | 95.6 | 2481 | 52 | 40 | - | 10 | 190 | 248 | yes | | |
| batch 2 | 2-7 | 95.6 | 2559 | 53 | 38-39 | - | 10 | 36 | 259 | yes | | |
| batch 3 | 3-1a | 96.5 | 2810 | 55 | 49 | 40 | 10 | 190 | 280 | yes | | |
| batch 3 | 3-1b | 96.5 | 2810 | 55 | 49 | 40 | 20 | 190 | 280 | yes | | |
| batch 3 | 3-1c | 96.5 | 2810 | 55 | 49 | 40 | 30 | 190 | 280 | yes | | |
| batch 3 | 3-2a | 96.5 | 3320 | 52 | 50 | 40 | 10 | 190 | 330 | yes | | |
| batch 3 | 3-2b | 96.5 | 3320 | 52 | 50 | 40 | 20 | 190 | 330 | yes | | |
| batch 3 | 3-2c | 96.5 | 3320 | 52 | 50 | 40 | 30 | 190 | 330 | yes | | |
| batch 3 | 3-3a | 96.5 | 3445 | 53 | 53 | 40 | 10 | 190 | 344 | yes | | |
| batch 3 | 3-3b | 96.5 | 3445 | 53 | 53 | 40 | 20 | 190 | 344 | yes | | |
| batch 3 | 3-3c | 96.5 | 3445 | 53 | 53 | 40 | 30 | 190 | 344 | yes | | |

Crystallization of allulose syrups having a dry substance content (ds) of less than about 95 wt.-% could not be observed without the addition of seed crystals.

Partial crystallization of an allulose syrup with a dry substance content (ds) of about 85 wt.-% could be observed after addition of seed crystals and more than 24 h crystallization time at room temperature following the kneading. The allulose syrup did not crystallize completely, which indicates that the residual water content was still too high.

Partial crystallization could also be observed for an allulose syrup having a dry substance content (ds) of about 95 wt.-% without addition of seed crystals after more than 24 h crystallization time at room temperature following the kneading. The allulose syrup did not crystallize completely, but remained sticky and formable. After a crystallization time of at least 76 h full crystallization of the allulose syrup was observed.

Allulose syrups having a dry substance content (ds) of about 95 wt.-% already crystallized during kneading, when seed crystals were added. The allulose syrups were crystallized completely after 10 to 20 minutes at room temperature following the kneading. Longer kneading times shortened the crystallization timer after kneading. Variation of the crystal size of the seed crystals did not show an influence on the crystallization of the allulose syrups.

## Claims

**1.** A process for the preparation of a product allulose composition comprising the steps of

(a) providing a liquid allulose composition comprising allulose dissolved in water, wherein the liquid allulose composition provided in step (a) has an allulose content of at least 69 wt.-% and a water content of at most 30 wt.-%; in each case relative to the total weight of the liquid allulose composition;
(b) heating the liquid allulose composition to an elevated temperature, wherein step (b) is performed under evaporative conditions thereby reducing the content of water of the allulose composition and wherein the elevated temperature is at most 65°C;
(c) feeding the allulose composition into an extruder, wherein the allulose composition that is fed into the extruder in step (c) has a water content of at most 5.0 wt.-%, relative to the total weight of the allulose composition that is fed into the extruder;
(d) extruding the allulose composition in the extruder;
(e) obtaining a product allulose composition from the extruder;
(f) allowing the product allulose composition to solidify; preferably precipitate and/or crystallize; and
(g) optionally, grinding and/or post-drying the product allulose composition.

**2.** The process according to claim 1, wherein the liquid allulose composition provided in step (a) has

(A) an allulose content of at least 73 wt.-%, preferably at least 77 wt.-%, more preferably at least 81 wt.-%, most preferably at least 85 wt.-%, and in particular at least 89 wt.-%; in each case relative to the total weight of the liquid allulose composition; and/or
(B) a water content of at least 2.5 wt.-%; preferably at least 5.0 wt.-%, more preferably at least 7.5 wt.-%, still more preferably at least 10 wt.-%, yet more preferably at least 15 wt.-%, even more preferably at least 20 wt.-%, and most preferably at least 25 wt.-%; in each case relative to the total weight of the liquid allulose composition.

**3.** The process according to any of the preceding claims, wherein the extruder is a single screw extruder, twin screw extruder or planetary roller extruder; preferably wherein the extruder is a twin screw extruder with contrarotating screw configuration or corotating screw configuration.

**4.** The process according to any of the preceding claims, wherein the allulose composition that is fed into the extruder in step (c) has a water content of at most 4.0 wt.-%, preferably at most 3.0 wt.-%, and in particular at most 2.0 wt.-%; in each case relative to the total weight of the allulose composition that is fed into the extruder.

**5.** The process according to any of the preceding claims, wherein step (c) comprises feeding seed crystals into the extruder.

**6.** The process according to any of the preceding claims, wherein in step (d) the extruder is operated at an elevated extrusion temperature; wherein the elevated extrusion temperature is

(A) at least 30°C; preferably at least 31°C, preferably at least 32°C, preferably at least 33°C, preferably at least 34°C, preferably at least 35°C, preferably at least 36°C, preferably at least 37°C, preferably at least 38°C,

preferably at least 39°C, preferably at least 40°C, preferably at least 41°C, preferably at least 42°C, preferably at least 43°C, preferably at least 44°C, preferably at least 45°C, preferably at least 46°C, preferably at least 47°C, preferably at least 48°C, preferably at least 49°C, preferably at least 50°C, preferably at least 51°C, preferably at least 52°C, preferably at least 53°C, preferably at least 54°C, preferably at least 55°C, preferably at least 56°C, preferably at least 57°C, preferably at least 58°C, preferably at least 59°C, preferably at least 60°C; and/or
(B) at most 80°C; preferably at most 79°C, preferably at most 78°C, preferably at most 77°C, preferably at most 76°C, preferably at most 75°C, preferably at most 74°C, preferably at most 73°C, preferably at most 72°C, preferably at most 71°C, preferably at most 70°C, preferably at most 69°C, preferably at most 68°C, preferably at most 67°C, preferably at most 66°C, preferably at most 65°C, preferably at most 64°C, preferably at most 63°C, preferably at most 62°C, preferably at most 61°C, preferably at most 60°C, preferably at most 59°C, preferably at most 58°C, preferably at most 57°C, preferably at most 56°C, preferably at most 55°C, preferably at most 54°C, preferably at most 53°C, preferably at most 52°C, preferably at most 51°C, preferably at most 50°C; and/or
(C) at least 35°C; preferably at least 40°C, more preferably at least 43°C, still more preferably at least 46°C, yet more preferably at least 49°C, even more preferably at least 52°C, most preferably at least 55°C, and in particular at least 58°C; and/or
(D) at most 70°C; preferably at most 65°C, more preferably at most 60°C, still more preferably at most 57°C, yet more preferably at most 54°C, even more preferably at most 51°C, most preferably at most 48°C, and in particular at most 45°C; and/or
(E) within the range of 53±20°C; preferably 53±15°C, more preferably 53±12°C, still more preferably 53±10°C, yet more preferably 53±8.0°C, even more preferably 53±6.0°C, most 53±4.0°C, and in particular 53±3.0°C.

**7.** The process according to any of the preceding claims, wherein the product allulose composition solidifies completely, preferably precipitates and/or crystallizes after being obtained from the extruder.

**8.** The process according to any of the preceding claims, wherein the product allulose composition solidifies completely, preferably precipitates and/or crystallizes within a time of at most 76 hours, preferably at most 24 hours, more preferably at most 12 hours, still more preferably at most 60 minutes, yet more preferably at most 40 minutes, even more preferably at most 20 minutes, most preferably at most 15 minutes, and in particular at most 10 minutes.

**9.** The process according to any of the preceding claims, wherein the product allulose composition obtained in step (e), (f) or (g) has a residual water content of at most 2.5 wt.-%; preferably at most 2.4 wt.-%, preferably at most 2.3 wt.-%, preferably at most 2.2 wt.-%, preferably at most 2.1 wt.-%, preferably at most 2.0 wt.-%, preferably at most 1.9 wt.-%, preferably at most 1.8 wt.-%, preferably at most 1.7 wt.-%, preferably at most 1.6 wt.-%, preferably at most 1.5 wt.-%, preferably at most 1.4 wt.-%, preferably at most 1.3 wt.-%, preferably at most 1.2 wt.-%, preferably at most 1.1 wt.-%, preferably at most 1.0 wt.-%, preferably at most 0.9 wt.-%, preferably at most 0.8 wt.-%, preferably at most 0.7 wt.-%, preferably at most 0.6 wt.-%, preferably at most 0.5 wt.-%; in each case relative to the total weight of the product allulose composition.

## Patentansprüche

**1.** Ein Verfahren zur Herstellung einer Produkt-Allulose-Zusammensetzung, umfassend die folgenden Schritte

(a) Bereitstellen einer flüssigen Allulose-Zusammensetzung, die in Wasser gelöste Allulose umfasst, wobei die in Schritt (a) bereitgestellte flüssige Allulose-Zusammensetzung einen Allulose-Gehalt von mindestens 69 Gew.-% und einen Wassergehalt von höchstens 30 Gew.-% aufweist, jeweils bezogen auf das Gesamtgewicht der flüssigen Allulose-Zusammensetzung;
(b) Erhitzen der flüssigen Allulosezusammensetzung auf eine erhöhte Temperatur, wobei Schritt (b) unter Verdampfungsbedingungen durchgeführt wird, wodurch der Wassergehalt der Allulosezusammensetzung verringert wird, und wobei die erhöhte Temperatur höchstens 65 °C beträgt;
(c) Einführen der Allulosezusammensetzung in einen Extruder, wobei die in Schritt (c) in den Extruder eingeführte Allulosezusammensetzung einen Wassergehalt von höchstens 5,0 Gew.-% aufweist, bezogen auf das Gesamtgewicht der in den Extruder eingeführten Allulosezusammensetzung;
(d) Extrudieren der Allulosezusammensetzung in dem Extruder;
(e) Gewinnen einer Produkt-Allulosezusammensetzung aus dem Extruder;
(f) Erstarrenlassen der Produkt-Allulosezusammensetzung; vorzugsweise Ausfällen und/oder Kristallisieren; und
(g) optional Mahlen und/oder Nachtrocknen der Produkt-Allulosezusammensetzung.

**2.** Das Verfahren nach Anspruch 1, wobei die in Schritt (a) bereitgestellte flüssige Allulosezusammensetzung

(A) einen Allulosegehalt von mindestens 73 Gew.-% hat, vorzugsweise mindestens 77 Gew.-%, noch bevorzugter mindestens 81 Gew.-%, am meisten bevorzugt mindestens 85 Gew.-% und insbesondere mindestens 89 Gew.-%; jeweils bezogen auf das Gesamtgewicht der flüssigen Allulosezusammensetzung; und/oder
(B) einen Wassergehalt von mindestens 2,5 Gew.-% hat, vorzugsweise mindestens 5,0 Gew.-%, noch bevorzugter mindestens 7,5 Gew.-%, noch mehr bevorzugt mindestens 10 Gew.-%, noch mehr bevorzugt mindestens 15 Gew.-%, noch mehr bevorzugt mindestens 20 Gew.-% und am meisten bevorzugt mindestens 25 Gew.-%, jeweils bezogen auf das Gesamtgewicht der flüssigen Allulosezusammensetzung.

**3.** Das Verfahren nach einem der vorstehenden Ansprüche, wobei der Extruder ein Einschneckenextruder, ein Doppelschneckenextruder oder ein Planetenwalzenextruder ist; vorzugsweise wobei der Extruder ein Doppelschneckenextruder mit gegenläufiger Schneckenkonfiguration oder gleichläufiger Schneckenkonfiguration ist.

**4.** Das Verfahren nach einem der vorstehenden Ansprüche, wobei die Allulosezusammensetzung, die in Schritt (c) in den Extruder eingespeist wird, einen Wassergehalt von höchstens 4,0 Gew.-%, vorzugsweise höchstens 3,0 Gew.-% und insbesondere höchstens 2,0 Gew.-% aufweist, jeweils bezogen auf das Gesamtgewicht der in den Extruder eingespeisten Allulosezusammensetzung.

**5.** Das Verfahren nach einem der vorstehenden Ansprüche, wobei Schritt (c) das Einbringen von Impfkristallen in den Extruder umfasst.

**6.** Das Verfahren nach einem der vorstehenden Ansprüche, wobei in Schritt (d) der Extruder bei einer erhöhten Extrusionstemperatur betrieben wird; wobei die erhöhte Extrusionstemperatur

(A) mindestens 30 °C beträgt; vorzugsweise mindestens 31 °C, vorzugsweise mindestens 32 °C, vorzugsweise mindestens 33 °C, vorzugsweise mindestens 34 °C, vorzugsweise mindestens 35 °C, vorzugsweise mindestens 36 °C, vorzugsweise mindestens 37 °C, vorzugsweise mindestens 38 °C, vorzugsweise mindestens 39 °C, vorzugsweise mindestens 40 °C, vorzugsweise mindestens 41 °C, vorzugsweise mindestens 42 °C, vorzugsweise mindestens 43 °C, vorzugsweise mindestens 44 °C, vorzugsweise mindestens 45 °C, vorzugsweise mindestens 46 °C, vorzugsweise mindestens 47 °C, vorzugsweise mindestens 48 °C, vorzugsweise mindestens 49 °C, vorzugsweise mindestens 50 °C, vorzugsweise mindestens 51 °C, vorzugsweise mindestens 52 °C, vorzugsweise mindestens 53 °C, vorzugsweise mindestens 54 °C, vorzugsweise mindestens 55 °C, vorzugsweise mindestens 56 °C, vorzugsweise mindestens 57 °C, vorzugsweise mindestens 58 °C, vorzugsweise mindestens 59 °C, vorzugsweise mindestens 60 °C; und/oder
(B) höchstens 80 °C beträgt; vorzugsweise höchstens 79 °C, vorzugsweise höchstens 78 °C, vorzugsweise höchstens 77 °C, vorzugsweise höchstens 76 °C, vorzugsweise höchstens 75 °C, vorzugsweise höchstens 74 °C, vorzugsweise höchstens 73 °C, vorzugsweise höchstens 72 °C, vorzugsweise höchstens 71 °C, vorzugsweise höchstens 70 °C, vorzugsweise höchstens 69 °C, vorzugsweise höchstens 68 °C, vorzugsweise höchstens 67 °C, vorzugsweise höchstens 66 °C, vorzugsweise höchstens 65 °C, vorzugsweise höchstens 64 °C, vorzugsweise höchstens 63 °C, vorzugsweise höchstens 62 °C, vorzugsweise höchstens 61 °C, vorzugsweise höchstens 60 °C, vorzugsweise höchstens 59 °C, vorzugsweise höchstens 58 °C, vorzugsweise höchstens 57 °C, vorzugsweise höchstens 56 °C, vorzugsweise höchstens 55 °C, vorzugsweise höchstens 54 °C, vorzugsweise höchstens 53 °C, vorzugsweise höchstens 52 °C, vorzugsweise höchstens 51 °C, vorzugsweise höchstens 50 °C; und/oder
(C) mindestens 35 °C beträgt; vorzugsweise mindestens 40 °C, noch bevorzugter mindestens 43 °C, noch bevorzugter mindestens 46 °C, noch bevorzugter mindestens 49 °C, noch bevorzugter mindestens 52 °C, am meisten bevorzugt mindestens 55 °C und insbesondere mindestens 58 °C; und/oder
(D) höchstens 70 °C beträgt; vorzugsweise höchstens 65 °C, noch bevorzugter höchstens 60 °C, noch mehr bevorzugt höchstens 57 °C, noch mehr bevorzugt höchstens 54 °C, noch mehr bevorzugt höchstens 51 °C, am meisten bevorzugt höchstens 48 °C und insbesondere höchstens 45 °C; und/oder
(E) im Bereich von 53 $\pm$ 20 °C liegt; vorzugsweise 53 $\pm$ 15 °C, noch bevorzugter 53 $\pm$ 12 °C, noch mehr bevorzugt 53 $\pm$ 10 °C, noch mehr bevorzugt 53 $\pm$ 8,0 °C, noch mehr bevorzugt 53 $\pm$ 6,0 °C, am meisten 53 $\pm$ 4,0 °C und insbesondere 53 $\pm$ 3,0 °C.

**7.** Das Verfahren nach einem der vorstehenden Ansprüche, wobei die Produkt-Allulose-Zusammensetzung vollständig erstarrt, vorzugsweise ausfällt und/oder kristallisiert, nachdem sie aus dem Extruder erhalten wurde.

8. Das Verfahren nach einem der vorstehenden Ansprüche, wobei die Produkt-Allulose-Zusammensetzung innerhalb einer Zeit von höchstens 76 Stunden vollständig erstarrt, vorzugsweise höchstens 24 Stunden, noch bevorzugter höchstens 12 Stunden, noch mehr bevorzugt höchstens 60 Minuten, noch bevorzugter innerhalb von höchstens 40 Minuten, noch bevorzugter innerhalb von höchstens 20 Minuten, am meisten bevorzugt innerhalb von höchstens 15 Minuten und insbesondere innerhalb von höchstens 10 Minuten.

9. Das Verfahren nach einem der vorstehenden Ansprüche, wobei die in Schritt (e), (f) oder (g) erhaltene Allulosezusammensetzung einen Restwassergehalt von höchstens 2,5 Gew.-% aufweist; vorzugsweise höchstens 2,4 Gew.-%, vorzugsweise höchstens 2,3 Gew.-%, vorzugsweise höchstens 2,2 Gew.-%, vorzugsweise höchstens 2,1 Gew.-%, vorzugsweise höchstens 2,0 Gew.-%, vorzugsweise höchstens 1,9 Gew.-%, vorzugsweise höchstens 1,8 Gew.-%, vorzugsweise höchstens 1,7 Gew.-%, vorzugsweise höchstens 1,6 Gew.-%, vorzugsweise höchstens 1,5 Gew.-%, vorzugsweise höchstens 1,4 Gew.-%, vorzugsweise höchstens 1,3 Gew.-%, vorzugsweise höchstens 1,2 Gew.-%, vorzugsweise höchstens 1,1 Gew.-%, vorzugsweise höchstens 1,0 Gew.-%, vorzugsweise höchstens 0,9 Gew.-%, vorzugsweise höchstens 0,8 Gew.-%, vorzugsweise höchstens 0,7 Gew.-%, vorzugsweise höchstens 0,6 Gew.-%, vorzugsweise höchstens 0,5 Gew.-%; jeweils bezogen auf das Gesamtgewicht der Allulosezusammensetzung des Produkts.

**Revendications**

1. Procédé de préparation d’une composition d’allulose comprenant les étapes suivantes :

   (a) la fourniture d’une composition d’allulose liquide comprenant de l’allulose dissoute dans de l’eau, dans lequel la composition d’allulose liquide fournie à l’étape (a) présente une teneur en allulose d’au moins 69 % en poids et une teneur en eau d’au plus 30 % en poids ; dans chaque cas par rapport au poids total de la composition d’allulose liquide ;
   (b) le chauffage de la composition d’allulose liquide à une température élevée, dans lequel l’étape (b) est réalisée dans des conditions d’évaporation réduisant ainsi la teneur en eau de la composition d’allulose et la température élevée étant d’au plus 65°C ;
   (c) l’introduction de la composition d’allulose dans une extrudeuse, dans lequel la composition d’allulose qui est introduite dans l’extrudeuse à l’étape (c) présente une teneur en eau d’au plus 5,0 % en poids, par rapport au poids total de la composition d’allulose qui est introduite dans l’extrudeuse ;
   (d) l’extrusion de la composition d’allulose dans l’extrudeuse ;
   (e) l’obtention d’une composition d’allulose produite à partir de l’extrudeuse ;
   (f) le fait de permettre à la composition d’allulose produite de se solidifier ; de préférence précipiter et/ou cristalliser ; et
   (g) éventuellement, le broyage et/ou le post-séchage de la composition d’allulose produite.

2. Procédé selon la revendication 1, dans lequel la composition d’allulose liquide fournie à l’étape (a) présente :

   (A) une teneur en allulose d’au moins 73 % en poids, de préférence d’au moins 77 %, plus préférablement d’au moins 81 %, le plus préférablement d’au moins 85 % en poids, et en particulier d’au moins 89 % en poids ; dans chaque cas par rapport au poids total de la composition liquide d’allulose ; et/ou
   (B) une teneur en eau d’au moins 2,5 % en poids ; de préférence d’au moins 5,0 % en poids, plus préférablement d’au moins 7,5 % en poids, encore plus préférablement d’au moins 10 % en poids, encore plus préférablement d’au moins 15 % en poids, encore plus préférablement d’au moins 20 % en poids, et le plus préférablement d’au moins 25 % en poids ; dans chaque cas par rapport au poids total de la composition d’allulose liquide.

3. Procédé selon l’une quelconque des revendications précédentes, dans lequel l’extrudeuse est une extrudeuse à vis unique, une extrudeuse à double vis ou une extrudeuse à rouleaux planétaires ; de préférence dans lequel l’extrudeuse est une extrudeuse à double vis avec une configuration de vis contrarotative ou une configuration de vis corotative.

4. Procédé selon l’une quelconque des revendications précédentes, dans lequel la composition d’allulose introduite dans l’extrudeuse à l’étape (c) présente une teneur en eau d’au plus 4,0 % en poids, de préférence d’au plus 3,0 % en poids, et en particulier d’au plus 2,0 % en poids, dans chaque cas par rapport au poids total de la composition d’allulose introduite dans l’extrudeuse.

5. Procédé selon l'une quelconque des revendications précédentes, dans lequel l'étape (c) comprend l'introduction de cristaux d'ensemencement dans l'extrudeuse.

6. Procédé selon l'une quelconque des revendications précédentes, dans lequel, à l'étape (d), l'extrudeuse fonctionne à une température d'extrusion élevée ; dans lequel la température d'extrusion élevée est

(A) au moins 30°C ; de préférence au moins 31 °C, de préférence au moins 32 °C, de préférence au moins 33 °C, de préférence au moins 34 °C, de préférence au moins 35 °C, de préférence au moins 36 °C, de préférence au moins 37 °C, de préférence au moins 38 °C, de préférence au moins 39 °C, de préférence au moins 40 °C, de préférence au moins 41 °C, de préférence au moins 42 °C, de préférence au moins 43 °C, de préférence au moins 44 °C, de préférence au moins 45 °C, de préférence au moins 46 °C, de préférence au moins 47 °C, de préférence au moins 48 °C, de préférence au moins 49 °C, de préférence au moins 50 °C, de préférence au moins 51 °C, de préférence au moins 52 °C, de préférence au moins 53 °C, de préférence au moins 54 °C, de préférence au moins 55 °C, de préférence au moins 56 °C, de préférence au moins 57 °C, de préférence au moins 58°C, de préférence au moins 59°C, de préférence au moins 60°C ; et/ou
(B) au plus 80°C ; de préférence au plus 79 °C, de préférence au plus 78 °C, de préférence au plus 77 °C, de préférence au plus 76 °C, de préférence au plus 75 °C, de préférence au plus 74 °C, de préférence au plus 73 °C, de préférence au plus 72 °C, de préférence au plus 71 °C, de préférence au plus 70 °C, de préférence au plus 69 °C, de préférence au plus 68 °C, de préférence au plus 67 °C, de préférence au plus 66 °C, de préférence au plus 65 °C, de préférence au plus 64 °C, de préférence au plus 63 °C, de préférence au plus 62 °C, de préférence au plus 61 °C, de préférence au plus 60 °C, de préférence au plus 59 °C, de préférence au plus 58 °C, de préférence au plus 57 °C, de préférence au plus 56 °C, de préférence au plus 55 °C, de préférence au plus 54 °C, de préférence au plus 53 °C, de préférence au plus 52°C, de préférence au plus 51°C, de préférence au plus 50°C ; et/ou
(C) au moins 35 °C ; de préférence au moins 40 °C, plus préférablement au moins 43 °C, encore plus préférablement au moins 46 °C, encore plus préférablement au moins 49 °C, encore plus préférablement au moins 52 °C, le plus préférablement au moins 55 °C, et en particulier au moins 58 °C ; et/ou
(D) au moins 70 °C ; de préférence au plus 65 °C, plus préférablement au plus 60 °C, encore plus préférablement au plus 57 °C, encore plus préférablement au plus 54 °C, encore plus préférablement au plus 51 °C, le plus préférablement au plus 48 °C, et en particulier au moins 45 °C ; et/ou
(E) dans la plage de 53±20°C ; de préférence 53±15°C, plus préférablement 53±12°C, encore plus préférablement 53±10°C, encore plus préférablement 53±8,0°C, encore plus préférablement 53±6,0°C, le plus souvent 53±4,0°C, et en particulier 53±3,0°C.

7. Procédé selon l'une quelconque des revendications précédentes, dans lequel la composition d'allulose produite se solidifie complètement, de préférence précipite et/ou cristallise après avoir été obtenue à partir de l'extrudeuse.

8. Procédé selon l'une quelconque des revendications précédentes, dans lequel la composition d'allulose produite se solidifie complètement, de préférence précipite et/ou cristallise dans un délai d'au plus 76 heures, de préférence au plus 24 heures, plus préférablement au plus 12 heures, encore plus préférablement au plus 60 minutes, encore plus préférablement au plus 40 minutes, encore plus préférablement au plus 20 minutes, le plus préférablement au plus 15 minutes, et en particulier au plus 10 minutes.

9. Procédé selon l'une quelconque des revendications précédentes, dans lequel la composition d'allulose produite obtenue à l'étape (e), (f) ou (g) présente une teneur en eau résiduelle d'au plus 2,5 % en poids ; de préférence au plus 2,4 % en poids, de préférence au plus 2,3 % en poids, de préférence au plus 2,2 % en poids, de préférence au plus 2,1 % en poids, de préférence au plus 2,0 % en poids, de préférence au plus 1,9 % en poids, de préférence au plus 1,8 % en poids, de préférence au plus 1,7 % en poids, de préférence au plus 1,6 % en poids, de préférence au plus 1,5 % en poids, de préférence au plus 1,4 % en poids, de préférence au plus 1,3 % en poids, de préférence au plus 1,2 % en poids, de préférence au plus 1,1 % en poids, de préférence au plus 1,0 % en poids, de préférence au plus 0,9 % en poids, de préférence au plus 0,8 % en poids, de préférence au plus 0,7 % en poids, de préférence au plus 0,6 % en poids, de préférence au plus 0,5 % en poids ; dans chaque cas par rapport au poids total de la composition d'allulose du produit.

Figure 1

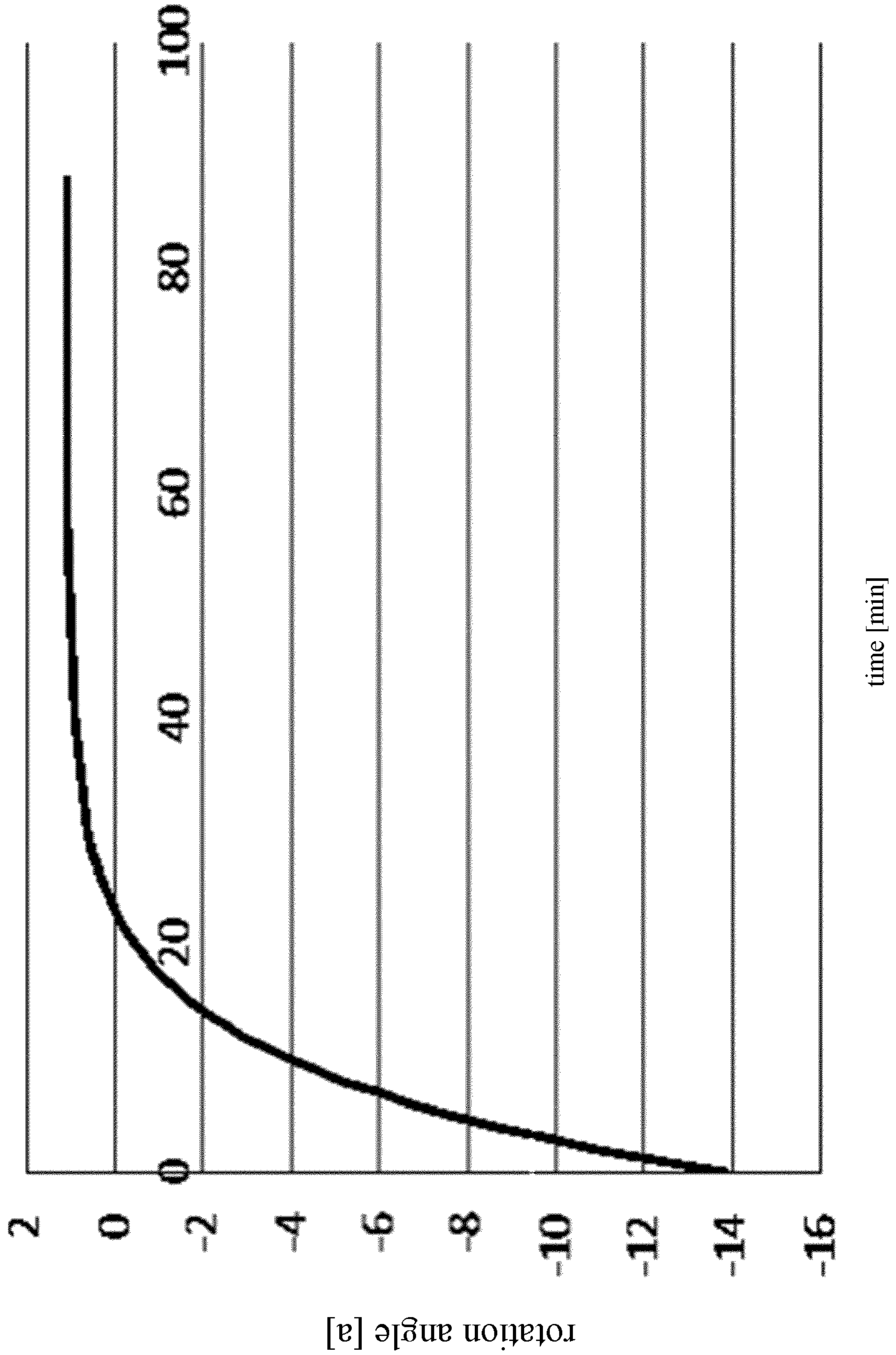

Figure 2

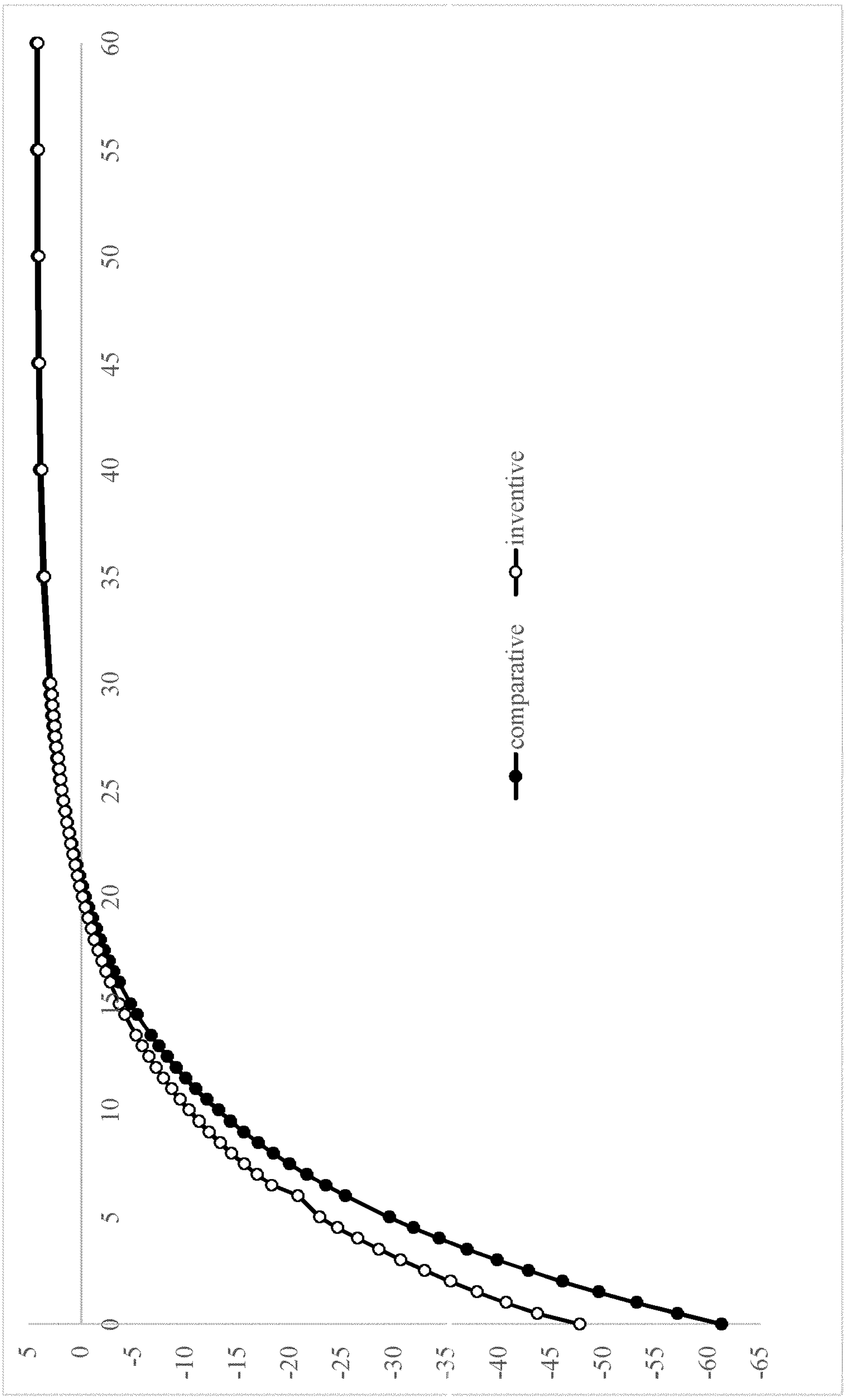

5
0
-5
-10
-15
-20
-25
-30
-35
-40
-45
-50
-55
-60
-65
0
5
10
15
20
25
30
35
40
45
50
55
60
comparative
inventive

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 104447888
- CN 106480125
- CN 107699557
- US 2011237790 A
- US 2014370171 A
- US 2016050954 A
- US 2016302463 A
- KR 2016062349
- WO 2017029244 A
- US 2017064988 A
- US 2017313734 A
- US 2018049458 A
- WO 2018105931 A
- WO 2018149707 A
- US 2018255814 A
- US 2018271112 A
- US 2018271113 A
- US 2018279643 A
- US 2018327796 A
- WO 2019004554 A
- WO 2019082206 A
- WO 2019083069 A
- WO 2019088654 A
- US 2019029299 A
- US 2019297931 A
- US 2019328014 A
- US 2019330253 A
- CN 110872332
- WO 2020005021 A
- US 2020040023 A
- US 2020062792 A
- US 2020085090 A
- US 2018281263 A
- US 2020001502 A
- EP 0919127 A1
- AR 403

### Non-patent literature cited in the description

- **A. KWIECIEN et al.** *Carboh Res*, 2008, vol. 343 (13), 2336-2339
- **K. FUKADA et al.** *Bull Chem Soc Japan*, 2010, vol. 83 (10), 1193-1197
- **P.H.M. HERVE DU PENHOAT et al.** *Carboh Res*, 1974, vol. 36 (1), 111-120
- **A.D. FRENCH et al.** *Computational Chemistry*, 1994, vol. 15 (5), 561-570
- **S.J. ANGYAL et al.** Advances in Carbohydrate Chemistry. Academic Press, 1984, vol. 42
- **R.N. GOLDBERG et al.** *J. Phys. Chem. Ref. Data*, 1989, vol. 18 (2), 809-880